(19) Europäisches Patentamt
European Patent Office
Office européen des brevets

(11) Publication number: **0 442 172 A1**

# (12) EUROPEAN PATENT APPLICATION

(21) Application number: **90301439.7**

(22) Date of filing: **12.02.90**

(51) Int. Cl.5: **C07C 251/48**, C07C 251/66, C07C 327/00, C07C 309/00, C07D 213/643, A01N 35/10, A01N 43/00

(43) Date of publication of application: **21.08.91 Bulletin 91/34**

(84) Designated Contracting States: **DK ES GR**

(71) Applicant: **TEIJIN LIMITED**
**6-7, Minamihonmachi 1-chome Chuo-ku**
**Osaka-shi Osaka 541(JP)**

(72) Inventor: **Azuma, Shizuo**
**4-22-1 Hirata**
**Iwakuni-shi, Yamaguchi-ken(JP)**
Inventor: **Hiramatsu, Toshiyuki**
**1981-9, Tsuzu**
**Iwakuni-shi, Yamaguchi-ken(JP)**
Inventor: **Nakagawa, Koji**
**478-61 Mizuno**
**Sayama-shi, Saitama-ken(JP)**
Inventor: **Ichikawa, Yataro**
**2-11-7, Kotesashi-machi**
**Tokorozawa-shi, Saitama-ken(JP)**

(74) Representative: **Votier, Sidney David et al**
**CARPMAELS & RANSFORD 43, Bloomsbury Square**
**London WC1A 2RA(GB)**

## (54) Oxime derivatives and Herbicides containing the same as an active ingredient.

(57) The present invention relates to a specific oxime derivative or its salts of formula I and herbicidal compositions containing the same. The oxime derivatives according to the present invention show excellent herbicidal effect against broad leaved weeds and narrow leaved weeds, and additionally, have quick-acting herbicidal activity with reduced application.

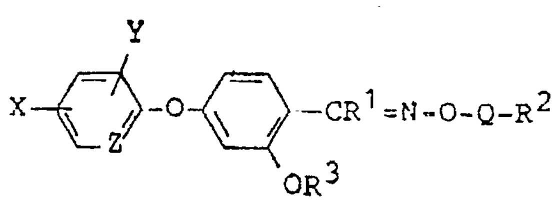

Wherein substituents in formula (I) are as defined in claim 1.

EP 0 442 172 A1

# OXIME DERIVATIVES AND HERBICIDES CONTAINING THE SAME AS AN ACTIVE INGREDIENT

## Field of the Invention:

The present invention relates to oxime derivatives and herbicidal compositions containing the same as an active ingredient. Particularly, the present invention relates to a herbicide which shows excellent herbicidal activity against broad leaved weeds and narrow leaved weeds, and has selective herbicidal activity without any adverse effect on the growth of crop plants by selecting the application methods, treating methods and application rates appropriately.

## Description on the Prior Arts:

Herbicides of the type which selectively kills broad-leaved weeds, typified by 2,4-dichlorophenoxyacetic acid, are known as selective herbicidally active compounds. the selectivity of the herbicidal activity of 2,4-dichlorophenoxyacetic acid is between narrow-leaved plants including both crop plants and weeds, and broad-leaved plants including both crop plants and weeds. It is known that 2,4-dichlorophenoxyacetic acid has very little or no activity against narrow-leaved plants [see, for example, Nature, 155, 498 (1945)]. It is known, on the other hand, that compounds resulting from introduction of a chloro- or trifluoromethyl-substituted phenoxy group or a chloro- or trifluoromethyl-substituted pyridyloxy group into the aromatic group of the above compound have the activity of selectively killing narrow-leaved plants (see U.S. Patents Nos. 4,270,948; 4,309,562; 4,314,069; 4,332,961 and 3,954,442; British Patent No. 1,579,201, and Japanese Laid- Open Patent Publication Nos. 125626/1977 and 15825/1977). These compounds, however, also kill useful crops such as rice or corn.

Herbicides containing some kinds of N-phosphonomethylglycine derivatives as active ingredients are known and commercially available. These N-phosphonomethylglycine derivatives are basically non-selective herbicides, but their herbicidal activity is reduced in a low application rate against perennial weeds such as Cyperus rotundus in Cyperaceae and against broad-leaved weeds such as Chenopodium album var. centrorubrum or Amaranthus retroflexas. Particularly, they hardly develop herbicidal activity against weeds in Convolvulaceae such as Ipomoea purpurea even 2 weeks after application. Further, the N-phosphonomethylglycine derivatives are slow acting and unsuitable for the cases that the weeds are whithered quickly and the next work will be started soon in crop lands or non-crop lands. For example, in a crop land, all of the weeds should be eradicated before sowing of a crop plant, but the slow acting may cause adverse effects such as delay of sowing time and damage to the crop seeds. Additionally, in non-crop lands, delays in constructions of buildings or railroads or weeding delay in roads may be caused, thus the N-phosphonomethylglycine derivatives are unsatisfactory as a non-selective herbicide quick-acting at a low application rate.

Herbicides containing some kinds of glufosinate compounds as a major active ingredient are known and commercially available. This type of herbicides also is basically non-selective, but has a disadvantage that a low application rate reduces herbicidal activity against broad-leaved weeds such as Chenopodium album var. centrorubrum, Amaranthus retroflexas or Abutilon theophrasti.

## The Objects and Summary of the Invention:

It is an object of the present invention to provide a novel oxime derivative.

Another object of the present invention is to provide a herbicide showing excellent herbicidal effect against broad-leaved weeds and narrow-leaved weeds.

Another object of the present invention is to provide a compound and a herbicide containing the same which selectively eradicates broad-leaved weeds and narrow-leaved weeds substantially without chemical injury to useful crop plants, especially soybeans and corns, by choosing appropriately the application methods, treating methods and application rates.

Another object of the present invention is to provide a compound and a selective herbicide containing the same which can act on plant bodies to kill or inhibit the growth of a plurality of broad-leaved weeds and narrow-leaved weeds substantially without chemical injury to narrow-leaved crops such as corn and broad-leaved crops such as soybean, accordingly can create a state where crop plants can easier grow than noxious weeds do in an area where both useful crops and noxious weeds are growing.

Another object of the present invention is to provide a selective herbicide which can not only kill or inhibit the growth of the target weeds, but also inhibit the germination of the weeds and kill the grown plants

substantially without inhibition of germination and growth of useful crop plants by treating the soil with the herbicide before germination, in other words, to provide a herbicide which can be used by both foliar spraying and soil treatment.

Another object of the present invention is to provide a herbicide which has low toxicity to animals and fish and remains little in the soil.

Another object of the present invention is to provide a herbicidal composition which can kill both narrow-leaved and broad-leaved weeds at a low application rate by combining the novel oxime derivative according to the present invention with known N-phosphonomethylglycine derivative and/or glufosinate derivative to utilize their herbicidal properties.

Another object of the present invention is to provide novel benzaldehyde derivative as an intermediate which can prepare the oxime derivatives in high yield efficiently in the production of novel oxime derivatives according to the present invention.

Further objects of the invention along with its advantage will become apparent from the following description.

According to the study by the inventors, these above objects and advantages of the invention are achieved by the following oxime derivatives.

Namely, according to the present invention,
oxime derivatives of formula (I)

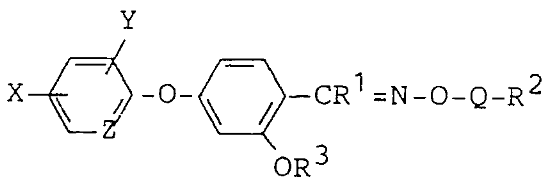

(I)

wherein
X and Y are identical or different and each represents a hydrogen atom, a halogen atom, $-CF_3$, or an alkyl group having 1 to 5 carbon atoms;
Z is =CH- or =N-;
$R^1$ represents a hydrogen atom, a hydroxyl group, an alkyl group having 1 to 5 carbon atoms, or an alkoxy group having 1 to 5 carbon atoms;
$R^2$ represents a group selected from the following groups shown in a) to g):

a) hydrogen atom:

b) a saturated or unsaturated aliphatic hydrocarbon group having 1 to 10 carbon atoms which may be optionally substituted by the following substituents:
substituents:

i) a halogen atom;

ii) a hydroxyl group;

iii) an alkoxy group having 1 to 5 carbon atoms;

iv) $-COR^4$;
wherein $R^4$ is a hydroxyl group, an alkyl group having 1 to 5 carbon atoms which may be substituted by a halogen atom, a phenyl group which may be substituted by a halogen atom, an alkoxy group having 1 to 5carbon atoms, a alkenyloxy group having 1 to 5 carbon atoms or a group of the formula

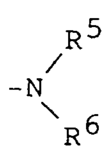

in which $R^5$ and $R^6$ are identical or different and each represents a hydrogen atom or an alkyl group having 1 to 5 carbon atoms;

v) a phenyl group;
wherein the phenyl group may be substituted by a halogen atom, a hydroxyl group, $-CF_3$, $-NO_2$, -CN, an alkyl group having 1 to 5 carbon atoms, an alkoxy group having 1 to 5 carbon atoms, $-COR_4$ or

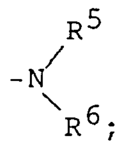

vi) a phenoxy group;
wherein the phenoxy group may be substituted by the groups cited in v), in addition to phenyl, phenoxy or pyridyloxy group (wherein the phenyl, phenoxy or pyridyloxy group may be substituted by halogen atom or $-CF_3$)
vii)

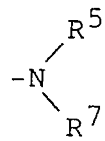

wherein $R^7$ is a hydrogen atom, an alkyl group having 1 to 5 carbon atoms or $-COR^4$;
viii) -CN;

c) an alkoxy group which may be substituted by the following substituents:
substituents:
i) a halogen atom;
ii) a phenyl group (wherein the phenyl group may be substituted by substituents v) in b);

d) a phenoxy group which may be substituted by the following substituents:
substituents: the same substituents as v) in b);

e) an aromatic hydrocarbon group having 6 to 20 carbon atoms which may be substituted by the following substituents:
substituents:
i) a halogen atom;
ii) a hydroxyl group;
iii) $-CF_3$;
iv) $-NO_2$;
v) -CN;
vi) an alkyl group having 1 to 5 carbon atoms;
vii) an alkoxy group having 1 to 5 carbon atoms;
viii) $-COR^4$;
ix)

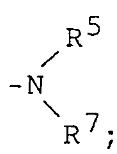

x) $-N^{+}R^5R^6R^8$
wherein $R^8$ is an alkyl group having 1 to 5 carbon atoms;
xi) a phenyl group
wherein the phenyl group may be substituted by the substituents v) in b);
xii) a phenoxy group
wherein the phenoxy group may be substituted by the substituents vi) in b);
xiii)-$CH_2COR^4$;

f) an aromatic heterocyclic group containing at least one nitrogen atom having 3 to 20 carbon atoms which may be substituted by the following substituents: substituents: the same substituents as shown in e);

g)

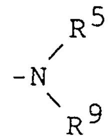

wherein $R^9$ is b), e) or f);
$R^3$ is a hydrogen atom ; or an aliphatic hydrocarbon group having 1 to 10 carbon atoms which may be substituted by a halogen atom, a hydroxyl group, an alkoxy group, $-COR^4$ or

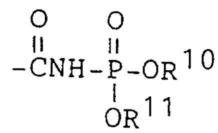

wherein $R^{10}$ and $R^{11}$ are identical or different and represent a hydrogen atom, an alkyl group having 1 to 5 carbon atoms, or a phenyl group;
Q represents a direct bond,

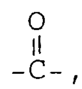 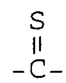 or 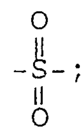

wherein in case that Q is a direct bond, $R^2$ is not c), d) or g);
or their salts are provided.

The oxime derivatives according to the present invention have a wide range of herbicidal spectrum, exhibiting excellent herbicidal activity against both broad-leaved weeds and narrow-leaved weeds. Further, the oxime derivatives according to the present invention can eradicate broad-leaved weeds and narrow-leaved weeds substantially without chemical injury to useful crops, especially soybeans and corns, accordingly without growth inhibition of these plants, by selecting application methods, treating methods and application rates optimally. The oxime derivatives according to the present invention develops herbicidal effect with a reduced amount of application and have quick-acting herbicidal activity.

The best embodiment of the invention:

The present invention will be illustrated in detail as follows.

In the general formula (I), X and Y are identical or different and each represents a hydrogen atom, a halogen atom, trifluoromethyl or an alkyl group having 1 to 5 carbon atoms.

The halogen atom is, for example, fluorine, chlorine or bromine. The alkyl group having 1 to 5 carbon atoms is straight-chained or branched, for example, methyl, ethyl, n-propyl, isopropyl, n-butyl, sec-butyl, iso-butyl, t-butyl, or n-pentyl. X and Y are identical or different and each preferably represents chlorine or trifluoromethyl.

In the formula (I), Z is =CH- or =N-.

In the formula (I), $R^1$ represents a hydrogen atom, a hydroxyl group, an alkyl group having 1 to 5 carbon atoms, or an alkoxy group having 1 to 5 carbon atoms.

The concrete examples having the alkyl group having 1 to 5 carbon atoms, prescribed here are the same as those cited in the alkyl group having 1 to 5 carbon atoms in context of X and Y.

The alkoxy group having 1 to 5 carbon atoms may be straight-chained or branched, and is, for example, methoxy, ethoxy, n-propoxy, iso-propoxy, n-butoxy, sec-butoxy, t-butoxy, n-pentoxy, etc.

$R^1$ is preferably a hydrogen atom.

In the formula (I), $R^2$ is a group selected from the groups shown in a) through g).

In this context, the aliphatic hydrocarbon group is a straight-chained or branched aliphatic hydrocarbon group and an alicyclic hydrocarbon group.

The concrete examples of the halogen atoms, alkyl groups having 1 to 5 carbon atoms, and the alkoxy groups having 1 to 5 carbon atoms are the same as those cited in the prescription of X, Y and $R^1$.

The alkenyloxy group having 2 to 5 carbon atoms is, for example, ethenoxy, propenoxy, butenoxy or pentenoxy.

The aromatic hydrocarbon group is benzene, naphthalene or their combinations.

The aromatic heterocyclic group has a structure of, for example, benzene or naphthalene in which at least one of carbon atom is replaced by an oxygen atom, a sulfur atom, a nitrogen atom or the like.

Additionally, $R^2$ is preferably, in case that Q is a direct bond, an aromatic hydrocarbon group having 6 to 20 carbon atoms which may be substituted by e) or an aromatic heterocyclic group having 3 to 20 carbon atoms, which contains at least one nitrogen atom and may be substituted by f), and is preferably a group other than a) a hydrogen atom, in case that

$$Q \text{ is } -\overset{\overset{\displaystyle O}{\|}}{C}-.$$

In $R^3$ of the formula (I), the aliphatic hydrocarbon group is the one which is substituted by a straight-chained or branched hydrocarbon group, an alicyclic hydrocarbon group and an aromatic hydrocarbon group. The concrete examples of the halogen atom, alkoxy group and $-COR^4$ are the same as those cited in $R^2$.

$R^3$ is preferably methyl, ethyl, propyl, butyl or other aliphatic chained hydrocarbon groups having 1 to 5 carbon atoms,

$$-CH_2-COR^4,\quad -\overset{\overset{\displaystyle CH_3}{|}}{C}H-COR^4,\quad -\overset{\overset{\displaystyle C_2H_5}{|}}{C}H-COR^4,$$

$$\text{particularly preferably}\quad -\overset{\overset{\displaystyle CH_3}{|}}{C}H-COR^{4'},\quad -\overset{\overset{\displaystyle C_2H_5}{|}}{C}H-COR^{4'},$$

wherein $R^{4'}$ represents a hydroxy, methoxy, ethoxy, propoxy or butoxy group.

In the formula (I), Q is a direct bond,

$$-\overset{\overset{\displaystyle O}{\|}}{C}-,\quad -\overset{\overset{\displaystyle S}{\|}}{C}-,\ \text{or}$$

$$-\underset{\underset{\displaystyle O}{\|}}{\overset{\overset{\displaystyle O}{\|}}{S}}-\ ,\ \text{and preferably a direct bond or}\ -\overset{\overset{\displaystyle O}{\|}}{C}-.$$

wherein, in case that Q is a direct bond, $R^2$ is not an alkoxy group which may be substituted by c), a phenoxy group which may be substituted by d) or

$$-N\begin{matrix} \diagup R^5 \\ \diagdown R^9 \end{matrix}.$$

Among the compounds of formula (I), the compounds selected from the following table have particularly excellent herbicidal activity

| | | |
|---|---|---|
| Q | a direct bond | 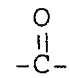 |
| X | Cl, $CF_3$ | Cl, $CF_3$ |
| Y | Cl, $CF_3$ | Cl, $CF_3$ |
| $R^1$ | H | H |
| $R^2$ | e) An aromatic hydrocarbon group having 6 - 20 carbon atoms which may be substituted<br>f) An aromatic heterocyclic group having 3 - 20 carbon atoms which contains at least one N atom and may be substituted | a) groups other than H atom |
| $R^3$ | 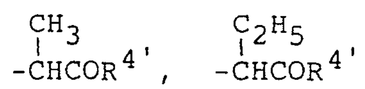 | 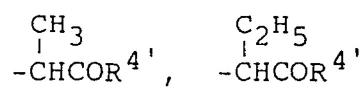 |

Examples of the oxime derivatives of formula (I) are shown in the following table.

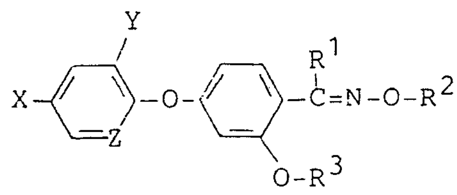

| No. | X | Y | Z | $R^1$ | $R^2$ | $R^3$ |
|---|---|---|---|---|---|---|
| (1B) | $-CF_3$ | -Cl | -CH= | -H | -H | $-C_2H_5$ |
| (2B) | $-CF_3$ | -Cl | -CH= | -H | -H | $-\overset{*}{C}H(CH_3)COOCH_3$ |
| (3B) | $-CF_3$ | -Cl | -CH= | -H | -H | $-\overset{*}{C}H(CH_3)COOC_2H_5$ |
| (4B) | $-CF_3$ | -Cl | -CH= | -H | $-CH_3$ | $-C_2H_5$ |
| (5B) | $-CF_3$ | -Cl | -CH= | -H | $-CH_3$ | $-\overset{*}{C}H(CH_3)COOCH_3$ |
| (6B) | $-CF_3$ | -Cl | -CH= | -H | $-C_2H_5$ | $-\overset{*}{C}H(CH_3)COOCH_3$ |
| (7B) | $-CF_3$ | -Cl | -CH= | -H | $-^{n}C_3H_7$ | $-\overset{*}{C}H(CH_3)COOCH_3$ |
| (8B) | $-CF_3$ | -Cl | -CH= | -H | $-^{iso}C_3H_7$ | $-\overset{*}{C}H(CH_3)COOCH_3$ |
| (9B) | $-CF_3$ | -Cl | -CH= | -H | -cyclohexyl | $-\overset{*}{C}H(CH_3)COOCH_3$ |
| (10B) | $-CF_3$ | -Cl | -CH= | -H | $-CH_2COOC_2H_5$ | $-\overset{*}{C}H(CH_3)COOCH_3$ |

| No. | X | Y | Z | $R^1$ | $R^2$ | $R^3$ |
|---|---|---|---|---|---|---|
| (11B) | $-CF_3$ | -Cl | -CH= | -H | $-CH_2-CH=CH_2$ | $-\overset{*}{C}HCOOCH_3$<br>$CH_3$ |
| (12B) | $-CF_3$ | -Cl | -CH= | -H | $-CH_2-CH=CH-CH_3$ | $-\overset{*}{C}HCOOCH_3$<br>$CH_3$ |
| (13B) | $-CF_3$ | -Cl | -CH= | -H | $-CH_2-CH_2-CH=CH_3$ | $-\overset{*}{C}HCOOCH_3$<br>$CH_3$ |
| (14B) | $-CF_3$ | -Cl | -CH= | -H | $-CH_2-CH=CH_2$ | $-\overset{*}{C}HCOOC_2H_5$<br>$CH_3$ |
| (15B) | $-CF_3$ | -Cl | -CH= | -H | $-CH_2-CH=CH_2$ | $-\overset{*}{C}HCOOCH_3$<br>$CH_3$ |
| (16B) | $-CF_3$ | -Cl | -CH= | $-CH_3$ | -H | $-\overset{*}{C}HCOOCH_3$<br>$CH_3$ |
| (17B) | $-CF_3$ | -Cl | -CH= | $-CH_3$ | $-CH_3$ | $-\overset{*}{C}HCOOCH_3$<br>$CH_3$ |
| (18B) | $-CF_3$ | -Cl | -CH= | -H | -benzyl | $-\overset{*}{C}HCOOCH_3$<br>$CH_3$ |
| (19B) | $-CF_3$ | -Cl | -CH= | -H | -phenyl | $-C_2H_5$ |
| (20B) | $-CF_3$ | -Cl | -CH= | -H | -phenyl | $-\overset{*}{C}HCOOCH_3$<br>$CH_3$ |
| (21B) | $-CF_3$ | -Cl | -CH= | -H | -4-fluorophenyl | $-\overset{*}{C}HCOOCH_3$<br>$CH_3$ |

| No. | X | Y | Z | $R^1$ | $R^2$ | $R^3$ |
|---|---|---|---|---|---|---|
| (22B) | $-CF_3$ | -Cl | -CH= | -H | -2-fluorophenyl | $-\overset{*}{C}HCOOCH_3$<br>$CH_3$ |
| (23B) | $-CF_3$ | -Cl | -CH= | -H | -3-fluorophenyl | $-\overset{*}{C}HCOOCH_3$<br>$CH_3$ |
| (24B) | $-CF_3$ | -Cl | -CH= | -H | -4-chlorophenyl | $-\overset{*}{C}HCOOCH_3$<br>CH3 |
| (25B) | $-CF_3$ | -Cl | -CH= | -H | -4-cyanophenyl | $-\overset{*}{C}HCOOCH_3$<br>$CH_3$ |
| (26B) | $-CF_3$ | -Cl | -CH= | -H | -4-nitrophenyl | $-\overset{*}{C}HCOOCH_3$<br>$CH_3$ |
| (27B) | $-CF_3$ | -Cl | -CH= | -H | -3-nitrophenyl | $-\overset{*}{C}HCOOCH_3$<br>$CH_3$ |
| (28B) | $-CF_3$ | -Cl | -CH= | -H | -3-trifluoro-methylphenyl | $-\overset{*}{C}HCOOCH_3$<br>$CH_3$ |
| (29B) | $-CF_3$ | -Cl | -CH= | -H | -3-methoxyphenyl | $-\overset{*}{C}HCOOCH_3$<br>$CH_3$ |
| (30B) | $-CF_3$ | -Cl | -CH= | -H | -4-methylphenyl | $-\overset{*}{C}HCOOCH_3$<br>$CH_3$ |
| (31B) | $-CF_3$ | -Cl | -CH= | -H | -4-phenylphenyl | $-\overset{*}{C}HCOOCH_3$<br>$CH_3$ |
| (32B) | $-CF_3$ | -Cl | -CH= | -H | -4-(4-trifluoro-methylphenoxy)-phenyl | $-\overset{*}{C}HCOOCH_3$<br>$CH_3$ |

| No. | X | Y | Z | $R^1$ | $R^2$ | $R^3$ |
|---|---|---|---|---|---|---|
| (33B) | $-CF_3$ | -Cl | -CH= | -H | -3,4-dichloro-phenyl | $-\underset{CH_3}{\overset{*}{C}}HCOOCH_3$ |
| (34B) | $-CF_3$ | -Cl | -CH= | -H | -2,4-dinitro-phenyl | $-\underset{CH_3}{\overset{*}{C}}HCOOCH_3$ |
| (35B) | $-CF_3$ | -Cl | -CH= | -H | -2-chloro-4-trifluoromethyl-phenyl | $-\underset{CH_3}{\overset{*}{C}}HCOOCH_3$ |
| (36B) | $-CF_3$ | -Cl | -CH= | -H | -3,5-dimethoxy-phenyl | $-\underset{CH_3}{\overset{*}{C}}HCOOCH_3$ |
| (37B) | $-CF_3$ | -Cl | -CH= | -H | -3,5-dimethyl-phenyl | $-\underset{CH_3}{\overset{*}{C}}HCOOCH_3$ |
| (38B) | -Cl | -Cl | -CH= | -H | -phenyl | $-\underset{CH_3}{\overset{*}{C}}HCOOCH_3$ |
| (39B) | -Cl | -Cl | -CH= | -H | -4-fluorophenyl | $-\underset{CH_3}{\overset{*}{C}}HCOOCH_3$ |
| (40B) | -Cl | -Cl | -CH= | -H | -4-nitrophenyl | $-\underset{CH_3}{\overset{*}{C}}HCOOCH_3$ |
| (41B) | -Cl | -Cl | -CH= | -H | -3-methoxyphenyl | $-\underset{CH_3}{\overset{*}{C}}HCOOCH_3$ |
| (42B) | -Cl | -Cl | -CH= | -H | -3-trifluoro-methylphenyl | $-\underset{CH_3}{\overset{*}{C}}HCOOCH_3$ |

| No. | X | Y | Z | $R^1$ | $R^2$ | $R^3$ |
|---|---|---|---|---|---|---|
| (43B) | -Cl | -Cl | -CH= | -H | -4-methylphenyl | $-\overset{*}{C}HCOOCH_3$<br>$\vert$<br>$CH_3$ |
| (44B) | -Cl | -Cl | -CH= | -H | -4-cyanophenyl | $-\overset{*}{C}HCOOCH_3$<br>$\vert$<br>$CH_3$ |
| (45B) | -Cl | -Cl | -CH= | -H | -2-chloro-4-trifluoromethyl-phenyl | $-\overset{*}{C}HCOOCH_3$<br>$\vert$<br>$CH_3$ |
| (46B) | $-CF_3$ | -H | -CH= | -H | -phenyl | $-\overset{*}{C}HCOOCH_3$<br>$\vert$<br>$CH_3$ |
| (47B) | $-CF_3$ | -H | -CH= | -H | -4-fluorophenyl | $-\overset{*}{C}HCOOCH_3$<br>$\vert$<br>$CH_3$ |
| (48B) | $-CF_3$ | -H | -CH= | -H | -4-chlorophenyl | $-\overset{*}{C}HCOOCH_3$<br>$\vert$<br>$CH_3$ |
| (49B) | $-CF_3$ | -H | -CH= | -H | -3-trifluoro-methylphenyl | $-\overset{*}{C}HCOOCH_3$<br>$\vert$<br>$CH_3$ |
| (50B) | $-CF_3$ | -Cl | -CH= | -H | -2-pyridyl | $-\overset{*}{C}HCOOCH_3$<br>$\vert$<br>$CH_3$ |
| (51B) | $-CF_3$ | -Cl | -CH= | -H | -6-methoxy-2-pyridyl | $-\overset{*}{C}HCOOCH_3$<br>$\vert$<br>$CH_3$ |
| (52B) | $-CF_3$ | -Cl | -CH= | -H | -3-chloro-2-pyridyl | $-\overset{*}{C}HCOOCH_3$<br>$\vert$<br>$CH_3$ |
| (53B) | $-CF_3$ | -Cl | -CH= | -H | -2-pyrazyl | $-\overset{*}{C}HCOOCH_3$<br>$\vert$<br>$CH_3$ |

| No. | X | Y | Z | $R^1$ | $R^2$ | $R^3$ |
|---|---|---|---|---|---|---|
| (54B) | $-CF_3$ | -Cl | -CH= | -H | -3,6-dimethyl-2-pyrazyl | *<br>$-CHCOOCH_3$<br>$CH_3$ |
| (55B) | $-CF_3$ | -Cl | -CH= | -H | -2-quinolyl | *<br>$-CHCOOCH_3$<br>$CH_3$ |
| (56B) | $-CF_3$ | -Cl | -CH= | -H | -6-chloro-3-pyridazyl | *<br>$-CHCOOCH_3$<br>$CH_3$ |
| (57B) | -Cl | -Cl | -CH= | -H | -6-chloro-2-pyridyl | *<br>$-CHCOOCH_3$<br>$CH_3$ |
| (58B) | -Cl | -Cl | -CH= | -H | -3,6-dimethyl-2-pyrazyl | *<br>$-CHCOOCH_3$<br>$CH_3$ |
| (59B) | $-CF_3$ | -H | -CH= | -H | -6-chloro-2-pyridyl | *<br>$-CHCOOCH_3$<br>$CH_3$ |
| (60B) | $-CF_3$ | -Cl | -CH= | -H | -6-methoxy-2-pyridyl | *<br>$-CHCOOCH_3$<br>$CH_3$ |
| (61B) | -Cl | -Cl | -CH= | -H | -3-chloro-2-pyridyl | *<br>$-CHCOOCH_3$<br>$CH_3$ |
| (62B) | -Cl | -H | -CH= | -H | -phenyl | *<br>$-CHCOOCH_3$<br>$CH_3$ |
| (63B) | $-CF_3$ | -Cl | -CH= | -H | -phenyl | *<br>$-CHCOOH$<br>$CH_3$ |
| (64B) | $-CF_3$ | -Cl | -CH= | -H | -phenyl | -H |

| No. | X | Y | Z | $R^1$ | $R^2$ | $R^3$ |
|---|---|---|---|---|---|---|
| (65B) | $-CF_3$ | -Cl | -N= | -H | -phenyl | *<br>$-CHCOOCH_3$<br>$CH_3$ |
| (66B) | $-CF_3$ | -Cl | -CH= | -H | $-CH_2-CH=CH_2$ | $-CH_2COOCH_3$ |
| (67B) | $-CF_3$ | -Cl | -CH= | -H | $-CH_2-CH=CH_2$ | $-CH_2COOC_2H_5$ |
| (68B) | $-CF_3$ | -Cl | -CH= | -H | -phenyl | $-CH_2COOCH_3$ |
| (69B) | $-CF_3$ | -Cl | -CH= | -H | -phenyl | $-CH_2COOC_2H_5$ |
| (70B) | $-CF_3$ | -Cl | -CH= | -H | $-CH_2-CH=CH_2$ | *<br>$-CHCOO^nC_4H_9$<br>$CH_3$ |
| (71B) | $-CF_3$ | -Cl | -CH= | -H | -phenyl | *<br>$-CHCOOC_2H_5$<br>$CH_3$ |
| (72B) | $-CF_3$ | -Cl | -CH= | -H | -phenyl | *<br>$-CHCOO^nC_3H_7$<br>$CH_3$ |
| (73B) | $-CF_3$ | -Cl | -CH= | -H | -H | *<br>$-CHCOOCH_3$<br>$C_5H_{11}$ |
| (74B) | $-CF_3$ | -Cl | -CH= | -H | -phenyl | *<br>$-CHCOO^nC_4H_9$<br>$CH_3$ |
| (75B) | $-CF_3$ | -Cl | -CH= | -H | -H | $-CH_2COOCH_3$ |
| (76B) | $-CF_3$ | -Cl | -CH= | -H | -phenyl | *<br>$-CHCONH^{iso}C_3H_7$<br>$CH_3$ |

| No. | X | Y | Z | $R^1$ | $R^2$ | $R^3$ |
|---|---|---|---|---|---|---|
| (77B) | $-CF_3$ | -Cl | -CH= | -H | -H | $-\overset{*}{C}HCOOCH_3$<br>$\vert$<br>$C_2H_5$ |
| (78B) | -Cl | -Cl | -CH= | -H | -H | $-\overset{*}{C}HCOOCH_3$<br>$\vert$<br>$CH_3$ |
| (79B) | $-CF_3$ | -Cl | -CH= | $-CH_3$ | -4-nitrophenyl | $-\overset{*}{C}HCOOCH_3$<br>$\vert$<br>$CH_3$ |
| (80B) | $-CF_3$ | -Cl | -CH= | -H | -4-nitrophenyl | $-\overset{*}{C}HCONH\overset{}{P}(OCH_3)_2$<br>$\vert$ ‖<br>$CH_3$ O |
| (81B) | $-CF_3$ | -Cl | -CH= | -H | -phenyl | $-\overset{*}{C}HCOOH_3$<br>$\vert$<br>phenyl |
| (82B) | $-CF_3$ | -Cl | -CH= | -H | -4-chloro-2-nitrophenyl | $-\overset{*}{C}HCOOCH_3$<br>$\vert$<br>$CH_3$ |
| (83B) | $-CF_3$ | -Cl | -CH= | -H | -2-nitrophenyl | $-\overset{*}{C}HCOOCH_3$<br>$\vert$<br>$CH_3$ |
| (84B) | $-CF_3$ | -Cl | -CH= | -H | -2-methylthio-pyrimidine | $-\overset{*}{C}HCOOCH_3$<br>$\vert$<br>$CH_3$ |
| (85B) | $-CF_3$ | -Cl | -CH= | -H | -4-nitrobenzyl | $-\overset{*}{C}HCOOCH_3$<br>$\vert$<br>$CH_3$ |
| (86B) | $-CF_3$ | -Cl | -N= | -H | -4-nitrophenyl | $-\overset{*}{C}HCOOCH_3$<br>$\vert$<br>$CH_3$ |
| (87B) | $-CF_3$ | -Cl | -N= | -H | -4-fluorophenyl | $-\overset{*}{C}HCOOCH_3$<br>$\vert$<br>$CH_3$ |

| No. | X | Y | Z | $R^1$ | $R^2$ | $R^3$ |
|---|---|---|---|---|---|---|
| (88B) | $-CF_3$ | -Cl | -N= | -H | -H | *<br>$-CHCOOCH_3$<br>$CH_3$ |
| (89B) | $-CF_3$ | -Cl | -CH= | -H | -1-nitro-2-naphthyl | *<br>$-CHCOOCH_3$<br>$CH_3$ |
| (90B) | $-CF_3$ | -Cl | -CH= | -H | -4-nitrophenyl | *<br>$-CHCONH_2$<br>$CH_3$ |
| (91B) | $-CF_3$ | -Cl | -CH= | -H | -4-(2,2-dimethyl-ethyl)phenyl | *<br>$-CHCOOCH_3$<br>$CH_3$ |
| (92B) | $-CF_3$ | -Cl | -CH= | $-OCH_3$ | $-CH_2COOCH_2CH=CH_2$ | *<br>$-CHCOOCH_3$<br>$CH_3$ |
| (93B) | $-CF_3$ | -Cl | -CH= | $-OCH_3$ | -4-nitrophenyl | *<br>$-CHCOOCH_3$<br>$CH_3$ |
| (94B) | $-CF_3$ | -Cl | -CH= | $-OCH_3$ | $-CH_2COOC_2H_5$ | *<br>$-CHCOOCH_3$<br>$CH_3$ |
| (95B) | $-CF_3$ | -Cl | -CH= | -H | -4-methoxy-carbonylmethyl-2-nitrophenyl | *<br>$-CHCOOCH_3$<br>$CH_3$ |
| (96B) | $-CF_3$ | -Cl | -CH= | -H | -4-methoxy-carbonylphenyl | *<br>$-CHCOOCH_3$<br>$CH_3$ |
| (97B) | $-CF_3$ | -Cl | -CH= | -H | -4-trifluoro-methylphenyl | *<br>$-CHCOOCH_3$<br>$CH_3$ |

| No. | X | Y | Z | $R^1$ | $R^2$ | $R^3$ |
| --- | --- | --- | --- | --- | --- | --- |
| (98B) | $-CF_3$ | -Cl | -CH= | -H | -4-nitrophenyl | $-CH_2COOCH_3$ |
| (99B) | $-CF_3$ | -Cl | -CH= | -H | -4-nitrophenyl | $-\overset{*}{C}HCOOCH_3$ with $C_2H_5$ on the starred C |
| (100B) | $-CF_3$ | -Cl | -CH= | -H | -4-nitrophenyl | $-\overset{*}{C}HCOOCH_3$ with $^nC_5H_{11}$ on the starred C |

| No. | X | Y | Z | $R^1$ | $R^2$ | $R^3$ |
|---|---|---|---|---|---|---|
| (143B) | $-CF_3$ | -Cl | -CH= | -H | 6-chloro-2-quinoxalyl | *<br>$-CHCOOCH_3$<br>$CH_3$ |
| (144B) | $-CF_3$ | -Cl | -CH= | -H | 4-acetylamino-phenyl | *<br>$-CHCOOCH_3$<br>$CH_3$ |
| (145B) | $-CF_3$ | -Cl | -CH= | $-CH_3$ | -H | *<br>$-CHCOOCH_3$<br>$CH_3$ |
| (146B) | $-CF_3$ | -Cl | -CH= | -H | 4-nitrophenyl | -H |
| (154B) | $-CF_3$ | -Cl | -CH= | -OH | 4-nitrophenyl | *<br>$-CHCOOCH_3$<br>$CH_3$ |
| (155B) | $-CF_3$ | -Cl | -CH= | -OH | $-CH_2COOC_2H_5$ | *<br>$-CHCOOCH_3$<br>$CH_3$ |
| (156B) | $-CF_3$ | -Cl | -CH= | -OH | $-CH_2COOCH_2CH=CH_2$ | *<br>$-CHCOOCH_3$<br>$CH_3$ |
| (157B) | $-CF_3$ | -Cl | -CH= | -H | -phenyl | *<br>$-CHCOO^{iso}C_3H_7$<br>$CH_3$ |
| (158B) | $-CF_3$ | -Cl | -CH= | -H | -phenyl | *<br>$-CHCONH_2$<br>$CH_3$ |
| (159B) | $-CF_3$ | -Cl | -CH= | -H | -phenyl | *<br>$-CHCON(CH_3)_2$<br>$CH_3$ |
| (160B) | $-CF_3$ | -Cl | -CH= | -H | -phenyl | *<br>$-CHCON(C_2H_5)_2$<br>$CH_3$ |

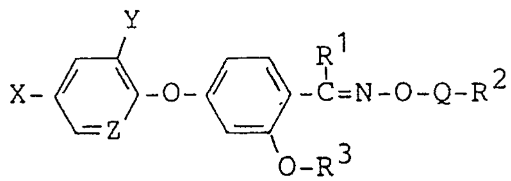

| No. | X | Y | Z | $R^1$ | $R^2$ | $R^3$ | Q |
|---|---|---|---|---|---|---|---|
| (101B) | $-CF_3$ | -Cl | -CH= | -H | -4-nitrophenyl | $-\overset{*}{C}HCOOCH_3$<br>$CH_3$ | $-\overset{O}{\overset{\|}{C}}-$ |
| (102B) | $-CF_3$ | -Cl | -CH= | -H | -3,5-dinitro-phenyl | $-\overset{*}{C}HCOOCH_3$<br>$CH_3$ | $-\overset{O}{\overset{\|}{C}}-$ |
| (103B) | $-CF_3$ | -Cl | -CH= | -H | -2,4-dinitro-phenyl | $-\overset{*}{C}HCOOCH_3$<br>$CH_3$ | $-\overset{O}{\overset{\|}{C}}-$ |
| (104B) | $-CF_3$ | -Cl | -CH= | -H | -4-fluorophenyl | $-\overset{*}{C}HCOOCH_3$<br>$CH_3$ | $-\overset{O}{\overset{\|}{C}}-$ |
| (105B) | $-CF_3$ | -Cl | -CH= | -H | -4-methoxy-carbonyl-2,3,5,6-tetra-chlorophenyl | $-\overset{*}{C}HCOOCH_3$<br>$CH_3$ | $-\overset{O}{\overset{\|}{C}}-$ |
| (106B) | $-CF_3$ | -Cl | -CH= | -H | 2-nitro-5-(2-chloro-4-trifluoromethyl-phenoxy)phenyl | $-\overset{*}{C}HCOOCH_3$<br>$CH_3$ | $-\overset{O}{\overset{\|}{C}}-$ |
| (107B) | $-CF_3$ | -Cl | -CH= | -H | -4-pyridyl | $-\overset{*}{C}HCOOCH_3$<br>$CH_3$ | $-\overset{O}{\overset{\|}{C}}-$ |
| (108B) | $-CF_3$ | -Cl | -CH= | $-CH_3$ | -4-nitrophenyl | $-\overset{*}{C}HCOOCH_3$<br>$CH_3$ | $-\overset{O}{\overset{\|}{C}}-$ |

| No. | X | Y | Z | $R^1$ | $R^2$ | $R^3$ | Q |
|---|---|---|---|---|---|---|---|
| (109B) | $-CF_3$ | -Cl | -CH= | -H | -4-(N,N-dimethyl amino)phenyl | $-\overset{*}{C}HCOOCH_3$<br>$CH_3$ | $-\overset{O}{\overset{\|}{C}}-$ |
| (110B) | $-CF_3$ | -Cl | -CH= | -H | -4-(N,N,N-tri-methylammonium iodo)phenyl | $-\overset{*}{C}HCOOCH_3$<br>$CH_3$ | $-\overset{O}{\overset{\|}{C}}-$ |
| (111B) | $-CF_3$ | -Cl | -CH= | -H | -4-trifluoro-methylphenyl | $-\overset{*}{C}HCOOCH_3$<br>$CH_3$ | $-\overset{O}{\overset{\|}{C}}-$ |
| (112B) | $-CF_3$ | -Cl | -CH= | -H | -4-cyanophenyl | $-\overset{*}{C}HCOOCH_3$<br>$CH_3$ | $-\overset{O}{\overset{\|}{C}}-$ |
| (113B) | $-CF_3$ | -Cl | -N= | -H | -4-nitrophenyl | $-\overset{*}{C}HCOOCH_3$<br>$CH_3$ | $-\overset{O}{\overset{\|}{C}}-$ |
| (114B) | $-CF_3$ | -Cl | -CH= | -H | -4-nitrophenyl | $-\overset{*}{C}HCOOCH_3$<br>$^nC_5H_{11}$ | $-\overset{O}{\overset{\|}{C}}-$ |
| (115B) | $-CF_3$ | -Cl | -CH= | -H | -4-nitrophenyl | $-\overset{*}{C}HCOOCH_3$<br>$C_2H_5$ | $-\overset{O}{\overset{\|}{C}}-$ |
| (116B) | $-CF_3$ | -Cl | -CH= | -H | -4-nitrophenyl | $-CH_2COOCH_3$ | $-\overset{O}{\overset{\|}{C}}-$ |
| (117B) | $-CF_3$ | -Cl | -CH= | -H | -4-phenoxy | $-\overset{*}{C}HCOOCH_3$<br>$CH_3$ | $-\overset{O}{\overset{\|}{C}}-$ |
| (118B) | $-CF_3$ | -Cl | -CH= | -H | -3,4-dichloro-anilino | $-\overset{*}{C}HCOOCH_3$<br>$CH_3$ | $-\overset{O}{\overset{\|}{C}}-$ |

| No. | X | Y | Z | $R^1$ | $R^2$ | $R^3$ | Q |
|---|---|---|---|---|---|---|---|
| (119B) | $-CF_3$ | -Cl | -CH= | -H | -4-chloro-anilino | *<br>$-CHCOOCH_3$<br>$CH_3$ | O<br>‖<br>-C- |
| (120B) | $-CF_3$ | -Cl | -CH= | -H | -3-chloro-anilino | *<br>$-CHCOOCH_3$<br>$CH_3$ | O<br>‖<br>-C- |
| (121B) | $-CF_3$ | -Cl | -CH= | -H | -4-fluoro-anilino | *<br>$-CHCOOCH_3$<br>$CH_3$ | O<br>‖<br>-C- |
| (122B) | $-CF_3$ | -Cl | -CH= | -H | -4-nitroanilino | *<br>$-CHCOOCH_3$<br>$CH_3$ | O<br>‖<br>-C- |
| (123B) | $-CF_3$ | -Cl | -CH= | -H | -4-chloroanilino | *<br>$-CHCOOCH_3$<br>$CH_3$ | S<br>‖<br>-C- |
| (124B) | $-CF_3$ | -Cl | -CH= | -H | -2,4-dichloro-phenyl | *<br>$-CHCOOCH_3$<br>$CH_3$ | O<br>‖<br>-C- |
| (125B) | $-CF_3$ | -Cl | -CH= | -H | -2,4-dichloro-phenoxymethyl | *<br>$-CHCOOCH_3$<br>$CH_3$ | O<br>‖<br>-C- |
| (126B) | $-CF_3$ | -Cl | -CH= | -H | 1-[4-(3-chloro-5-trifluoromethyl-2-pyridyloxy)phenoxy-ethyl | *<br>$-CHCOOCH_3$<br>$CH_3$ | O<br>‖<br>-C- |
| (127B) | $-CF_3$ | -Cl | -CH= | -H | $-CH_3$ | *<br>$-CHCOOCH_3$<br>$CH_3$ | O<br>‖<br>-C- |
| (128B) | $-CF_3$ | -Cl | -CH= | -H | 1-[4-(5-tri-fluoromethyl-2-pyridyloxy)phenoxy-ethyl | *<br>$-CHCOOCH_3$<br>$CH_3$ | O<br>‖<br>-C- |

| No. | X | Y | Z | $R^1$ | $R^2$ | $R^3$ | Q |
|---|---|---|---|---|---|---|---|
| (129B) | $-CF_3$ | -Cl | -CH= | -H | $-OC_2H_5$ | *<br>$-CHCOOCH_3$<br>$CH_3$ | O<br>‖<br>-C- |
| (130B) | $-CF_3$ | -Cl | -CH= | -H | $-{}^{n}C_9H_{19}$ | *<br>$-CHCOOCH_3$<br>$CH_3$ | O<br>‖<br>-C- |
| (131B) | $-CF_3$ | -Cl | -CH= | -H | $-CCl_2CH_3$ | *<br>$-CHCOOCH_3$<br>$CH_3$ | O<br>‖<br>-C- |
| (132B) | $-CF_3$ | -Cl | -N= | -H | $-CH_3$ | *<br>$-CHCOOCH_3$<br>$CH_3$ | O<br>‖<br>-C- |
| (133B) | $-CF_3$ | -Cl | -CH= | -H | $-OCH_2CCl_3$ | *<br>$-CHCOOCH_3$<br>$CH_3$ | O<br>‖<br>-C- |
| (134B) | $-CF_3$ | -Cl | -CH= | -H | $-NH-(CH_2)_3CH_3$ | *<br>$-CHCOOCH_3$<br>$CH_3$ | O<br>‖<br>-C- |
| (135B) | $-CF_3$ | -Cl | -CH= | -H | -3-aminophenyl | *<br>$-CHCOOCH_3$<br>$CH_3$ | O<br>‖<br>-C- |
| (136B) | $-CF_3$ | -Cl | -CH= | -H | -3-(N,N-dimethyl-carbamoylamino)-phenyl | *<br>$-CHCOOCH_3$<br>$CH_3$ | O<br>‖<br>-C- |
| (137B) | $-CF_3$ | -Cl | -CH= | -H | -4-ethoxycarbonyl-aminophenyl | *<br>$-CHCOOCH_3$<br>$CH_3$ | O<br>‖<br>C- |

| No. | X | Y | Z | $R^1$ | $R^2$ | $R^3$ | Q |
|---|---|---|---|---|---|---|---|
| (138B) | $-CF_3$ | -Cl | -CH= | -H | -phenyl | *<br>$-CHCOOCH_3$<br>$\vert$<br>$CH_3$ | O<br>‖<br>-C- |
| (139B) | $-CF_3$ | -Cl | -CH= | -H | -2,4,6-trimethyl-phenyl | *<br>$-CHCOOCH_3$<br>$\vert$<br>$CH_3$ | O<br>‖<br>-S-<br>‖<br>O |
| (140B) | $-CF_3$ | -Cl | -CH= | -H | 4-tert butyl-phenyl | *<br>$-CHCOOCH_3$<br>$\vert$<br>$CH_3$ | O<br>‖<br>-C- |
| (141B) | $-CF_3$ | -Cl | -CH= | -H | 4-chloroacetyl-aminophenyl | *<br>$-CHCOOCH_3$<br>$\vert$<br>$CH_3$ | O<br>‖<br>-C- |
| (142B) | $-CF_3$ | -Cl | -CH= | -H | 4-(1,1-dimethyl-propionylamino)-phenyl | *<br>$-CHCOOCH_3$<br>$\vert$<br>$CH_3$ | O<br>‖<br>-C- |

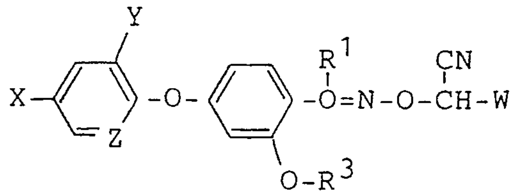

| No. | X | Y | Z | $R^1$ | W | $R^3$ |
|---|---|---|---|---|---|---|
| (147B) | $-CF_3$ | -Cl | -CH= | -H | 1,1-dimethyl-propionylamino | *<br>$-CHCOOCH_3$<br>$CH_3$ |
| (148B) | $-CF_3$ | -Cl | -CH= | -H | 4-nitrophenyl | *<br>$-CHCOOCH_3$<br>$CH_3$ |
| (149B) | $-CF_3$ | -Cl | -CH= | -H | 4-fluorobenzoyl-amino | *<br>$-CHCOOCH_3$<br>$CH_3$ |
| (150B) | $-CF_3$ | -Cl | -CH= | -H | ethenyl | *<br>$-CHCOOCH_3$<br>$CH_3$ |
| (151B) | $-CF_3$ | -Cl | -CH= | -H | ethoxycarbonyl-amino | *<br>$-CHCOOCH_3$<br>$CH_3$ |
| (152B) | $-CF_3$ | -Cl | -CH= | -H | trichloroacetyl-amino | *<br>$-CHCOOCH_3$<br>$CH_3$ |
| (153B) | $-CF_3$ | -Cl | -CH= | -H | 3-fluorobenzyl-amino | *<br>$-CHCOOCH_3$<br>$CH_3$ |

The compound of formula (I) can be produced, for example, by the processes shown by the following reaction schemes (i) and (ii). The processes are not limited only to them.

method (i)

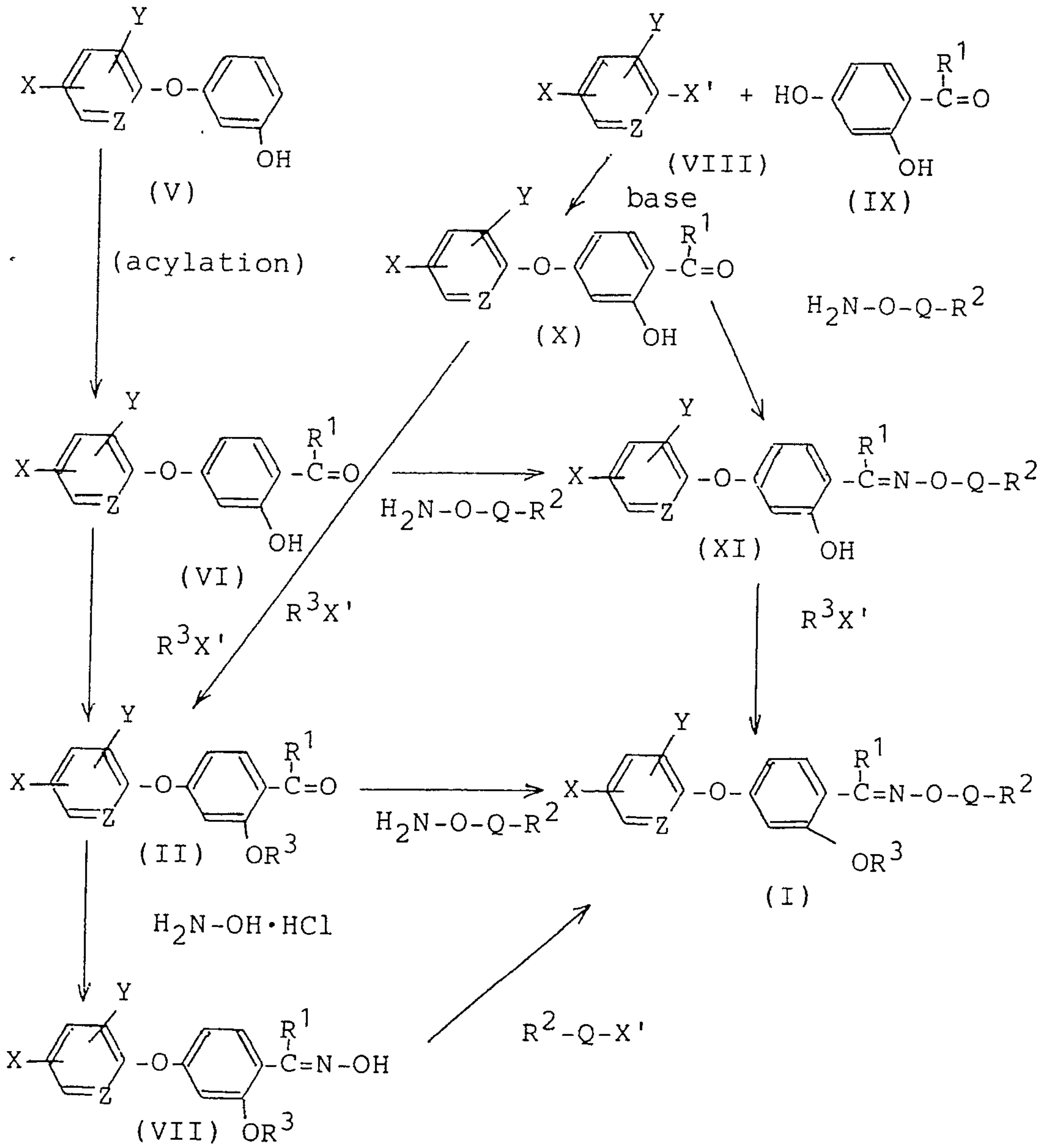
(V)
(VIII)
base
(IX)
(acylation)
(X)
$H_2N-O-Q-R^2$
(VI)
$H_2N-O-Q-R^2$
(XI)
$R^3X'$
$R^3X'$
$R^3X'$
(II)
$H_2N-O-Q-R^2$
(I)
$H_2N-OH \cdot HCl$
$R^2-Q-X'$
(VII)

method (ii)

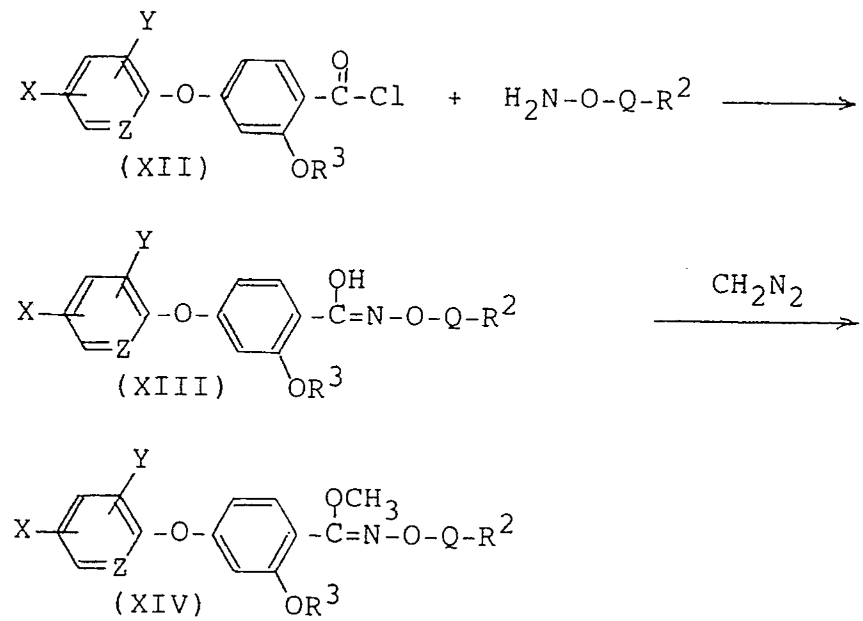


(XII) (XIII) (XIV)

method (iii)

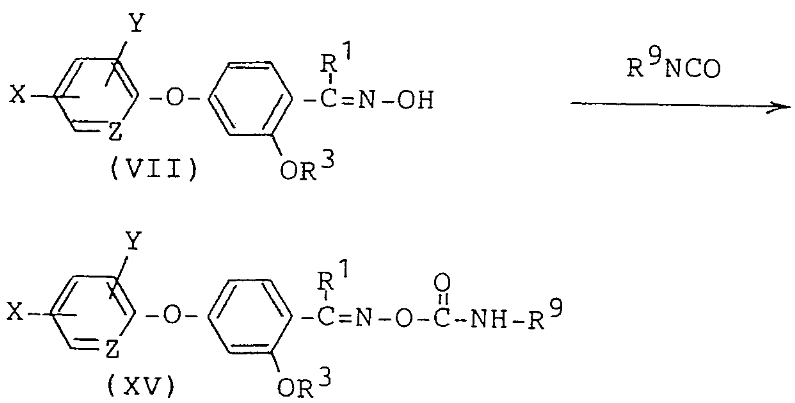


(VII) (XV)

method (iv)

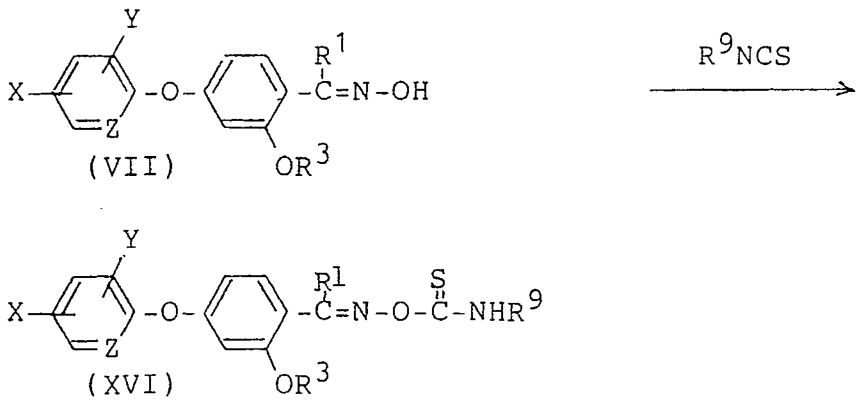


(VII)

(XVI)

wherein X, Y, Z, $R^1$ $R^2$ $R^3$, $R^9$ and Q are the same as in formula (I) and X′ means a halogen atom or $-OR^{40}$; wherein $R^{40}$ is an alkylsulfonyl or an arylsulfonyl.

Additionally, optically active compounds of formula (I) (R enantiomer) can be obtained by appropriate selection of $R^3$. For example, the compound R-enantiomer of (26B) is synthesized according to following reaction process (V) or (VI):

(V)

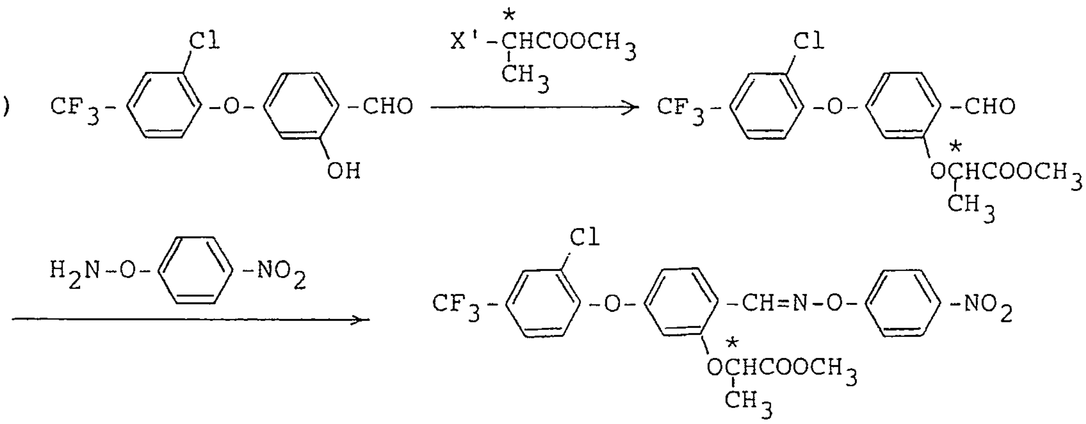


(VI)

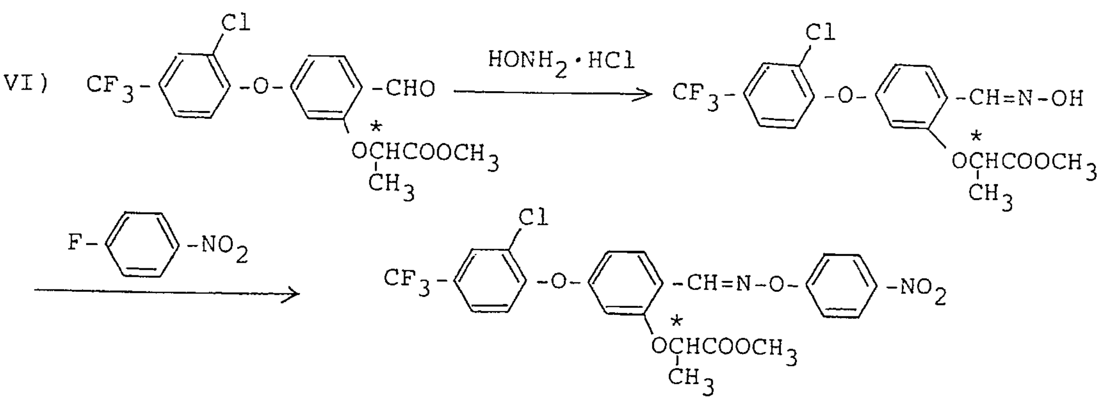


where, X′ represents halogen atom or $-OR^{40}$, herein $R^{40}$ is alkylsylfonyl or arylsulfonyl.

In the reaction processes (V) and (VI),

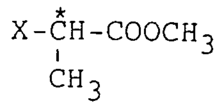

should be employed in the form of its S-enantiomer in the preparation of the R-enantiomer, because Walden inversion occurs in the asymmeric carbon during the reaction of the compound with a prescribed hydroxy compound. In the same way, a prescribed S-enantiomer is used, instead of

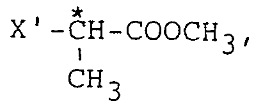

to effect the reaction with a prescribed hydroxy compound whereby a desired R-enantiomer is obtained. In addition, a variety of O-substituted hydroxylamines and a variety of halogen compounds relating to

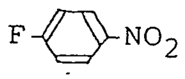 (p-fluoronitrobenzene)

can be suitably selected to obtain, the subject compounds of other types.

The formylation reaction in processes (i) and (ii) can be conducted by the Gattermann reaction in which, for example, hydrogen cyanide or zinc cyanide are used together with hydrogen chloride, if necessary, in the presence of aluminum trichloride or zinc chloride to produce a prescribed aldehyde derivative [see, for example, Organic Reactions, 9, 37 (1957); Ber., 31, 1149 (1898); Ber., 32, 284 (1899); Ann., 35, 313 (1907); J. Am. Chem. Soc., 45, 2373 (1932)], or the dichloromethyl alkyl ether method in which a dichloromethyl alkyl ether such as dichloromethyl methyl ether, dichloromethyl ethyl ether or dichloromethyl butyl ether is allowed to react with in the presence of titanium tetrachloride or aluminum trichloride followed by hydrolysis [see, for example, Chem. Ber., 93, 88 (1960); Ann., 662, 105 (1963); Chem. Ber., 96, 308 (1963)], or the Reimer-Tiemann reaction in which a formylation reagent such as chloroform, bromoform, trichloracetic acid or chloral hydrate is allowed to react in the presence of an alkali such as an alkali hydroxide, or an alkali carbonate [see, for example, Ber., 9, 423 (1876); J. Chem. Soc., 1929, 469; Organic Syntheses, III, 463 (1955)], or the orthoformate method in which an orthoformate such as methyl orthoformate or ethyl orthoformate is allowed to react with in the presence of aluminum trichloride, zinc chloride or titanium tetrachloride and the acetal produced is hydrolyzed to give a prescribed aldehyde [see, for example, Chem. Ber., 96, 308 (1963)], or the Duff method in which hexamethylenetetramine is allowed to react with in the presence of glycerol-boric acid or acetic acid-formic acid, and the Schiff base formed is hydrolyzed to produce the aldehyde [see, for example, J. Chem. Soc., 1945, 276], or the N-methylformanilide method in which a formyl group is introduced by allowing N-methylformanilide to react with in the presence of phosphorus oxychloride [see, for example, Ber., 60, 119 (1927)], or the dimethylformamide method in which dimethylformamide is used instead of the above-cited N-methylformanilide to introduce a formyl group [see, for example, J. Am. Chem. Soc., 75, 989 (1953)].

A benzaldehyde derivative of formula (II)

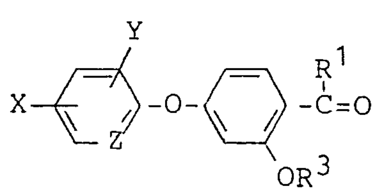

wherein

X, Y, Z, $R^1$, and $R^3$ have the same meanings as in formula (I), wherein, in case that Z is =CH-, $R^1$ is not an alkyl group having 1 to 5 carbon atoms, and $R^3$ is not an aliphatic hydrocarbon group having 1 to 10 carbon atoms which is substituted by $-COR^4$,

and its salt are used as an intermediate of a compound of formula (I).

The examples of benzaldehyde of formula (II) are shown in the following table.

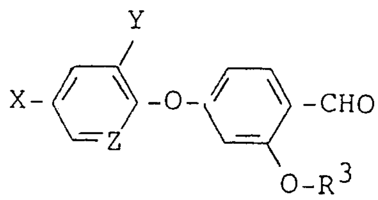

| No. | X | Y | Z | $R^3$ |
| --- | --- | --- | --- | --- |
| (1A) | $-CF_3$ | -Cl | -CH= | -H |
| (2A) | $-CF_3$ | -H | -CH= | -H |
| (3A) | -Cl | -H | -CH= | -H |
| (4A) | -Cl | -Cl | -CH= | -H |
| (5A) | $-CF_3$ | -Cl | -N= | -H |
| (6A) | $-CF_3$ | -H | -N= | -H |
| (7A) | $-CF_3$ | -Cl | -CH= | $-CH_3$ |
| (8A) | $-CF_3$ | -Cl | -CH= | $-C_2H_5$ |
| (9A) | $-CF_3$ | -Cl | -CH= | $-CH_2COOCH_3$ |
| (10A) | $-CF_3$ | -Cl | -CH= | $-CH_2COOC_2H_5$ |
| (11A) | $-CF_3$ | -Cl | -CH= | $-\overset{*}{C}H(CH_3)COOCH_3$ |
| (12A) | $-CF_3$ | -Cl | -CH= | $-\overset{*}{C}H(CH_3)COOC_2H_5$ |
| (13A) | $-CF_3$ | -Cl | -CH= | $-\overset{*}{C}H(CH_3)COO^nC_4H_9$ |

| No. | X | Y | Z | $R^3$ |
|---|---|---|---|---|
| (14A) | $-CF_3$ | -H | -CH= | $-\overset{*}{C}HCOOCH_3$<br>$CH_3$ |
| (15A) | -Cl | -H | -CH= | $-\overset{*}{C}HCOOCH_3$<br>$CH_3$ |
| (16A) | -Cl | -Cl | -CH= | $-\overset{*}{C}HCOOCH_3$<br>$CH_3$ |
| (17A) | $-CF_3$ | -Cl | -N= | $-\overset{*}{C}HCOOCH_3$<br>$CH_3$ |
| (18A) | $-CF_3$ | -H | -N= | $-\overset{*}{C}HCOOCH_3$<br>$CH_3$ |
| (19A) | $-CF_3$ | -Cl | -CH= | $-\overset{*}{C}HCOOCH_3$<br>$CH_3$ |
| (20A) | $-CF_3$ | -Cl | -CH= | $-\overset{*}{C}HCOOCH_3$<br>$C_2H_5$ |
| (21A) | $-CF_3$ | -Cl | -CH= | $-\overset{*}{C}HCOOCH_3$<br>$C_5H_{11}$ |
| (22A) | $-CF_3$ | -Cl | -CH= | $-\overset{*}{C}HCONH\overset{O}{\overset{\parallel}{P}}(OCH_3)_2$<br>$CH_3$ |

The oxime derivatives of formula (I) according to the present invention have the property of affecting the metabolism of plants, for example, to inhibit the growth of a certain kind of plants, regulate the growth of a certain kind of plants, dwarf a certain kind of plants, or kill a certain kind of plants.

The compounds of formula (I) according to the present invention can be applied to seeds of plants, as well as to plants in various growth stages through foliage and/or roots. In other words, the compounds provided by the present invention are applied, either as such or in the form of a composition, to the plants whose growth is to be inhibited, namely whose metabolism is to be regulated, to seeds of such plants, to a locus where such plants are growing or a locus where such plants are expected to grow in application amounts sufficient to control the metabolism of such plants.

The compounds according to the present invention can be used in the racemic modification, as well as in the form of R-enantiomers.

The compounds according to the present invention can control the metabolism of plants at a rate of, for example, 0.001 to 20 kg/ha, preferably 0.005 to 10 kg/ha, particularly preferably 0.01 to 5kg/ha.

The compounds according to the present invention can be used in a usual formulation such as a solution, an emulsifiable concentrate, a suspension, a dust, a paste or granules.

Such formulations are prepared by using at least one agriculturally acceptable diluent. Examples include solid carriers such as talc, bentonite, clay, kaolin, diatomaceous earth, white carbon, vermiculite, slaked lime, ammonium sulfate and urea; liquid carriers such as water, alcohols, dioxane, acetone, xylene,

cyclohexane, methylnaphthalene, dimethylformamide, N-methylpyrrolidone, dimethyl sulfoxide, cyclohexanone, methyl ethyl ketone, methyl isobutyl ketone; surfactant, emulsifiers or dispersants such as alkyl sulfate, alkylsulfonic acid salts, ligninsulfonic acid salts, polyoxyethylene glycol ethers, polyoxyethylene alkylaryl ethers, polyoxyethylene sorbitan monoalkylates and dinaphthylmethanedisulfonic acid salts; and a variety of adjuvants such as carboxymethyl cellulose and gum arabic.

For example, such a formulation can be prepared by mixing the compound according to the invention with the aforesaid carrier and/or emulsifier, etc.

The compound according to the invention may be present in a proportion of usually 0.01 to 99% by weight, preferably 0.1 to 96% by weight, in the formulation.

The compound according to the invention can be applied to plants in usual methods such as spraying, atomizing or dusting, as such or in the form of a mixture with other active compounds or in such a formulation as aforesaid.

When it is desired to inhibit the growth of, or eradicate hazardous plants, the compound according to the invention can be applied, either as such or in the form of a composition, to the plants or seeds directly or to the soil in an amount sufficient to inhibit the growth of or eradicate the hazardous plants in a locus where useful plants or their seeds and hazardous plants or their seeds are present together or likely to grow together.

The hazardous plants may be defined as plants which come into an environment created by man, such as a paddy or an upland farm, from the surrounding nature, and grow there and which are considered by man to be useless in that environment or do harm to it. Such hazardous plants are generally called weeds. Examples of the weeds are shown below.

<u>Amaranthaceae</u>
Amaranthus retroflexas
Amaranthus lividus
<u>Convolvulaceae</u>
Ipomoea purpurea
Cuscuta japonica
<u>Polygonaceae</u>
Polygonum convolvulus
Polygonum hydropiper
Polygonum lapathifolium
Rumex obtusifolius
Rumex crispus
Rumex bidens
<u>Chenopodiaceae</u>
Chenopodium album
Chenopodium album var. centrorubrum
Chenopodium ficifolium
<u>Cruciferae</u>
Brassica kaber
Capsella bursa-pastoris
Thlaspi arvense
Raphanus raphanistrum
<u>Portulacaceae</u>
Portulaca oleracea
<u>Oeguminosae</u>
Cassia obtusifolia
Desmodium tortuosum
Sesbania exaltata
Daubentonia texana
<u>Asclepiadaceae</u>
Asclepias syriaca
<u>Labiatae</u>
Lamium amplexicaule
<u>Malvaceae</u>
Abutilon theophrasti
Sida spinosa
<u>Solanaceae</u>

Solanum nigrum
Datula stramonium
Solanum carolinense
Plantaginaceae
Plantago lanceolata
Plantago major
Plantago rugelii
Compositae
Erigeron annuus
Ambrosia artemisiaefolia var. elator
Xanthium strumarium
Cirsium arvense var. etosum
Brdens pilosa
Helianthus sp.
Sonchus oleraceus
Matricaria chamomilla
Taraxacum officinale
Rubiaceae
Galium aparine
Gramineae
Sorghum halepense
Avena fatua
Digitaria adscendens
Setaria faberi
Agropyron repens
Panicum texanum
Echinochloa crus-galli
Setaria viridis
Poa annua
Eleusine indica
Axonopus affinis
Bachiaria platyphylla
Bromus tectorum
Cynodon dactylon
Panicum dichotomiflorum
Paspalum dilatatum
Echinochloa colona
Panicum capillare
Setaria faberi
Alopecurus acqualis var. amurensis
Dactyloctenium
Imperata cylindrica
Pennisetum alopecuroides
Alopecurus myosuroides
Bromus secalinus
Agrostis alba
Apera spica-venti
Avena ludoviciana
Bromus sterilis
Brachiaria eruciformis
Cenchrus pauciflorus
Digitaria ischaemum
Caryophyllaceae
Stellaria media
Euphorbiaceae
Euphorbia sp.
Scrophulariaceae
Veronica didyma

Cyperaceae
Cyperus rotundus
Ceperus microiria
Cyperus serotinus
Scirpus hotarui
Eleocharis acicularis var. longiseta
Alismataceae
Sagittaria pygmaea
Pontederiaceae
Monochoria vaginalis

Moreover, the compounds of formula (I) also can be used as a selective herbicide which can eradicate both broad-leaved weeds and narrow-leaved weeds substantially without chemical injury to useful crops, especially soybeans and corns, accordingly without growth inhibition of these plants, by selecting the application methods, treating methods and application rates.

For example, the compounds of the invention or compositions containing the compounds of the invention can be used as a selective herbicide which can not only eradicate the target weeds or inhibit their growth by foliar application, but also can inhibit germination and growth of the weeds by applying them before germination, substantially without inhibition of germination and growth of useful crops.

The compounds of formula (I) provided by this invention may be used in combination with various herbicidal compounds known per se. At this time, the compounds of formula (I) according to the invention are combined with another compounds showing excellent herbicidal activity against narrow-leaved weeds so that it may manifest satisfactorily herbicidal effects against both broad-leaved weeds and narrow-leaved weeds. Thus, there can be obtained a herbicidal composition which is effective against both broad-leaved weeds and narrow-leaved weeds.

Thus, the present invention provides a herbicidal composition comprising, as a herbicidal ingredient, a combination of the compound of formula (I) and an N-phosphonomethylglycine derivative of formula (III)

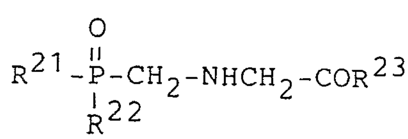

(III)

wherein
$R^{21}$ and $R^{22}$ are identical or different and each represents a hydroxyl or $-OR^{24}$,
$R^{23}$ represents a hydroxyl , $-OR^{24}$ or $-NR^{25}R^{26}$,
wherein
$R^{24}$ represents an alkyl group having 1 to 5 carbon atcms, a cyclohexyl group, a haloalkyl group having 1 to 5 carbon atoms, an alkenyl group having 2 to 5 carbon atoms or an alkoxyalkyl group or a haloalkoxyalkyl group or an alkoxyalkoxyalkyl group (wherein each of the alkoxy, haloalkoxy, and alkyl groups has 1 to 5 carbon atoms), and a phenoxy group;
$R^{25}$ and $R^{26}$ are identical or different and each represents a hydrogen atom, an alkyl group having 1 to 5 carbon atoms, a hydroxyalkyl group having 1 to 5 carbon atoms, an alkenyl group having 2 to 5 carbon atoms, or $R^{25}$ and $R^{26}$, together with the nitrogen atom to which they are attached, may form a morpholino group, a piperidino group or a pyrrolidino group,
and/or a glufosinate compound of formula (IV)

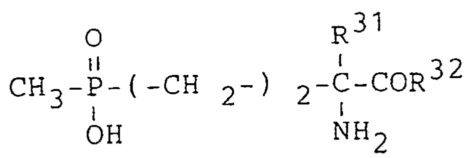

(IV)

wherein
$R^{31}$ is a hydrogen atom or an alkyl group having 1 to 4 carbon atoms,
$R^{32}$ is -OH, $-NH_2$, $-NHNH_2$, $-NHC_6H_5$ or an alkoxy group having 1 to 12 carbon atoms which may be substituted by -OH,

or its acid addition salt or a salt thereof with a base, additionally a carrier and/or a surfactant.

In the formula (III), $R^{21}$ and $R^{22}$ are identical or different and each represents -OH, or $-OR^{24}$,
$R^{23}$ represents -OH, $-OR^{24}$ or $-NR^{25}R^{26}$,
wherein
$R^{24}$ represents an alkyl group having 1 to 5 carbon atoms, a cyclohexyl group, a haloalkyl group having 1 to 5 carbon atoms, an alkenyl group having 2 to 5 carbon atoms or an alkoxyalkyl, haloalkoxyalkyl or alkoxyalkoxyalkyl group (wherein each of the alkoxy, haloalkoxy, and alkyl groups has 1 to 5 carbon atoms), and a phenoxy group,
$R^{25}$ and $R^{26}$ are identical or different and each represents a hydrogen atom, an alkyl group having 1 to 5 carbon atoms, a hydroxyalkyl group having 1 to 5 carbon atoms, and an alkenyl group having 2 to 5 carbon atoms, or
$R^{25}$ and $R^{26}$, together with the nitrogen atom to which they are attached, may form a morpholino group, a piperidino or a pyrrolidino group.

Examples of the alkyl group for $R^{24}$, $R^{25}$ and $R^{26}$ may be the same as those given hereinabove with regard to X and Y in formula (I).

Examples of the haloalkyl group having 1 to 5 carbon atoms for $R^{24}$ include halomethyl, haloethyl, dihaloethyl, halopropyl, halobutyl, and halopentyl groups. The halogen atoms may be, for example, fluorine, chlorine or bromine.

Examples of the alkenyl group having 2 to 5 carbon atoms for $R^{24}$, $R^{25}$ and $R^{26}$ include vinyl, propenyl, butenyl and pentenyl groups.

Examples of preferred alkoxyalkyl groups for $R^{24}$ are methoxymethyl and ethoxyethyl groups. Examples of preferred haloalkoxyalkyl groups for $R^{24}$ are chlorethoxyethyl and chloromethoxyethyl groups.

Examples of preferred alkoxyalkoxyalkyl groups for $R^{24}$ are methoxyethoxyethyl and ethoxyethoxyethyl groups.

Examples of preferred hydroxyalkyl groups having 1 to 5 carbon atoms for $R^{25}$ and $R^{26}$ are hydroxymethyl, hydroxyethyl and hydroxypentyl groups.

Compounds of formula (III) are disclosed in the Japanese Patent Published Specification No.6401/(1981) (U.S. Patent No. 7,123,057) and are known per se.

The compound of formula (III) also may be used in the form of its acid addition salt or its salt with a base in the composition of this invention.

Strong acids with a pKa of, for example, not more than 2.5 are preferred as acids for forming the acid adducts. As such an acid, are cited hydrochloric acid, sulfuric acid, phosphoric acid, trifluoroacetic acid, and trichloroacetic acid.

The salt with a base is formed, for example, as a salt with a cation such as an alkali metal, an alkaline earth metal, copper, zinc, ammonium or an organic ammonium or trimethylsulfonium, triethylsulfonium, tripropylsulfonium, trimethylsulfoxonium, triethylsulfoxonium, tripropylsulfoxonium, when at least one of $R^{21}$, $R^{22}$ and $R^{23}$ represent -OH.

The alkali metal represents, for example, lithium, sodium and potassium, and the alkaline earth metal represents, for example, magnesium and calcium.

The organic ammonium salt is produced from an organic amine having a low molecular weight of less than about 300.

Examples of the organic amine include alkylamines, alkylenepolyamines, and alkanolamines, such as methylamine, ethylamine, n-propylamine, isopropylamine, n-butylamine, isobutylamine, secondary butylamine, n-amylamine, isoamylamine, hexylamine, heptylamine, octylamine, nonylamine, decylamine, undecylamine, dodecylamine, tridecylamine, tetradecylamine, pentadecylamine, hexadecylamine, heptadecylamine, octadecylamine, methylethylamine, methylisopropylamine, methylhexylamine, methylnonylamine, methylpentadecylamine, methyloctadecylamine, ethylbutylamine, ethylheptylamine, ethyloctylamine, hexylheptylamine, hexyloctylamine, dimethylamine, diethylamine, di-n-propylamine, diisopropylamine, di-n-amylamine, di-isoamylamine, dihexylamine, diheptylamine, dioctylamine, trimethylamine, triethylamine, tri-n-propylamine, tri-isopropylamine, tri-n-butylamine, tri-isobutylamine, trisecondary butylamine, tri-n-amylamine, ethanolamine, n-propanolamine, isopropanolamine, diethanolamine, N,N-diethylethanolamine, N-ethylethanolamine, N-butylethanolamine, allylamine, n-butenyl-2-amine, n-pentenyl-2-amine, 2,3-dimethylbutenyl-2-amine, di-butenyl-2- amine, n-hexenyl-2-amine, and propylenediamine, primary arylamines, such as aniline, methoxyaniline, ethoxyaniline, o, m, p-toluidine, phenylenediamine, 2,4,6-tribromoaniline, benzidine, naphthylamine, and o, m, p-chloroaniline; and heterocyclic amines such as pyridine, morpholine, piperidine, pyrrolidine, indoline, and azepine.

Preferred compounds of formula (II) are those in which one or two of $R^{21}$, $R^{22}$, and $R^{23}$ are -OH, -OH salt or $-OR^{24}$, and the remainder of $R^{21}$, $R^{22}$ and $R^{23}$ is -OH or its salt.

There can also be cited compounds of formula (III) in which one or two of $R^{21}$, $R^{22}$ and $R^{23}$ are salts of -OH, and the remainder of $R^{21}$, $R^{22}$ and $R^{23}$ is -OH. Examples of the salts of -OH are ammonium or organic ammonium salts in which the organic ammonium group is selected from monoalkylammonium, dialkylammonium, trialkylammonium, monoalkenylammonium, dialkenylammonium, trialkenylammonium, monoalkynylammonium, dialkynylammonium, trialkynylammonium, monoalkanolammonium, dialkanolammonium, trialkanolammonium, heterocyclic ammonium and arylammonium, and contains 1 to 18 carbon atoms.

The salts of the compounds of formula (III) with acids or bases can be produced by methods known per se from the compounds of formula (III) with the acids or bases.

Examples of the compounds of formula (III) and their acid addition salts or their salts with bases are shown below.

(101) N-phosphonomethylglycine.
(102) N-phosphonodimethylglycine sodium salt.
(103) N-phosphonomethylglycine ammonium salt.
(104) N-phosphonomethylglycine calcium salt monohydrate.
(105) N-phosphonomethylglycine magnesium salt.
(106) N-phosphonomethylglycine potassium salt.
(107) N-phosphonomethylglycine dimethylammonium salt.
(108) N-phosphonomethylglycine copper salt.
(109) N-phosphonomethylglycine zinc salt.
(110) N-phosphonomethylglycinamide.
(111) methyl N-phosphonomethylglycinate.
(112) ethyl N-phosphonomethylglycinate.
(113) n-propyl N-phosphonomethylglycinate.
(114) n-butyl N-phosphonomethylglycinate.
(115) cyclohexyl N-phosphonomethylglycinate.
(116) chloroethyl N-phosphonomethylglycinate.
(117) N-phosphonomethylglycine isopropylammonium salt.
(118) N-phosphonomethylglycine methylammonium salt.
(119) N-phosphonomethylglycine diisopropylammonium salt.
(120) N-phosphonomethylglycine pyridinium salt.
(121) N-phosphonomethylglycine anilinium salt.
(122) N-phosphonomethylglycine trimethylsulfonium salt.
(123) N-phosphonomethylglycine trimethylsulfoxonium salt.

In the formula (IV), $R^{31}$ is a hydrogen atom or an alkyl group having 1 to 4 carbon atoms,

$R^{32}$ is -OH, $-NH_2$, $-NHNH_2$, $-NHC_6H_5$ or an alkoxy group having 1 to 12 carbon atoms which may be substituted by -OH.

Examples of the alkyl group for $R^{31}$ may be alkyl groups having 1 to 4 carbon atoms among the alkyl groups exemplified for X and Y in formula (I).

The alkoxy group for $R^{32}$ may be straight-chained or branched, and includes, for example, methoxy, ethoxy, n-propoxy, iso-propoxy, n-butoxy, sec-butoxy, iso-butoxy, tert-butoxy, n-pentoxy, n-hexoxy, n-heptoxy, n-octoxy, n-nonanoxy, n-decanoxy, n-undecanoxy, and dodecanoxy groups. These alkoxy groups may be substituted by -OH, thus, for example, $R^{32}$ may represent a hydroxyethoxy group.

Compound of formula (IV) are disclosed in Japanese Patent Publication No. 26564/1982 (U. S. Patent No. 4,168,963) and believed to be known.

The compound of formula (IV) may be used in the composition of this invention, as an acid addition salt or a salt with a base.

Examples of acids for forming the acid addition salts may be those exemplified hereinabove with regard to the acid addition salts of the compound of formula (IV). It is believed that the acid addition salts are formed at the primary amino group in formula (IV).

Examples of the bases for forming the salt may be those exemplified above with regard to the compounds of formula (III).

In the formula (IV), $R^{31}$ is preferably a hydrogen atom and $R^{32}$ is preferably -OH, $-NH_2$, $-NHNH_2$, an alkoxy group having 1 to 4 carbon atoms, and a hydroxyalkoxy group having 2 to 4 carbon atoms.

Examples of preferred salts of the compounds of formula (IV) include salts with Na, K, Cu, Mg, Ca, Zn, Ni, Mn or Fe, ammonium salts, salts with bases such as mono-, di-, or tri-alklylamine having 1 to 4 carbon atoms in each alkyl moiety, or aniline, acid adducts with acids such as hydrochloric acid, sulfuric acid, hydrobromic acid, chloric acid, or oxalic acid.

The salts of the compounds of formula (IV) with the acids or bases may be produced by known

methods from the compounds of formula (III) and the acids or bases.

Examples of the compounds of formula (IV) and their acid adducts or their salts with bases which are preferably used in this invention are shown below.

(500) [(3-amino-3-carboxy)-propyl-1]-methyl-phosphinic acid
(501) [(3-amino-3-carboxy)-propyl-1]-methyl-phosphinic acid monosodium salt.
(502) [(3-amino-3-carboxy)-propyl-1]-methyl-phosphinic acid monopotassium salt.
(503) [(3-amino-3-carboxy)-propyl-1]-methyl-phosphinic acid monoammonium salt.
(504) [(3-amino-3-carboxy)-propyl-1]-methyl-phosphinic acid diammonium salt.
(505) [(3-amino-3-carboxy)-propyl-1]-methyl-phosphinic acid magnesium salt.
(506) [(3-amino-3-carboxy)-propyl-1]-methyl-phosphinic acid monopropylammonium salt.
(507) [(3-amino-3-carboxy)-propyl-1]-methyl-phosphinic acid mono(diisopropylammonium) salt.
(508) [(3-amino-3-carbomethoxy)-propyl-1]-methyl-phosphinic acid.
(509) [(3-amino-3-carbomethoxy)-propyl-1]-methyl-phosphinic acid sodium salt.
(510) [(3-amino-3-carbomethoxy)-propyl-1]-methyl-phosphinic acid diisopropylammonium salt.
(511) [(3-amino-3-carbamido)-propyl-1]-methyl-phosphinic acid.
(512) [(3-amino-3-carbamido)-propyl-1]-methyl-phosphinic acid sodium salt.
(513) [(3-amino-3-carbamido)-propyl-1]-methyl-phosphinic acid ammonium salt.
(514) [(3-amino-3-methyl-3-carboxy)-propyl-1]-methyl-phosphinic acid.
(515) [(3-amino-3-methyl-3-carboxy)-propyl-1]-methyl-phosphinic acid monosodium salt.
(516) [(3-amino-3-methyl-3-carboxy)-propyl-1]-methyl-phosphinic acid monoammonium salt.

Bialaphos also may be used as a similar compound of formula (IV) for mixing with the compound of formula (I). Further, a triazine herbicide (simazine, atrazine, cyanazine, simetryn, prometrin or metribuzin) can be mixed with the compound of formula (I) of the invention in addition to the compounds of formulas (III) and (IV).

The composition according to the present invention contains the compound of formula (I) and other herbicides in a (I) : the other compounds weight ratio of from 1 : 500 to 500 : 1, more preferably from 1 : 200 to 200 :1, especially preferably from 1 : 100 to 100 : 1.

The amount of the composition to be actually applied varies depending upon many factors, for example, the type of a plant whose growth is to be controlled. Suitably, it is generally 0.01 to 10 kg/ha, preferably 0.05 to 5 kg/ha. Any one skilled in the art can easily determine the suitable proportions and amount of the composition by an ordinary standardized test without conducting many experiments.

The composition of this invention may be applied in the form of a composition comprising the active ingredient and a carrier of a solid or liquid diluent. The composition may also include an additive such as surfactant. Examples of such diluents, carriers and surfactants may be those which have been cited hereinabove.

The composition of this invention can be used as a usual formulation, for example, as a solution, an emusifiable concentrate, a suspension, a dust or a paste in combination with a carrier and/or a surfactant.

The composition of this invention can be prepared, for example, by mixing the compound (I) and the other herbicidal compounds and then with a carrier or other additives, and formulating the resulting mixture, or by separately preparing a composition comprising the compound (I) and another composition comprising the other herbicidal compositions, adding a carrier or the like as required, then mixing these compositions, and formulating the mixture.

According to the present invention, there is also provided a method of eradicating weeds, which comprises applying the compound of formula (I) and the other herbicidal compounds, simultaneously or successively, to a locus where weeds are growing in an amount effective for eradicating the weeds.

In this method, the compound of formula (I) and the other herbicidal compounds may be applied simultaneously to the aforesaid locus, as the composition comprising these compounds, or as the composition of the compound (I) and the composition of the other herbicidal compounds separately prepared.

Alternatively, the composition of the compound (I) and the composition of the other herbicidal compounds, separately prepared, may be applied to the aforesaid locus successively.

The sequence of application of the composition of the compound (I) and the composition of the other herbicidal compounds is not limited.

After one of the compositions is applied, the other may preferably be applied, while the active compound or compounds still remain on the foliage of the weeds. Usually, after one of the composition is applied, the other may preferably be applied immediately or within 2 to 3 days, although this period may vary depending upon the type of the plant to be controlled, the climatic conditions, etc.

According to the present invention, the compound of formula (I) and the other herbicidal compounds, for

example, may be applied to a locus where a crop is cultivated before emergence of the crop. As a result, weeds growing in the locus before emergence of the crop can be killed.

The amount of the composition to be applied is determined suitably as a measure of the amount of these compounds described in the compositions.

Advantageously, according to the method of this invention, both broad-leaved weeds and narrow-leaved weeds can be eradicated at a low application rate.

Moreover, the compounds of formula (I) according to the present invention also can be applied in combination with a variety of pesticides such as insecticides or herbicides which are known per se. Examples of such insecticides include organophosphate insecticipdes such as parathion, fenitrothion, diazinon, or fenthion; carbamate insecticides such as carbaryl, dioxacarb, carbanolate; pyrethroid insecticides such as permethrin, allethrin, fenvalerate, cypermethrin, bifenthrin, flucythrinate, cyhalothrin or tefluthrin; diphenyl insecticides such as DDT, methoxychlor, dicofol, chlorobenzilate, or bromopropylate; additionally, cyhexatin, lindane, dinochlor, chlordane and others.

In addition to triazine herbicides, glyphosate and glufosinate aforementioned, following herbicides also can be combined:
cyclohexadione herbicides such as alloxydim, sethoxydim, cloproxydim or cycloxydim; phenoxyacetic acid herbicides such as fluazifop, dichlofop, guinofop, poppenate, trifopsime, haloxyfop, chlorazifop, fenoxaprop, fenthiaprop, 2,4-D or MCP; benzoic acid herbicides such as 2,3,6-TBA, dinoben, Chloramben, dicamba, or chlorthal; carbamate herbicides such as chlorpropham, phenmedipham, asulam, molinate, benthiocarb, or ethiolate; amide herbicides such as propanil, naptalam, allidochlor, metolachlor, alachlor or butachlor; nitrile herbicides such as bromofenoxim, ioxynil or dichlobenil; urea herbicides such as diuron, linuron, fenuron or benzthiazuron; diphenyl ether herbicides such as nitrofen, acifluorfen or chlomethoxynil; further diquat, praquat, trifluralin or dinoseb.

The following examples illustrate the present invention in greater detail.

In these examples, all parts means parts by weight, unless otherwise noted. The herbicidal activity was evaluated on a scale of 6 degrees. In other words, 0 means the state where the plants are as sound as before the application of the active compound, while 5 is the state where the plants are withered or killed by application of the active compound, and 1, 2, 3 and 4 mean varying degrees of the enfeebled state of the plants between 0 and 5.

Example 1A

Synthesis of 2-Hydroxy-4-(2-chloro-4-trifluoromethylphenoxy)benzaldehyde (1A)

3-(2-Chloro-4-trifluoromethylphenoxy)phenol (52 parts) was dissolved in dichloromethane (900 parts by volume) and $TiCl_4$ (39.6 parts by volume) was added to the solution, as they were cooled with ice. Then, dichloromethyl methyl ether (34.1 parts) was added dropwise, as the reaction temperature was kept at about 0 °C. After completion of addition, stirring was continued for 15 minutes, then 5 % HCl (113 parts by volume) was added cautiously. The reaction mixture was extracted with ether, the organic phase was washed with water, dried over anyhydrous sodium sulfate, evaporated under reduced pressure. The residue was purified by silica-gel chromatography to give the subject compound (1A) (42 parts). The product melted at 37 to 40 °C. The IR and NMR spectra data are given in Table 1A.

Examples 2A through 5A

Syntheses of compounds (2A) through (5A)

Instead of 3-(2-chloro-4-trifluoromethylphenoxy)phenol in Example 1A, phenol derivatives shown in Table 2A were used in the same molar amount to react and treated as in Example 1A. The yields of products are given in Table 2A and the IR and NMR spectral data are shown in Table 1A.

Table 2A

| Example No. | Phenol derivative | Compound No. | Yield (parts) |
|---|---|---|---|
| 2A | 3-(4-Trifluoromethylphenoxy)phenol | (2A) | 35 |
| 3A | 3-(4-Chlorophenoxy)phenol | (3A) | 32 |
| 4A | 3-(2,4-Dichlorophenoxy)phenol | (4A) | 40 |
| 5A | 3-(3-Chloro-5-trifluoromethyl-2-pyridyloxy)phenol | (5A) | 30 |

Example 6A

Synthesis of 2-ethoxy-4-(2-chloro-4-trifluoromethylphenoxy)benzaldehyde (8A)

A mixture of the compound synthesized in Example 1A (2.0 parts), ethyl iodide (1.0 part), anhydrous potassium carbonate (1.7 part), and methyl ethyl ketone (30 parts by volume) was refluxed for about 5 hours. The reaction mixture was filtered, then the filtrate was washed with water, dried over anhydrous sodium sulfate, and concentrated under reduced pressure. The crude product was purified by silica gel chromatography to give the subject compound 8A (1.8 part). The IR and NMR spectral data are given in Table 1A.

Example 7A

Synthesis of 2-methoxycarbonylmethoxy-4-(2-chloro-4-trifluoromethylphenoxy)benzaldehyde (9A)

The compound obtained in Example 1A (1.5 part) was mixed with methyl α-bromoacetate (0.73 part), anhydrous potassium carbonate (1.3 part), and methyl ethyl ketone (25 parts by volume), and the mixture was refluxed for about 2 hours. After filtration of the reaction mixture, the filtrate was washed with water, dried over anhydrous sodium sulfate, and concentrated under reduced pressure. The crude product was purified on silica gel chromatography to give the subject compound (9A) (1.6 part). The IR and NMR spectral data are shown in Table 1A.

Example 8A

Synthesis of 2-ethoxycarbonylmethoxy-4-(2-chloro-4-trifluoromethylphenoxy)benzaldehyde (10A)

Example 7A was repeated except that ethyl α-bromoacetate (0.79 part) was used instead of methyl α-bromoacetate. The yield of the compound (10A) was 1.7 part. The IR and NMR spectral data are shown in Table 1A.

Example 9A

Synthesis of 2-(1-methoxycarbonylethoxy)-4-(2-chloro-4-trifluoromethylphenoxy)benzaldehyde (11A)

The compound obtained in Example 1A (8.2 part) was mixed with methyl α-bromopropionate (5.2 part), anhydrous potassium carbonate (7.2 part), and methyl ethyl ketone (100parts by volume), and the mixture was refluxed for about 3 hours. After filtration of the reaction mixture, the filtrate was concentrated under reduced pressure, the crude product was purified by silica gel chromatography to give the subject compound (11A) (9.5 part). The melting point of the product was 77 to 79 °C and the IR and NMR spectral data are shown in Table 1A.

Example 10A

Synthesis of 2-(1-ethoxycarbonylethoxy)-4-(2-chloro-4-trifluoromethylphenoxy)benzaldehyde (12A)

Example 9A was repeated except that ethyl α-bromopropionate (5.6 parts) was used instead of methyl

α-bromopropionate. The yield of the compound (12A) was 9.6 parts. The IR and NMR spectral data are shown in Table 1A.

Example 11A

Synthesis of 2-(1-n-butoxycarbonylethoxy)-4-(2-chloro-4-trifluoromethylphenoxy)benzaldehyde (13A)

Example 9A was repeated except that n-butyl α-bromopropionate (6.5 parts) was used instead of methyl α-bromopropionate. The yield of the compound (13A) was 9.7 parts. The IR and NMR spectral data are shown in Table 1A.

Examples 12A through 15A

syntheses of compounds (12A) through (15A)

Example 9A was repeated except that aldehyde derivatives of (2A) through (5A) were used instead of 2-hydroxy-4-(2-chloro-4-trifluoromethylphenoxy) benzaldehyde (1A) in the same molar amount as in (1A). The reaction yields are shown in Table 3A. The IR and NMR spectral data are given in Table 1A.

Table 3A

| Examples No. | Aldehyde derivatives | Compounds No. | Yield (parts) |
|---|---|---|---|
| 12A | (2A) | (14A) | 8.8 |
| 13A | (3A) | (15A) | 7.9 |
| 14A | (4A) | (16A) | 8.5 |
| 15A | (5A) | (17A) | 9.4 |

Example 16A

Synthesis of 2-(1-carboxyethoxy)-4-(2-chloro-4-trifluoromethylphenoxy)benzaldehyde (19A)

The compound (11A) synthesized in Example 9A (0.5 part) was dissolved in a mixed solvent of methanol (6 parts by volume) and water (0.4 part by volume), the solution was combined with 1N KOH (2.48 parts by volume) and allowed to stand 1 overnight at room temperature. Methanol is removed under reduced pressure, the residue was acidified with hydrochloric acid and the liberating product was taken into ether. The organic layer was washed with water, dried over anhydrous sodium sulfate, and concentrated under reduced pressure to give the subject compound (19A) (0.4 part). The IR and NMR spectral data are shown in Table 1A.

Example 17A

Synthesis of compound (5A)

2-3-Dichloro-5-trifluoromethylpyridine (2.16 parts), 2,4-dihydroxybenzaldehyde (1.41 part), and dimethylsulfoxide (10 parts by volume) were mixed, combined with 60% strength sodium hydride (0.42 part), stirred at 120°C for 5 hours, cooled down to room temperature, then poured into ice water, and extracted with diethyl ether. The organic phase is dried over anhydrous sodium sulfate, the ether is evaporated under reduced pressure, the residue was purified by silica-gel chromatography to give the subject compound (5A) (2.5 parts). The IR and NMR spectral data are shown in Table 1A.

Examples 18A through 20A

Syntheses of compounds (20A) through (22A)

The subject compounds were obtained by using halogen compounds described in the following Table 4A instead of methyl α-bromopropionate in Example 9A. The IR and NMR spectral data of these products are given in Table 1A.

Example 21A

Preparation of the R-enantiomer of compound (11A)

A solution of p-toluenesulfonyl chloride (38.2 parts) was added to a solution of methyl (s)-(-)-lactate (20.8 parts) and triethylamine (24.3 parts) in benzene (80 parts), and stirring was additionally continued at room temperature for 18 hours. It was worked up to obtain methyl O-(p-toluenesulfonyl)-(S)-(-)-lactate

A mixture of (1A) (31.7 parts), methyl O-(p-toluenesulfonyl)-(S)-(-)-lactate (25.8 parts) and anhydrous potassium carbonate (28.0 parts) in dry acetonitrile (300 parts) was stirred under reflux for 6 hours. Upon work-up and purification and column chromatography (silica gel, benzene), R-enantiomer of (11a) was obtained, $[\alpha]_D^{25}$ + 14.1° ($C_2H_5OH$, c = 0.347).

The IR and NMR spectral data are shown in Table 1A.

Table 1A

| Compounds No. | IR Spectra ($cm^{-1}$) | NMR Spectra (in $CDCl_3$) δ (ppm) |
|---|---|---|
| (1A) | 3100 1650 1630 1600 1495 | 6.40(1H), 6.57(1H), 7.23(1H), 7.47-7.70(2H), 7.77(1H) 9.78(1H), 11.37(1H) |
| (2A) | 3100 1650 1630 1605 1495 | 6.45-6.67(2H), 7.07-7.20(2H), 7.40-7.70(3H), 9.73(1H), 11.40(1H) |
| (3A) | 3100 1650 1640 1620 1490 | 6.37-6.63(2H), 6.90-7.05(2H), 7.23-7.50(3H), 9.72(1H), 11.33(1H) |
| (4A) | 3100 1650 1640 1620 1490 | 6.35-6.73(2H), 7.06-7.70(4H), 9.73(1H), 11.35(1H) |
| (5A) | 3100 1650 1620 | 6.73-7.00(2H), 7.57(1H), 8.00(1H), 8.28(1H), 9.80(1H), 11.27(1H) |
| (8A) | 1690 1590 1320 | 1.47(3H), 4.07(3H), 6.30-6.55(2H), 7.12(1H), 7.43-7.72(3H), 10.22(1H) |

Table 1A (continued)

| Compounds No. | IR Spectra ($cm^{-1}$) | NMR Spectra (in $CDCl_3$) δ (ppm) |
|---|---|---|
| (9A) | 1750 1670 1590 1320 | 3.78(2H), 4.70(2H), 6.45-6.60(2H), 7.13(1H), 7.55(1H), 7.73-7.90(2H), 10.40(1H) |
| (10A) | 1750 1670 1590 1320 | 1.28(3H), 4.27(2H), 4.70(2H), 6.47-6.63(2H), 7.17(1H), 7.57(1H), 7.77-7.93(2H), 10.43(1H) |
| (11A) | 1750 1670 1590 1325 | 1.68(3H), 3.73(3H), 4.83(1H), 6.45-6.61(2H), 7.17(1H), 7.57(1H), 7.73-7.87(2H), 10.40(1H) |
| (12A) | 1740 1680 1590 1320 | 1.22(3H), 1.68(3H), 4.17(2H), 4.75(1H), 6.37-6.57(2H), 7.10(1H), 7.53(1H), 7.68-7.87(2H), 10.37(1H) |
| (13A) | 1740 1680 1590 1320 | 0.77-1.83(7H), 1.67(3H), 4.13(2H), 4.80(1H), 6.43-6.63(2H), 7.13(1H), 7.57(1H), 7.77-7.93(2H) |

Table 1A (continued)

| Compounds No. | IR Spectra ($cm^{-1}$) | NMR Spectra (in $CDCl_3$) δ (ppm) |
| --- | --- | --- |
| (14A) | 1750 1680 1590 1325 | 1.65(3H), 3.73(3H), 4.73(1H), 6.37-6.63(2H), 7.07(2H), 7.53-7.77(4H) 10.27(1H) |
| (15A) | 1750 1675 1580 1250 | 1.65(3H), 3.70(3H), 4.77(1H), 6.35-6.62(2H), 6.97(2H), 7.33(2H), 7.77(1H), 10.37(1H) |
| (16A) | 1750 1675 1580 1250 | 1.67(3H), 3.73(3H), 4.77(1H), 6.30-6.73(2H), 7.07-7.47(3H), 7.80(1H), 10.40(1H) |
| (17A) | 1750 1675 1590 | 1.67(3H), 3.73(3H), 4.78(1H), 6.74-7.02(2H), 7.59(1H), 7.90-8.28(2H), 10.40(1H) |
| (19A) | 2700-3500 (broad) 1720 1670 1590 | 1.72(3H), 4.85(1H), 6.45-6.63(2H), 7.17(1H), 7.57(1H), 7.73-7.90(2H), 8.18(1H), 10.33(1H) |

Table 1A (continued)

| Compounds No. | IR Spectra ($cm^{-1}$) | | NMR Spectra (in $CDCl_3$) δ (ppm) | |
|---|---|---|---|---|
| (20A) | 1750 | 1680 | 1.13(3H), | 2.06(2H), |
| | 1590 | 1485 | 3.70(3H), | 4.55(1H), |
| | 1460 | 1430 | 6.30(1H), | 6.50(1H), |
| | 1400 | 1320 | 7.10(1H), | 7.50(1H), |
| | 1260 | | 7.70(1H), | 7.75(1H), |
| | | | 10.33(1H) | |
| (21A) | 1750 | 1680 | 0.7-1.1(3H), | 3.67(3H), |
| | 1595 | 1485 | 1.1-1.7(6H), | 4.60(1H), |
| | 1460 | 1435 | 1.7-2.3(2H), | 6.30(1H), |
| | 1400 | 1320 | 6.50(1H), | 7.10(1H), |
| | 1260 | | 7.50(1H), | 7.70(1H), |
| | | | 7.75(1H), | 10.33(1H) |
| (22A) | 1700 | 1590 | 1.63(3H), | 3.77(6H), |
| | 1490 | 1435 | 4.75(1H), | 6.50(1H), |
| | 1400 | 1330 | 6.60(1H), | 7.10(2H), |
| | 1250 | | 7.53(1H), | 7.70(2H), |
| (11A/R-enantiomer) | 1750 | 1670 | 1.68(3H), | 3.73(1H), |
| | 1590 | 1325 | 4.83(1H), | 6.45-6.61(2H), |
| | | | 7.17(1H), | 7.57(1H), |
| | | | 7.73-7.87(2H), | 10.40(1H) |

Example 1B

Synthesis of 2-ethoxy-4-(2-chlcro-4-trifluoromethylphenoxy)benzaldehyde oxime (1B)

A solution of hydroxylamine hydrochloride (0.7 part) in water (5 parts) was added to a solution of 2-ethoxy-4-(2-chloro-4-trifluoromethylphenoxy)benzaldehyde (8A) (3.45 parts) in tetrahydrofuran (20 parts), then potassium carbonate (0.76 part ) was added to the solution with stirring at room temperature, and stirring was additionally continued at room temperature for 1 hour. Tetrahydrofuran was removed under reduced pressure, combined with water (20 parts), and extracted with ether. The ether layer was dried over anhydrous sodium sulfate, then ether was removed. The residue was purified by silica-gel chromatography to give compound (1B) (3.2 parts). The IR and NMR spectral data of the compound are shown in Table 1B.

Example 2B

Synthesis of 2-(1-methoxycarbonylethoxy)-4-(2-chloro-4-trifluoromethylphenoxy)benzaldehyde oxime (2B)

Example 1B was repeated except that 2-(1-methoxycarbonylethoxy)-4-(2-chloro-4-trifluoromethylphenoxy) benzaldehyde (11A) (4.03 parts) was used instead of 2-ethoxy-4-(2-chloro-4-trifluoromethylphenoxy)benzaldehyde (8A) (3.45 parts). There was obtained compound (2B) (3.85 parts). The IR and NMR spectral data of the compound are given in Table 1B.

Example 3B

Synthesis of 2-(1-ethoxycarbonylethoxy)-4-(2-chlorotrifluoromethylphenoxy)benzaldehyde oxime (3B)

Example 1B was repeated except that 2-(1-ethoxy-carbonylethoxy)-4-(2-chloro-4-trifluoromethylphenoxy)benzaldehyde (12A) (4.16 parts) was used instead of 2-ethoxy-4-(2-chloro-4-trifluoromethylphenoxy)benzaldehyde (8A) (3.45 parts) to produce the subject compound (3.90 parts). The IR and NMR spectral data of the compound are given in Table 1B.

Example 4B

Synthesis of 2-ethoxy-4-(2-chloro-4-trifluoromethylphenoxy)benzaldehyde O-methyloxime (4B)

Example 1B was repeated except that O-methylhydroxylamine hydrochloride (0.7 part) was used instead of hydroxylamine hydrochloride (0.7 part) to produce the subject compound (3.80 parts). The IR and NMR spectral data of the compound are given in Table 1B.

Example 5B

Synthesis of 2-(1-methoxycarbonylethoxy)-4-(2-chloro-4-trifluoromethylphenoxy)benzaldehyde O-methyloxime (5B)

Example 2B was repeated except that O-methylhydroxylamine hydrochloride (0.84 part) was used instead of hydroxylamine hydrochloride (0.7 part) to produce the subject compound (3.85 parts) (5B). The IR and NMR spectral data of the compound are given in Table 1B.

Examples 6B through 13B

Syntheses of compounds (6B) through (13B)

A solution of 2-(1-methoxycarbonylethoxy)-4-(2-chloro-4-trifluoromethylphenoxy)benzaldehyde (11A) (4.03 parts) in tetrahydrofuran (20 parts) is combined with an aqueous solution containing an equimolar amount of O-substituted hydroxylamine given in Table 1B and the mixture was stirred at room temperature for additional 1 hour. Then, tetrahydrofuran was removed under reduced pressure, the residue was mixed with water (20 parts), extracted with ether, the ether layer was dried over anhydrous sodium sulfate, and ether was evaporated off. The residue was purified by silica-gel chromatography to give the products in weights given in Table 2B. The IR and NMR spectral data of the compounds are given in Table 1B.

Table 2B

| Examples No. | O-substituted hydroxylamine | Yield (parts) | Compounds No. |
|---|---|---|---|
| 6B | $H_2N-OCH_2CH_3$ | 3.66 | (6B) |
| 7B | $H_2N-OCH_2CH_2CH_3$ | 3.72 | (7B) |
| 8B | 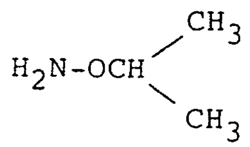 | 4.05 | (8B) |
| 9B | 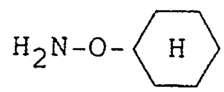 | 4.00 | (9B) |
| 10B | $H_2N-O-CH_2COOC_2H_5$ | 3.78 | (10B) |
| 11B | $H_2N-O-CH_2CH=CH_2$ | 3.89 | (11B) |
| 12B | $H_2N-O-CH_2CH=CH-CH_3$ | 3.75 | (12B) |
| 13B | $H_2N-O-(CH_2)_2CH=CH_2$ | 3.93 | (13B) |

Example 14B

Synthesis of 2-(1-ethoxycarbonylethoxy)-4-(2-chloro-4-trifluoromethylphenoxy)benzaldehyde O-allyloxime (14B)

Example 11B was repeated except that 2-(1-ethoxycarbonylethoxy)-4-(2-chloro-4-trifluoromethylphenoxy)benzaldehyde (12A) (4.16 parts) was used instead of 2-(1-methoxycarbonylethoxy)-4-(2-chloro-4-trifluoromethylphenoxy)benzaldehyde (11A) (4.03 parts) to produce the subject compound (14B) (3.90 parts). The IR and NMR spectral data of the compound are given in Table 1B.

Example 15B

Synthesis of 2-(1-methoxycarbonylethoxy)-4-(2-chloro-4-trifluoromethylphenoxy)benzaldehyde O-propargyloxime (15B)

Example 6B was repeated except that O-propargylhydroxylamine hydrochloride was used as an O-substituted hydroxylamine to produce the subject compound (3.0 parts) (15B). The IR and NMR spectral data of the compound are given in Table 1B.

Example 16B

Synthesis of 2-(1-methoxycarbonylethoxy)-4-(2-chloro-4-trifluoromethylphenoxy)acetophenone oxime (16B)

Example 1B was repeated except that 2-(1-methoxycarbonylethoxy)-4-(2-chloro-4-trifluoromethylphenoxy)acetophenone (21 A) (4.17 parts) was used instead of 2-ethoxy-4-(2-chloro-4-trifluoromethylphenoxy)benzaldehyde (8A) to produce the subject compound (3.0 parts) (16B). The IR and NMR spectral data of the compound are given in Table 1B.

Example 17B

Synthesis of 2-(1-methoxycarbonylethoxy)-4-(2-chloro-4-trifluoromethylphenoxy)acetophenone O-methyloxime (17B)

Example 16B was repeated except that O-methylhydroxylamine hydrochloride (0.84 part) was used instead of hydroxylamine hydrochloride (0.74 part) to produce the subject compound (3.1 parts) (17B). The IR and NMR spectral data of the compound are given in Table 1B.

Example 18B

Synthesis of 2-(1-methoxycarbonylethoxy)-4-(2-chloro-4-trifluoromethylphenoxy)benzaldehyde O-benzyloxime (18B)

O-Benzylhydroxylamine (1.23 part) was added to a solution of 2-(1-methoxycarbonylethoxy)-4-(2-chloro-4-trifluoromethylphenoxy)benzaldehyde (11A) in tetrahydrofuran (20 parts) and stirred at room temperature for 2 hours, then tetrahydrofuran was evaporated under reduced pressure. The residue was purified by silica-gel chromatography to give the subject compound (18B) (4.67 parts). The IR and NMR spectral data of the compound are given in Table 1B.

Example 19B

Synthesis of 2-ethoxy-4-(2-chloro-4-trifluoromethylphenoxy)benzaldehyde O-phenyloxime (19B)

Example 18B was repeated except that 2-ethoxy-4-(2-chloro-4-trifluoromethylphenoxy)benzaldehyde (8A) (3.45 parts) was used instead of 2-(1-methoxycarbonylethoxy)-4-(2-chloro-4-trifluoromethylphenoxy)-benzaldehyde (11A) (4.03 parts), and O-phenylhydroxylamine (1.10 part), instead of O-benzylhydroxylamine (1.23 part) to give the subject compound (19B) (3.70 parts). The IR and NMR spectral data of the compound are shown in Table 1B.

Examples 20B through 37B

Syntheses of compounds (20B) through (37B)

2-(1-Methoxycarbonylethoxy)-4-(2-chloro-4-trifluoromethylphenoxy)benzaldehyde (11A) (4.03 parts) was dissolved in tetrahydrof uran (20 parts). Then, an equimolar amount of O-substituted hydroxylamines given in the following table 3B were added to the solution, and they were stirred at room temperature for 2 to 5 hours, respectively. Tetrahydrofuran was evaporated under reduced pressure, the residue was purified by silica-gel chromatography to give the product. The yields of individual products were given in Table 3B. The IR and NMR spectral data of the products are shown in Table 1B.

Table 3B

| Examples No. | O-substituted hydroxylamines | Yields (parts) | Compounds No. |
|---|---|---|---|
| 20B | O-phenylhydroxylamine | 4.4 | (20B) |
| 21B | O-4-fluorophenylhydroxylamine | 4.3 | (21B) |
| 22B | O-2-fluorophenylhydroxylamine | 4.2 | (22B) |
| 23B | O-3-fluorophenylhydroxylamine | 4.3 | (23B) |
| 24B | O-4-chlorophenylhydroxylamine | 4.7 | (24B) |
| 25B | O-4-cyanophenylhydroxylamine | 4.6 | (25B) |
| 26B | O-4-nitrophenylhydroxylamine | 4.8 | (26B) |
| 27B | O-3-nitrophenylhydroxylamine | 4.7 | (27B) |
| 28B | O-3-trifluoromethylphenylhydroxyl amine | 5.0 | (28B) |
| 29B | O-3-methoxyphenylhydroxylamine | 4.7 | (29B) |
| 30B | O-4-methylphenylhydroxylamine | 4.1 | (30B) |
| 31B | O-4-phenylphenylhydroxylamine | 4.5 | (31B) |
| 32B | O-4-(4-trifluoromethylphenoxy)phenylhydroxylamine | 5.0 | (32B) |
| 33B | 0-3,4-dichlorophenylhydroxyl amine | 5.0 | (33B) |
| 34B | O-2,4-dinitrophenylhydroxylamine | 5.0 | (34B) |
| 35B | O-2-chloro-4-trifluoromethyl phenylhydroxylamine | 5.3 | (35B) |
| 36B | O-3,5-dimethoxyphenylhydroxyl amine | 4.9 | (36B) |
| 37B | O-3,5-dimethylphenylhydroxyl amine | 4.2 | (37B) |

Examples 38B through 45B

Syntheses of compounds (38B) through (45B)

Example 20B was repeated except that 2-(1-methoxycarbonylethoxy)-4-(2,4-dichlorophenoxy)-benzaldehyde (16A) (3.70 parts) was used instead of 2-(1-methoxycarbony lethoxy)-4-(2-chloro-4-trifluoromethylphenoxy)benzaldehyde (11A) (4.03 parts) and O-substituted hydroxylamines given in the following table 3B were used to give the products in yields shown in Table 3B, respectively. The IR and NMR spectral data are given in Table 1B.

Table 4B

| Examples No. | O-substituted hydroxylamines | Yields (parts) | Compoounds No. |
|---|---|---|---|
| 38B | O-phenylhydroxylamine | 4.1 | (38B) |
| 39B | O-4-fluorophenylhydroxylamine | 4.0 | (39B) |
| 40B | O-4-nitrophenylhydroxylamine | 4.5 | (40B) |
| 41B | O-3-methoxyphenylhydroxylamine | 4.4 | (41B) |
| 42B | O-3-trifluoromethylphenyl hydroxylamine | 4.5 | (42B) |
| 43B | O-4-methylphenylhydroxyamine | 4.0 | (43B) |
| 44B | O-4-cyanophenylhydroxylamine | 4.2 | (44B) |
| 45B | O-2-chloro-4-trifluoromethyl phenylhydroxylamine | 5.0 | (45B) |

Examples 46B through 49B

Syntheses of compounds (46B) through (49B)

Example 20B was repeated except that 2-(1-methoxycarbonylethoxy)-4-(2-chloro-4-trifluoromethyl-phenoxy)benzalde hyde (14A) (3.68 parts) was used instead of 2-(1-methoxycarbonylethoxy)-4-(2-chloro-4-trifluoromethylphenoxy)benzaldehyde (11A) (4.03 parts) and O-substituted hydroxylamines given in the following table 5B were used to give the products in yields shown in Table 5B, respectively. The IR and NMR spectral data are given in Table 1B.

Table 5B

| Examples No. | O-substituted hydroxylamines | Yields (parts) | Compounds No. |
|---|---|---|---|
| 46B | O-phenylhydroxylamine | 4.1 | (46B) |
| 47B | O-4-fluorophenylhydroxylamine | 4.0 | (47B) |
| 48B | O-4-chlorophenylhydroxylamine | 4.2 | (48B) |
| 49B | O-3-trifluoromethylphenyl hydroxylamine | 4.5 | (49B) |

Examples 50B through 56B

Syntheses of compounds (50B) through (56B)

Example 20B was repeated except that O-substituted hydroxylamines given in the following table 6B were used to give the products in yields shown in Table 6B, respectively. The IR and NMR spectral data are given in Table 1B.

Table 6B

| Examples No. | O-substituted hydroxylamines | Yields (parts) | Compounds No. |
|---|---|---|---|
| 50B | O-pyrid-2-ylhydroxylamine | 3.5 | (50B) |
| 51B | O-6-methoxypyrid-2-ylhydroxyl amine | 3.8 | (51B) |
| 52B | O-3-chloropyrid-2-ylhdroxyl amine | 3.7 | (52B) |
| 53B | O-pyraz-2-ylhydroxylamine | 3.4 | (53B) |
| 54B | O-3,6-dimethylpyraz-2-ylhydroxyl amine | 3.7 | (54B) |
| 55B | O-quinol-2-ylhydroxylamine | 3.8 | (55B) |
| 56B | O-5-chloro-pyridaz-2-ylhydroxyl amine | 3.7 | (56B) |

Examples 57B, 58B and 61B

Syntheses of compounds (57B), (58B), (61B)

Example 38B was repeated except that O-substituted hydroxylamines given in the following table 7B were used to give the products in yields shown in Table 7B, respectively. The IR and NMR data are given in Table 1B.

Table 7B

| Examples No. | O-substituted hydroxylamines | Yields (parts) | Compounds No. |
|---|---|---|---|
| 57B | O-6-chloropyrid-2-ylhydroxylamine | 3.5 | (57B) |
| 58B | O-2,6-dimethylpyraz-2-ylhdroxyl amine | 3.4 | (58B) |
| 61B | O-3-chloropyrid-2-ylhydroxylamine | 3.2 | (61B) |

Examples 59B and 60B

Syntheses of compounds (59B) and (60B)

Example 20B was repeated except that 2-(1-methoxycarbonylethoxy)-4-(4-trifluoromethylphenoxy)-benzaldehyde (14A) (3.68 parts) was used instead of 2-(1-methoxycarbonylethoxy)-4-(2-chloro-4-trifluoromethylphenoxy)-benzaldehyde (11A) (4.03 parts) and O-substituted hydroxylamines given in the following table 8B were used to give the products in yields shown in Table 8B, respectively. The IR and NMR spectral data are given in Table 1B.

Table 8B

| Examples No. | O-substituted hydroxylamines | Yields (parts) | Compounds No. |
|---|---|---|---|
| 59B | O-6-chloropyrid-2-ylhydroxylamine | 3.4 | (59B) |
| 60B | O-6-methoxypyrid-2-ylhydroxyl amine | 3.2 | (60B) |

Example 62B

Synthesis of 2-(1-methoxycarbonylethoxy)-4-(4-chlorophenoxy)benzaldehyde O-phenyloxime (62B)

Example 20B was repeated except that 2-(1-methoxycarbonylethoxy)-4-(4-chlorophenoxy)benzaldehyde (15A) (3.4 parts) was used instead of 2-(1-methoxycarbonylethoxy)-4-(2-chloro-4-trifluoromethylphenoxy)-benzaldehyde (11A) (4.03 parts) to give the subject product (3.8 parts). The IR and NMR spectral data are given in Table 1B.

Example 63B

Synthesis of 2-(1-carboxyethoxy)-4-(2-chloro-4-trifluoromethylphenoxy)benzaldehyde O-phenyloxime (63B)

2-(1-Carboxyethoxy)-4-(2-chloro-4-trifluoromethylphenoxy)benzaldehyde (19A) (0.41 parts) was dissolved in tetrahydrofuran (30 parts), and combined with O-phenylhydroxylamine hydrochloride (0.23 part), sodium carbonate (0.14 part), and water (2 parts by volume). The mixture was stirred at room temperature for about 3 hours. After completion of the reaction, terahydrofuran was removed under reduced pressure, diluted with water, acidified with hydrochloric acid, then extracted with ether. The organic layer was washed with water, dried over anhydrous sodium sulfate, and concentrated under reduced pressure. The crude product was purified by silica gel column chromatography to give the compound (63B) (0.3 part). The spectroscopic data is given in Table 1B.

Example 64B

Synthesis of 2-hydroxy-4-(2-chloro-4-trifluoromethylphenoxy)benzaldehyde O-phenyloxime (64B)

Example 20B was repeated except that 2-hydroxy-4-(2-chloro-4-trifluoromethylphenoxy)benzaldehyde (1A) (3.17 parts) was used instead of 2-(1-methoxycarbonylethoxy)-4-(2- chloro-4-trifluoromethylphenoxy)-benzaldehyde (11A) (4.03 parts) to give the subject compound (64B) (2.90 parts). The IR and NMR spectral data are given in Table 1B.

Example 65B

Synthesis of 2-(1-methoxycarbonylethoxy)-4-(3-chloro-5-trifluoromethyl-2-pyridyloxy)benzaldehyde O-phenyloxime (65B)

Example 20B was repeated except that 2-(1-methoxycarbonylethoxy)-4-(3-chloro-5-trifluoromethyl-pyridyloxy)benzaldehyde (17A) (4.03 parts) was used instead of 2-(1-methoxycarbonylethoxy)-4-(2-chloro-4-trifluoromethylphenoxy)benzaldehyde (11A) (4.03 parts) to give the subject product (3.90 parts). The IR and NMR data are given in Table 1B.

Example 66B

Syntheses of compounds (26B), (34B), (50B) through (61B), (84B) and (143B)

Compounds (2B) obtained in Example 2B (4.18 parts) and 4-fluoronitrobenzene (1.55 part) were dissolved in N-methylpyrrolidone (20 parts) under stirring, then combined with 60% strength sodium hydride (0.42 part) at room temperature. Then, they were stirred at room temperature for additional 5 hours, the reaction mixture was poured into ice water, and extracted with diethyl ether. The organic layer was dried over anhydrous sodium sulfate, ether was removed under reduced pressure, and the residue was purified by silica gel chromatography to give the compound (26B) (4.2 parts). The same procedures were repeated except that aldehyde oximes prescribed in the following Table 9B were used instead of the compound (2B) and halogen compounds prescribed in the following Table 9B were employed instead of 4-fluoronitrobenzene to give the subject compounds. The IR and NMR spectral data are given in Table 1B.

Table 9B

| Compounds No. | Aldehyde oxime No. | Halogen compounds |
|---|---|---|
| (26B) | (2B) | 4-fluoronitrobenzene |
| (34B) | (2B) | 2,4-dinitrochlorobenzene |
| (50B) | (2B) | 2-chloropyridine |
| (51B) | (2B) | 2-chloro-6-methoxypyridine |
| (52B) | (2B) | 2,3-dichloropyridine |
| (53B) | (2B) | 2-chloropyrazine |
| (54B) | (2B) | 2-chloro-3,6-dimethyl pyrazine |
| (55B) | (2B) | 2-chloroquinoline |
| (56B) | (2B) | 3,6-dichloropyridazine |
| (57B) | (78B) | 2,6-dichloropyridine |
| (58B) | (78B) | 2-chloro-3,6-dimethylpyrazine |
| (59B) | (2B) | 2,6-dichloropyridine |
| (60B) | (2B) | 2-chloro-6-methoxypyridine |
| (61B) | (78B) | 2,3-dichloropyridine |
| (84B) | (2B) | 4-chloro-2-methylthiopyrimidine |
| (143B) | (2B) | 2,6-dichloroquinoxaline |

Example 67B

Syntheses of (66B) through (70B), (74B), (79B), (80B), (82B), (83B), (85B) through (87B), (89B), (91B), (95B) through (100B), and (146B)

The reactions between the aldehydes and O-substituted hydroxylamine which are prescribed in the following Table 10B were carried out as in Example 6B to give the subject compounds in Table 10B. The IR and NMR spectral data are shown in Table 1B.

Table 10B

| Compounds | Aldehyde derivatives | O-substituted hydroxylamine |
|---|---|---|
| No. | No. | |
| (66B) | (9A) | O-allylhydroxylamine |
| (67B) | (10A) | O-allylhydroxylamine |
| (68B) | (9A) | O-phenylhydroxylamine |
| (69B) | (10A) | O-phenylhydroxylamine |
| (70B) | (13A) | O-allylhydroxylamine |
| (74B) | (13A) | O-phenylhydroxylamine |
| (79B) | (24A) | O-4-nitrophenyl hydroxylamine |
| (80B) | (22A) | O-4-nitrophenyl hydroxylamine |
| (82B) | (11A) | O-4-chloro-2-nitrophenyl hydroxylamine |
| (83B) | (11A) | O-2-nitrophenylhydrxylamine |
| (85B) | (11A) | O-4-nitrobenzylhydroxylamine |
| (86B) | (17A) | O-4-nitrophenylhydroxylamine |
| (87B) | (17A) | O-4-fluorophenylhydroxylamine |
| (89B) | (11A) | O-1-nitro-2-naphthyl hydroxylamine |
| (91B) | (11A) | O-4-(1,1-dimethylethyl)phenyl hydroxylamine |
| (95B) | (11A) | O-(4-methoxycarbonylmethyl)-2-nitrophenylhydroxylamine |
| (96B) | (11A) | O-4-methoxycarbonylphenyl hydroxylamine |
| (97B) | (11A) | O-4-trifluoromethylphenyl hydroxylamine |
| (98B) | (9A) | O-4-nitrophenylhydroxylamine |
| (99B) | (20A) | O-4-nitrophenylhydroxylamine |
| (100B) | (21A) | O-4-nitrophenylhydroxylamine |

| | | |
|---|---|---|
| (146B) | (1A) | O-4-nitrophenylhydroxylamine |
| (150B) | (11A) | O-1-cyano-2-propenyl hydroxylamine |

Example 68B

Syntheses of compounds (73B), (75B), (77B), (78B), (88B) and (145B)

Example 2B was repeated except that aldehydes prescribed in the following Table 11B were used instead of the compound (11A) to give the compounds given in Table 11B. The IR and NMR spectral data are shown in Table 1B.

Table 11B

| Compounds No. | Aldehyde derivatives |
|---|---|
| (73B) | (21A) |
| (75B) | ( 9A) |
| (77B) | (20A) |
| (78B) | (16A) |
| (88B) | (17A) |
| (145B) | (24A) |

Example 69B

Synthesis of compound (90B)

Compound (146B) (0.24 part), -bromopropionamide (0.09 part), and anhydrous potassium carbonate (0.15 part) were added to methyl ethyl ketone (20 parts by volume) and they were refluxed for 5 hours. The reaction mixture was filtered, the filtrate was concentrated, and the residue was purified by silica-gel chromatography to give the subject compound (90B) (0.05 part). The IR and NMR spectral data are shown in Table 1B.

Example 70B

Syntheses of compounds (92B) through (94B)

Compound (154B) (0.2 part) was dissolved in ether (4 parts by volume), and a solution of diazomethane in ether was added in portions to the compound solution. After completion of the reaction, ether was removed under reduced pressure, and the residue was purified by silica-gel chromatography to give the subject compound (93B) (0.16 part). The IR and NMR spectral data of the compound are given in Table 1B.

Compounds (155B) and (156B) were used instead of compound (154B) to repeat the above reaction whereby the subject compounds (94B) and (92B) were obtained. The IR and NMR spectral data of these compounds are shown in Table 1B.

Example 71B

Syntheses of compounds (101B) through (109B), (111B) through (117B), (124B) through (133B) and (135B) through (142B)

Compound (2B) obtained according to Example 2B (4.18 parts) and triethylamine (1.11 part) were dissolved in tetrahydrofuran (40 parts), and stirred in an ice bath. A solution of 4-nitrobenzoyl chloride (1.94 part) in tetrahydrofuran (5 parts) was added dropwise, stirred at room temperature for 1 hour, then

tetrahydrofuran was removed under reduced pressure. Then, the residue was combined with water (20 parts), extracted with diethyl ether. The organic layer was dried over anhydrous sodium sulfate, ether was removed, and the residue was purified by silica-gel column chromatography to give compound (101B) (4.3 parts). The same procedures were repeated except that aldehyde oximes listed in the following Table 12B were used instead of compound (2B) and acid chlorides in Table 12B were used instead of 4-nitrobenzoyl chloride to give the objective compounds. The IR and NMR spectral data are showing in Table 1B.

Table 12B

| Compounds No. | Aldehyde oxime No. | Acid chlorides |
|---|---|---|
| (101B) | (2B) | 4-nitrobenzoyl chloride |
| (102B) | (2B) | 3,5-dinitrobenzoyl chloride |
| (103B) | (2B) | 2,4-dinitrobenzoyl chloride |
| (104B) | (2B) | 4-fluorobenzoyl chloride |
| (105B) | (2B) | 4-methoxycarbonyl-2,3,5,6-tetrachlorobenzoyl chloride |
| (106B) | (2B) | 2-nitro-5-(2-chloro-4-trifluoromethylphenoxy)-benzoyl chloride |
| (107B) | (2B) | isonicotinoyl chloride |
| (108B) | (145B) | 4-nitromethylbenzoyl chloride |
| (109B) | (2B) | 4-N,N-dimethylaminobenzoyl chloride |
| (111B) | (2B) | 4-trifluoromethylbenzoyl chloride |
| (112B) | (2B) | 4-cyanobenzoyl chloride |
| (113B) | (88B) | 4-nitrobenzoyl chloride |
| (114B) | (73B) | 4-nitrobenzoyl chloride |
| (115B) | (77B) | 4-nitrobenzoyl chloride |
| (116B) | (75B) | 4-nitrobenzoyl chloride |
| (117B) | (2B) | phenyl chloroformate |
| (124B) | (2B) | 2,4-dichlorobenzoyl chloride |
| (125B) | (2B) | 2,4-dichlorophenoxyacetyl chloride |
| (126B) | (2B) | 1-[4-(3-chloro-5-trifluoromethyl-2-pyridyloxy)phenoxy-propionyl chloride |

| | | |
|---|---|---|
| (127B) | (2B) | acetyl chloride |
| (128B) | (2B) | 1-[4-(5-trifluoromethyl-2-pyridyloxy)phenoxypropionyl chloride |
| (129B) | (2B) | ethyl chloroformate |
| (130B) | (2B) | n-capryl chloride |
| (131B) | (2B) | 1,1-dichloropropionyl chloride |
| (132B) | (88B) | acetyl chloride |
| (133B) | (2B) | 2,2,2-trichloroethyl chloroformate |
| (135B) | (2B) | 3-aminobenzoyl chloride |
| (136B) | (2B) | 3-(N,N-dimethylcarbamoyl-amino)benzoyl chloride |
| (137B) | (2B) | 4-ethoxycarbonylaminobenzoyl chloride |
| (138B) | (2B) | benzoyl chloride |
| (139B) | (2B) | 2,4,6-trimethylbenzoyl chloride |
| (140B) | (2B) | 4-(1,1-dimethylethyl)benzoyl chloride |
| (141B) | (2B) | 4-chloroacetylaminobenzoyl chloride |
| (142B) | (2B) | 4-(1,1-dimethylpropionyl-amino)benzoyl chloride |

Example 72B

Synthesis of compound (110B)

Compound (109B) was dissolved in diethyl ether (50 parts), combined with methyl iodide (4.3 parts), and stirred at room temperature for 1 week. The crystals formed were filtered and rinsed with diethyl ether (50 parts) to give compound (110B) (3.2 parts). The IR and NMR spectral data are shown in Table 1B.

Example 73B

Syntheses of compounds (118B) through (123B), and (134B)

The reaction was carried out at room temperature by adding 3,4-dichlorophenyl isocyanate (0.19 part) and triethylamine (0.01 part) to a solution of compound (2B) (0.42 part) prepared in Example 2B in ether (5 parts by volume). After completion of the reaction, ether was removed under reduced pressure, and the crude product was purified by silica-gel column chromatography to give the compound (118B). The IR and NMR spectral data are given in Table 1B. The reaction was repeated except that corresponding isocyanates were employed, instead of 3,4-dichlorophenyl isocyanate, respectively, to give compounds (119B) through

(123B), and (134B). The IR and NMR spectral data of these compounds were shown in Table 1B, respectively.

Example 74B

Syntheses of compounds (147B), (149B) and (151B) through (153B)

4-Fluorobenzoylaminoacetonitrile (0.59 part) was dissolved in ethyl acetate (35 parts by volume), and bromine (0.58 part) was added dropwise to the solution, as they are cooled to 0° C (after several drops of bromine were added, a solution of hydrogen bromide in acetic acid was added several drops, then remaining bromine was added). After completion of addition, the reaction mixture was cooled down to -25° C, then a solution of the compound (2B) (1.5 part) prepared in Example 2B in ethyl acetate (7 parts by volume) and triethylamine (0.73 part) were added dropwise, simultaneously, thereto. After addition, stirring was continued at 0° C for 15 minutes, then the reaction mixture was filtered with suction, the filtrate was concentrated, and purified by silica gel column chromatography to give the objective compound (149B). The IR and NMR spectral data are shown in Table 1B. Corresponding acetonitrile derivatives were used instead of 4-fluorobenzoylaminoacetonitrile to carry out similar reactions whereby compounds (147B), (151B) through (153B) were obtained. The IR and NMR spectral data of these compounds are given in Table 1B.

Example 75B

Synthesis of compound (148B)

Compound (2B) (4.18 parts), 1-bromo-1-(4-nitrophenyl)acetonitrile (2.41 parts), anhydrous potassium carbonate (1.39 part) and acetonitrile (40 parts) were mixed, stirred at 100° C for 5 hours, then, acetonitrile was removed under reduced pressure, combined with water, and extracted with ethyl ether. The organic layer was dried over anhydrous sodium sulfate, ether was removed, and the residue was purified by silica-gel column chromatography to give the compound (148B) (3.2 parts). The IR and NMR spectral data are given in Table 1B.

Example 76B

Syntheses of compounds (154B) through (156B)

Methyl 2-[2-carboxy-5-(2-chloro-4-trifluoromethylphenoxy)]phenoxypropionate (1.9 part) was combined with thionyl chloride (1.07 part) and heated at 60 to 70° C for 2 hours. After completion of the reaction, the excessive thionyl chloride was removed under reduced pressure, and the resulting solution of the crude product in benzene (5 parts by volume) was combined with a solution of O-4-nitrophenylhydroxylamine (0.83 part) in benzene (10 parts by volume) at room temperature. After completion of the reaction, water was added to the reaction mixture to separate the organic layer, which was dried over anhydrous sodium sulfate. The solvent was removed under reduced pressure, and the crude product was purified by silica-gel column chromatography to give the compound (154B) (2.5 parts). The IR and NMR spectral data of the compound are shown in Table 1B. O-ethoxycarbonylmethylhydroxylamine and O-2-propenyloxycarbonylmethylhydroxylamine were used instead of O-4-nitrophenylhydroxylamine, to carry out similar reactions, respectively whereby compounds (155B) and (156B) were obtained. The IR and NMR spectral data are shown in Table 1B.

Example 77B

Preparation of the R-enantiomer of compound (26B)

Compound (23A) (12.1 parts) was dissolved in tetrahydrofuran (60 parts). Then, an equimolar amount of O-4-nitrophenylhydroxylamine and a drop of concentrated hydrochloric acid were added to the solution, and they were stirred at room temperature for 20 minutes.

Tetrahydrofuran was evaporated under reduced pressure, the residue was re-crystallized from ethanol to give the product, m.p. 92-93.5° C $[\alpha]_D^{25}$ - 1.0° ($C_2H_5OH$, c = 1.0).

The IR and NMR spectral data are shown in Table 1B.

Example 78B

Preparation of the R-enantiomer of compound (2B)

Compound (23A) (12.1 parts) was dissolved in benzene (100 parts). A solution of hydroxylamine hydrochloride (2.5 parts in water (50 parts) was added to the solution, and stirred vigorously. Then, sodium carbonate (1.91 part) was added, and stirring was continued at 70° C for 7 hours.

Organic layer was separated, concentrated under reduced pressure, and purified by silica gel column chromatography to give compound (162B), $[\alpha]_D^{25}$ + 3.5° (THF, c=0.74).

The IR and NMR spectral data are shown in Table 1B.

Table 1B

| Compounds No. | IR Spectra ($cm^{-1}$) | NMR Spectra (in $CDCl_3$) δ (ppm) |
|---|---|---|
| (1B) | 3250 1595 1075 | 1.43(3H), 4.03(2H), 6.40-6.67(2H), 7.07(1H), 7.50(1H), 7.67-7.83(2H), 8.33(1H), 10.80(1H) |
| (2B) | 3300 1755 1595 1320 1120 1080 | 1.62(3H), 3.70(3H), 4.72(1H), 6.40-6.63(2H), 7.00(1H), 7.47(1H), 7.63-7.80(2H), 8.50(1H), 8.93(1H) |
| (3B) | 3350 1740 1590 1320 1120 1080 | 1.20(3H), 1.62(3H), 4.13(2H), 4.67(1H), 6.37-6.60(2H), 6.97(1H), 7.43(1H), 7.63-7.77(2H), 8.47(1H) |
| (4B) | 1595 1320 1120 1050 | 1.42(3H), 3.87(3H), 3.97(2H), 6.35-6.50(2H), 6.97(1H), 7.43(1H), 7.67-7.80(2H), 8.23(1H) |
| (5B) | 1755 1595 1320 1125 1050 | 1.62(3H), 3.70(3H), 3.97(3H), 4.68(1H), 7.47(1H), 7.67-7.87(2H), 8.43(1H) |

Table 1B (continued)

| Compounds No. | IR Spectra ($cm^{-1}$) | NMR Spectra (in $CDCl_3$) δ (ppm) |
|---|---|---|
| (6B) | 1755 1595 1320 1125 1050 | 1.30(3H), 1.62(3H), 3.70(3H), 4.20(2H), 4.70(1H), 6.40-6.63(2H), 7.00(1H), 7.47(1H), 7.70-7.90(2H), 8.47(1H) |
| (7B) | 1755 1595 1320 1125 1050 | 0.98(3H), 1.62(3H), 1.47-2.05(2H), 3.72(3H), 4.12(2H), 4.72(1H), 6.40-6.67(2H), 7.00(1H), 7.48(1H), 7.70-7.90(2H), 8.48(1H) |
| (8B) | 1750 1595 1320 1120 - | 1.27(3H), 1.62(3H), 3.67(3H), 4.32(1H), 4.63(1H), 6.33(2H), 6.53(1H), 6.97(1H), 7.43(1H), 7.67-7.87(2H), 8.27(1H) |
| (9B) | 1750 1595 1320 1125 | 1.60(3H), 1.23-2.23(10H), 3.67(3H), 3.87-4.37(1H), 4.68(1H), 6.37-6.63(2H), 6.97(1H), 7.47(1H), 7.67-7.93(2H), 8.45(1H) |
| (10B) | 1750 1740 1595 1320 1120 | 1.27(3H), 1.62(3H), 3.67(3H), 4.23(2H), 4.63(1H), 4.70(1H), 6.40(1H), 6.67(1H), 7.00(1H), 7.47(1H), 7.67-7.80(2H), 8.52(1H), |

Table 1B (continued)

| Compounds No. | IR Spectra ($cm^{-1}$) | NMR Spectra (in $CDCl_3$) δ (ppm) |
|---|---|---|
| (11B) | 1755 1595 1320 1125 | 1.60(3H), 3.60(3H), 4.47-4.80(3H), 6.33(1H), 5.03-5.37(2H), 6.50(1H), 5.67-6.27(1H), 6.97(1H), 7.67-7.83(2H), 7.43(1H), 8.33(1H) |
| (12B) | 1755 1595 1320 1125 | 1.53-1.80(6H), 3.70(3H), 4.50-4.87(2H), 6.40(1H), 6.70(1H), 6.97(1H), 7.47(1H), 7.67-7.87(2H), 8.47(1H) |
| (13B) | 1750 1595 1320 1125 | 1.60(3H), 2.30-2.63(2H), 3.67(3H), 4.17(2H), 4.67(1H), 4.93-5.23(2H), 5.43-6.23(1H), 6.93(1H), 6.33-6.60(2H), 7.43(2H), 7.67-7.83(2H), 8.40(1H) |
| (14B) | 1750 1595 1320 1125 | 1.23(3H), 1.60(3H), 4.13(2H), 4.43-4.70(2H), 5.70-6.27(1H), 7.40(1H), 6.33-6.70(2H), 7.63-7.83(2H) |
| (15B) | 3300 1750 1595 1320 1125 | 1.60(3H), 2.33(1H), 3.63(3H), 4.47-4.80(3H), 6.33(1H), 6.50(1H), 6.97(1H), 7.43(1H), 7.67-7.87(2H), 8.33(1H) |

Table 1B (continued)

| Compounds No. | IR Spectra ($cm^{-1}$) | NMR Spectra (in $CDCl_3$) δ (ppm) |
|---|---|---|
| (16B) | 3250 1750 1595 1320 1130 | 1.60(3H), 2.27(3H), 3.67(3H), 4.70(1H), 6.37-6.57(2H), 6.97(1H), 7.20-7.67(4H) |
| (17B) | 1755 1595 1320 1130 | 1.60(3H), 2.27(3H), 3.70(3H), 3.97(3H), 4.73(1H), 6.40-6.63(2H), 6.97(1H), 7.23-7.73(3H) |
| (18B) | 1750 1595 1320 1125 | 1.60(3H), 3.70(3H), 4.73(1H), 5.23(2H), 6.40-6.70(2H), 7.03(1H), 7.27-7.60(6H), 8.63(1H), 7.77-7.97(2H) |
| (19B) | 1590 1320 1125 1080 | 1.40(3H), 3.95(2H), 6.27-6.63(2H), 6.77-7.47(7H), 7.63-7.98(2H), 8.68(1H) |
| (20B) | 1750 1590 1320 1125 1080 | 1.67(3H), 3.73(3H), 4.78(1H), 6.47-6.73(2H), 7.03-7.63(7H), 7.82(1H), 8.03(1H), 8.90(1H) |

Table 1B (continued)

| Compounds No. | IR Spectra ($cm^{-1}$) | NMR Spectra (in $CDCl_3$) δ (ppm) |
|---|---|---|
| (21B) | 1750 1590 1320 1125 1080 | 1.63(3H), 3.67(3H), 4.70(1H), 6.33-6.60(2H), 6.73-7.23(5H), 7.27(1H), 7.67(1H), 7.87(1H), 8.70(1H) |
| (22B) | 1750 1590 1320 1125 1080 | 1.65(3H), 3.67(3H), 4.70(1H), 6.37-6.63(2H), 6.83-7.60(6H), 7.70(1H), 7.87(1H)., 8.83(3H) |
| (23B) | 1755 1595 1320 1120 1080 | 1.63(3H), 3.67(3H), 4.70(1H), 6.37-7.23(7H), 7.43(1H), 7.67(1H), 7.87(1H), 8.72(1H) |
| (24B) | 1750 1590 1320 1125 1080 | 1.63(3H), 3.67(3H), 4.70(1H), 6.33-6.60(2H), 6.90-7.23(5H), 7.43(1H), 7.67(1H), 7.87(1H), 8.73(1H) |
| (25B) | 2210 1750 1595 1320 1125 1080 | 1.67(3H), 3.70(3H), 4.73(1H), 6.37-6.63(2H), 6.93-7.67(7H), 7.85(1H), 8.73(1H) |

Table 1B (continued)

| Compounds No. | IR Spectra ($cm^{-1}$) | NMR Spectra (in $CDCl_3$) δ (ppm) |
|---|---|---|
| (26B) | 1750 1590<br>1510 1320<br>1125 1080 | 1.65(3H), 3.68(3H),<br>4.73(1H), 6.37-6.63(2H),<br>6.97-7.70(5H),<br>7.78-8.18(3H), 8.78(1H) |
| (27B) | 1750 1595<br>1525 1320<br>1130 1080 | 1.67(3H), 3.70(3H),<br>4.73(1H), 6.37-6.63(2H),<br>7.00(1H), 7.33-8.03(7H),<br>8.75(1H) |
| (28B) | 1755 1595<br>1320 1125<br>1080 | 1.65(3H), 3.67(3H),<br>4.72(1H), 6.37-6.63(2H),<br>7.00(1H), 7.17-7.52(5H),<br>7.67(1H), 8.77(1H) |
| (29B) | 1750 1595<br>1320 1130<br>1080 | 1.65(3H), 3.68(3H),<br>3.77(3H), 4.72(1H),<br>6.30-7.53(8H), 7.02(1H),<br>7.90(1H), 8.73(1H) |
| (30B) | 1750 1595<br>1320 1130<br>1080 | 1.63(3H), 2.27(3H),<br>3.67(3H), 4.70(1H),<br>6.35-6.63(2H), 7.68(1H),<br>6.90-7.53(6H), 7.88(1H),<br>8.70(1H) |

Table 1B (continued)

| Compounds No. | IR Spectra ($cm^{-1}$) | NMR Spectra (in $CDCl_3$) δ (ppm) |
|---|---|---|
| (31B) | 1750 1590<br>1320 1120<br>1080 | 1.63(3H), 3.67(3H),<br>4.70(1H), 6.33-8.13(15H),<br>8.77(1H) |
| (32B) | 1750 1595<br>1320 1120<br>1080 | 1.67(3H), 3.70(3H),<br>4.72(1H), 6.37-6.63(2H),<br>6.83-7.60(10H), 7.70(1H),<br>7.87(1H), 8.73(1H) |
| (33B) | 1755 1590<br>1320 1120<br>1080 | 1.63(3H), 3.68(3H),<br>4.72(1H), 6.35-6.63(2H),<br>6.85-7.50(5H), 7.70(1H),<br>7.85(1H), 8.72(1H) |
| (34B) | 1750 1600<br>1525 1320<br>1130 1080 | 1.70(3H), 3.70(3H),<br>4.77(1H), 6.37-6.63(2H),<br>7.08(1H), 7.50(1H),<br>7.70-7.97(3H), 8.37(1H),<br>8.73(1H), 8.92(1H) |
| (35B) | 1755 1595<br>1320 1120<br>1080 | 1.68(3H), 3.70(3H),<br>4.77(1H), 6.40-6.67(2H),<br>7.07(1H), 7.40-7.63(4H),<br>7.73(1H), 7.93(1H),<br>8.92(1H) |

Table 1B (continued)

| Compounds No. | IR Spectra ($cm^{-1}$) | | NMR spectra (in $CDCl_3$) δ (ppm) | |
|---|---|---|---|---|
| (36B) | 1750 | 1590 | 1.67(3H), | 3.70(3H), |
| | 1320 | 1130 | 3.77(6H), | 4.73(1H), |
| | 1080 | | 6.00-6.67(5H), | 7.03(1H), |
| | | | 7.50(1H), | 7.73(1H), |
| | | | 7.93(1H), | 8.77(1H) |
| (37B) | 1755 | 1590 | 1.63(3H), | 2.33(6H), |
| | 1320 | 1130 | 3.72(3H), | 4.72(1H), |
| | 1080 | | 6.40-7.20(6H), | 7.50(1H), |
| | | | 7.73(1H), | 7.93(1H), |
| | | | 8.77(1H) | |
| (38B) | 1755 | 1605 | 1.63(3H), | 3.70(3H), |
| | 1270 | 1090 | 4.70(1H), | 6.33-6.63(2H), |
| | | | 7.00-7.53(8H), | 7.93(1H), |
| | | | 8.78(1H) | |
| (39B) | 1750 | 1605 | 1.63(3H), | 1.73(3H), |
| | 1270 | 1225 | 4.73(1H), | 6.33-6.63(2H), |
| | 1235 | 1090 | 6.80-7.57(7H), | |
| | | | 7.90(1H), | 8.77(1H) |
| (40B) | 1750 | 1605 | 1.67(3H), | 1.73(3H), |
| | 1510 | 1270 | 4.77(1H), | 6.37-6.67(2H), |
| | 1235 | 1090 | 7.03-7.47(6H), | 7.93(1H), |
| | | | 8.10-8.27(2H), | 8.83(1H), |

Table 1B (continued)

| Compounds No. | IR Spectra ($cm^{-1}$) | NMR spectra (in $CDCl_3$) δ (ppm) |
|---|---|---|
| (41B) | 1750 1600 1270 1225 1090 | 1.65(3H), 3.70(1H), 3.73(3H), 4.70(1H), 6.33-6.63(3H), 7.93(1H), 6.73-7.57(6H), 8.78(1H) |
| (42B) | 1755 1605 1325 1125 1090 | 1.67(3H), 3.73(3H), 4.74(1H), 6.37-6.67(2H), 7.03-7.53(7H), 7.93(1H), 8.83(1H) |
| (43B) | 1750 1605 1270 1220 1090 | 1.67(3H), 2.30(3H), 3.72(3H), 4.73(1H), 6.33-6.67(2H), 6.87-7.60(7H), 7.93(1H), 8.80(1H) |
| (44B) | 2210 1750 1600 1270 1230 1090 | 1.67(3H), 3.73(3H), 4.75(1H), 6.37-6.67(2H), 7.03-7.67(7H), 7.90(1H) 8.83(1H) |
| (45B) | 1750 1605 1320 1120 1080 | 1.67(3H), 3.70(3H), 4.73(1H), 6.33-6.62(2H), 7.00-7.70(6H), 7.87(1H), 8.88(1H) |

Table 1B (continued)

| Compounds No. | IR Spectra ($cm^{-1}$) | NMR spectra (in $CDCl_3$) δ (ppm) |
|---|---|---|
| (46B) | 1755 1595 1320 1120 1080 | 1.63(3H), 3.67(3H), 4.70(1H), 6.40(1H), 6.60(1H), 6.97-7.30(7H), 7.57(2H), 7.93(2H), 8.77(1H) |
| (47B) | 1740 1595 1320 1120 | 1.67(3H), 3.72(3H), 4.75(1H), 6.45(1H), 6.67(1H), 7.00-7.33(6H), 7.63(2H), 7.95(1H), 8.80(1H) |
| (48B) | 1740 1595 1325 1120 | 1.67(3H), 3.70(3H), 4.75(1H), 6.45(1H), 6.67(1H), 7.00-7.33(6H), 7.63(2H), 7.95(1H), 8.82(1H) |
| (49B) | 1755 1595 1325 1120 | 1.67(3H), 3.70(3H), 4.73(1H), 6.45(1H), 6.65(1H), 7.00-7.70(8H), 7.97(1H), 8.83(1H) |
| (50B) | 1730 1580 1320 1160 1120 | 1.65(3H), 3.63(3H), 4.63(1H), 6.23(1H), 6.50(1H), 6.70-7.10(2H), 7.15(1H), 7.30-7.60(2H), 7.70(1H), 7.90(1H), 8.10(1H), 8.70(1H) |

Table 1B (continued)

| Compounds No. | IR Spectra (cm⁻¹) | | NMR spectra (in $CDCl_3$) δ (ppm) | |
|---|---|---|---|---|
| (51B) | 1750 | 1600 | 1.65(3H), | 3.65(3H), |
| | 1570 | 1320 | 3.85(3H), | 4.70(1H), |
| | 1160 | | 6.20-6.30(2H), | 6.55(1H), |
| | | | 6.80(1H), | 7.05(1H), |
| | | | 7.30-7.60(2H), | 7.65(1H), |
| | | | 7.90(1H), | 8.10(1H) |
| (52B) | 1750 | 1640 | 1.65(3H), | 3.70(3H), |
| | 1590 | 1320 | 4.70(1H), | 6.30-6.60(2H), |
| | 1170 | 1120 | 6.90-7.10(2H), | 8.00(1H), |
| | | | 7.40-7.80(3H), | 8.10(1H), |
| | | | 8.90(1H) | |
| (53B) | 1750 | 1590 | 1.65(3H), | 3.70(3H), |
| | 1320 | 1170 | 4.73(1H), | 6.30-6.70(2H), |
| | 1125 | | 7.10(1H), | 7.50(1H), |
| | | | 7.70(1H), | 7.90(1H), |
| | | | 8.10-8.30(2H), | 8.60(1H), |
| | | | 8.90(1H) | |
| (54B) | 1740 | 1595 | 1.70(3H), | 2.55(3H), |
| | 1320 | 1160 | 2.55(3H), | 3.70(3H), |
| | 1120 | | 4.75(1H), | 6.40-6.70(2H), |
| | | | 7.00(1H), | 7.50(1H), |
| | | | 7.70(1H), | 8.00(1H), |
| | | | 8.90(1H) | |
| (55B) | 1740 | 1590 | 1.63(3H), | 3.65(3H), |
| | 1320 | 1170 | 4.65(1H), | 6.30(1H), |
| | 1125 | | 6.50(1H), | 6.90(1H), |
| | | | 7.00-7.90(7H), | 7.95(2H), |
| | | | 8.75(1H) | |

Table 1B (continued)

| Compounds No. | IR Spectra ($cm^{-1}$) | NMR spectra (in $CDCl_3$) δ (ppm) |
|---|---|---|
| (56B) | 1740 1590 1325 1160 1110 | 1.65(3H), 3.70(3H), 4.73(1H), 6.30-6.70(2H), 7.05(1H), 7.30-7.70(4H), 7.90(1H), 8.93(1H) |
| (57B) | 1750 1600 1570 1160 1125 | 1.65(3H), 3.70(3H), 4.65(1H), 6.30(1H), 6.56(1H), 6.90-7.80(6H), 7.90(1H), 8.76(1H) |
| (58B) | 1740 1600 1570 1160 1120 | 1.66(3H), 2.50(3H), 2.53(3H), 3.73(3H), 4.75(1H), 6.30(1H), 6.56(1H), 7.00(1H), 7.20-7.50(1H), 8.00(2H), 8.90(1H) |
| (59B) | 1745 1590 1320 1160 1120 | 1.67(3H), 3.70(3H), 4.73(1H), 6.30(1H), 6.66(1H), 6.90-7.30(4H), 7.50-7.70(3H), 7.90(1H), 8.80(1H) |
| (60B) | 1730 1580 1320 1160 1120 | 1.65(3H), 3.70(3H), 3.90(3H), 4.70(1H), 6.20-6.50(2H), 6.66(1H), 6.70-7.20(3H), 7.90(1H), 7.30-7.70(3H), 8.80(1H) |

Table 1B (continued)

| Compounds No. | IR Spectra ($cm^{-1}$) | NMR spectra (in $CDCl_3$) δ (ppm) |
|---|---|---|
| (61B) | 1740 1600 1580 1160 1120 | 1.66(3H), 3.70(3H), 4.66(1H), 6.30(1H), 6.60(1H), 7.00(1H), 6.90-7.20(2H), 7.40(1H), 7.65(1H), 8.00-8.20(2H), 8.80(1H) |
| (62B) | 1750 1605 1270 1215 1090 | 1.60(3H), 3.67(3H), 4.67(1H), 6.35(1H), 6.55(1H), 6.80-7.67(9H), 7.88(1H), 8.78(1H) |
| (63B) | 3300 - 2400 1720 1590 1320 1080 | 1.67(3H), 4.78(1H), 6.45-6.73(2H), 7.70(1H), 6.83-7.53(8H), 7.87(1H), 8.75(1H) |
| (64B) | 3200 1590 1320 1125 1080 | 6.43-6.60(2H), 7.72(1H), 6.87-7.60(8H), 8.43(1H), 9.90(1H) |
| (65B) | 1750 1590 1320 1130 1125 1090 | 1.67(3H), 3.73(3H), 4.78(1H), 6.70-7.67(6H), 7.93-8.30(4H), 8.83(1H) |

Table 1B (continued)

| Compounds No. | IR Spectra ($cm^{-1}$) | NMR spectra (in $CDCl_3$) δ (ppm) |
|---|---|---|
| (66B) | 1760 1590<br>1490 1210 | 3.75(3H), 4.58(2H), 4.65(2H), 5.10-5.43(2H), 5.73-6.30(1H), 7.00(1H), 6.42-6.63(2H), 7.47(1H), 7.70-7.87(2H), 8.48(1H) |
| (67B) | 1755 1590<br>1490 1210 | 1.23(3H), 4.20(2H), 4.57(2H), 5.08-5.43(2H), 4.62(2H), 5.73-6.27(1H), 6.40-6.63(2H), 7.00(1H), 7.47(1H), 7.67-7.85(2H), 8.47(1H) |
| (68B) | 1750 1590<br>1480 1210 | 3.75(3H), 4.60(2H), 6.45-6.67(2H), 7.73(1H), 6.97-7.57(7H), 7.93(1H), 8.80(1H) |
| (69B) | 1750 1590<br>1490 1210 | 1.25(3H), 4.23(2H), 4.58(2H), 6.45-6.67(2H), 7.96-7.57(7H), 7.73(1H), 7.95(1H), 8.80(1H) |
| (70B) | 1740 1580<br>1490 1240 | 0.7-1.0(3H), 1.0-1.7(4H), 1.60(3H), 4.10(2H), 4.5-4.9(3H), 5.1-5.5(2H), 5.7-6.3(1H), 6.45(1H), 6.50(1H), 6.95(1H), 7.45(1H), 7.70(1H), 7.76(1H), 8.50(1H) |

Table 1B (continued)

| Compounds No. | IR Spectra ($cm^{-1}$) | | NMR spectra (in $CDCl_3$) δ (ppm) | |
|---|---|---|---|---|
| (73B) | 3300 | 1760 | 0.7-1.0(3H), | 1.0-2.3(8H), |
| | 1595 | 1320 | 3.70(3H), | 4.65(1H), |
| | 1120 | 1080 | 6.45(1H), | 6.50(1H), |
| | | | 7.00(1H), | 7.50(1H), |
| | | | 7.70(1H), | 7.75(1H), |
| | | | 8.53(1H), | 9.30(1H) |
| (74B) | 1740 | 1590 | 0.7-1.0(3H), | 1.0-1.7(4H), |
| | 1490 | 1215 | 1.65(3H), | 4.10(2H), |
| | | | 4.73(3H), | 6.45(1H), |
| | | | 6.50(1H), | 7.00(1H), |
| | | | 6.9-7.4(5H), | 7.45(1H), |
| | | | 7.70(1H), | 7.93(1H), |
| | | | 8.80(1H) | |
| (75B) | 3300 | 1760 | 3.70(3H), | 4.60(2H), |
| | 1590 | 1325 | 6.45(1H), | 6.50(1H), |
| | 1120 | 1080 | 7.00(1H), | 7.50(1H), |
| | | | 7.6-7.9(2H), | 8.50(1H), |
| | | | 8.70(1H) | |
| (77B) | 3300 | 1760 | 1.05(3H), | 2.00(2H), |
| | 1590 | 1320 | 3.70(3H), | 4.60(1H), |
| | 1120 | 1075 | 6.45(1H), | 6.50(1H), |
| | | | 7.00(1H), | 7.45(1H), |
| | | | 7.70(1H), | 7.75(1H), |
| | | | 8.55(1H), | 9.40(1H) |

Table 1B (continued)

| Compounds No. | IR Spectra ($cm^{-1}$) | | NMR spectra (in $CDCl_3$) δ (ppm) | |
|---|---|---|---|---|
| (78B) | 3300 | 1760 | 1.65(3H), | 3.70(3H), |
| | 1600 | 1320 | 4.75(1H), | 6.35-6.73(2H), |
| | 1120 | 1080 | 7.05-7.70(4H), | 8.55(1H), |
| | | | 9.3(1H) | |
| (79B) | 1740 | 1600 | 1.65(3H), | 2.50(3H), |
| | 1580 | 1165 | 3.70(3H), | 4.76(1H), |
| | 1125 | | 6.40(1H), | 6.55(1H), |
| | | | 7.10(1H), | 7.26(2H), |
| | | | 7.33(1H), | 7.53(1H), |
| | | | 7.75(1H), | 8.15(2H) |
| (80B) | 3100 | 1710 | 1.66(3H), | 3.70(6H), |
| | 1585 | 1505 | 4.75(1H), | 6.52(1H), |
| | 1480 | 1460 | 6.60(1H), | 7.10(1H), |
| | | | 7.20(1H), | 7.26(2H), |
| | | | 7.55(1H), | 7.75(1H), |
| | | | 8.20(2H), | 8.60(1H), |
| | | | 8.80(1H) | |
| (81B) | 1750 | 1600 | 3.70(3H), | 4.82(1H), |
| | 1495 | 1320 | 6.45(1H), | 6.55(1H), |
| | | | 7.10(1H), | 7.4-8.0(12H), |
| | | | 7.88(1H), | 8.90(1H), |
| (82B) | 1730 | 1600 | 1.65(3H), | 3.70(3H), |
| | 1530 | 1330 | 4.75(1H), | 6.45(1H), |
| | | | 6.55(1H), | 7.10(1H), |
| | | | 7.4-8.0(6H), | 8.93(1H) |

Table 1B (continued)

| Compounds No. | IR Spectra ($cm^{-1}$) | NMR spectra (in $CDCl_3$) δ (ppm) |
|---|---|---|
| (83B) | 1755 1600 1525 1490 1325 | 1.70(3H), 3.73(3H), 4.76(1H), 6.43-6.70(2H), 7.00-7.80(6H), 7.93(2H), 8.95(1H) |
| (84B) | 1760 1730 1600 1560 1530 | 1.65(3H), 2.50(3H), 3.70(3H), 4.70(1H), 6.43(1H), 6.60(1H), 6.5-6.7(1H), 7.15(1H), 7.3-7.8(2H), 7.80(1H), 7.97(1H), 8.90(1H) |
| (85B) | 1755 1600 1490 1325 | 1.63(3H), 3.70(3H), 4.73(1H), 5.27(2H), 6.38-6.63(2H), 7.02(1H), 7.38-7.83(5H), 8.20(2H), 8.57(1H) |
| (86B) | 1755 1585 1510 1480 1460 | 1.67(3H), 3.75(3H), 4.80(1H), 6.70(1H), 6.83(1H), 7.30(2H), 7.97(1H), 8.03(1H), 8.20(2H), 8.25(1H), 8.90(1H) |
| (87B) | 1755 1590 1495 1460 | 1.62(3H), 3.70(3H), 4.76(1H), 6.6-7.3(6H), 7.95(1H), 8.10(1H), 8.25(1H), 8.80(1H) |

Table 1B (continued)

| Compounds No. | IR Spectra ($cm^{-1}$) | | NMR spectra (in $CDCl_3$) δ (ppm) | |
|---|---|---|---|---|
| (88B) | 3300 | 1760 | 1.60(3H), | 3.70(3H), |
| | 1595 | 1320 | 4.75(1H), | 6.60(1H), |
| | 1120 | 1080 | 6.75(1H), | 7.80(1H), |
| | | | 7.95(1H), | 8.25(1H), |
| | | | 8.45(1H), | 8.52(1H), |
| (89B) | 1745 | 1600 | 1.67(3H), | 3.72(3H), |
| | 1490 | 1325 | 4.75(1H), | 6.40-6.67(2H), |
| | | | 7.07(1H), | 7.23-8.03(9H), |
| | | | 8.87(1H) | |
| (90B) | 3300 | 3200 | 1.53(3H), | 4.80(1H), |
| | 1675 | 1585 | 6.67(1H), | 6.75(1H), |
| | 1500 | 1480 | 7.2-7.7(4H), | 7.40(2H), |
| | 1460 | | 7.8-8.0(2H), | 8.25(2H), |
| | | | 8.96(1H) | |
| (91B) | 1755 | 1595 | 1.33(9H), | 1.63(3H), |
| | 1490 | 1230 | 4.73(1H), | 6.40-6.67(2H), |
| | | | 6.96-7.57(6H), | 7.75(1H), |
| | | | 7.95(1H), | 8.80(1H) |
| (92B) | 1760 | 1600 | 1.67(3H), | 3.73(3H), |
| | 1490 | 1325 | 3.83(3H), | 4.63(2H), |
| | | | 4.70(2H), | 4.77(1H), |
| | | | 5.10-5.43(2H), | 7.07(1H), |
| | | | 5.67-6.20(1H), | 7.37(1H), |
| | | | 6.40-6.63(2H), | 7.53(1H), |
| | | | 7.77(1H) | |

Table 1B (continued)

| Compounds No. | IR Spectra ($cm^{-1}$) | | NMR spectra (in $CDCl_3$) δ (ppm) | |
|---|---|---|---|---|
| (93B) | 1735 | 1640 | 1.63(3H), | 3.70(3H), |
| | 1600 | 1590 | 3.88(3H), | 4.77(1H), |
| | 1510 | 1490 | 6.40-6.63(2H), | |
| | 1325 | | 7.00-7.73(6H), | 8.13(2H) |
| (94B) | 1755 | 1600 | 1.25(3H), | 1.27(3H), |
| | 1490 | 1325 | 3.68(3H), | 3.80(3H), |
| | | | 4.20(2H), | 4.55(2H), |
| | | | 4.70(1H), | 6.35-6.62(2H), |
| | | | 7.03(1H), | 7.33(1H), |
| | | | 7.50(1H), | 7.72(1H) |
| (95B) | 1750 | 1735 | 1.68(3H), | 3.67(2H), |
| | 1590 | 1510 | 3.72(6H), | 4.78(1H), |
| | 1490 | 1320 | 6.40-6.70(2H), | |
| | | | 7.00-8.00(9H), | 8.93(1H) |
| (96B) | 1750 | 1710 | 1.62(3H), | 3.63(3H), |
| | 1600 | 1500 | 3.80(3H), | 4.65(1H), |
| | 1460 | | 6.32(1H), | 6.53(1H), |
| | | | 6.98(1H), | 7.13(2H), |
| | | | 7.43(1H), | 7.70(1H), |
| | | | 7.87(3H), | 8.70(1H) |
| (97B) | 1760 | 1615 | 1.65(3H), | 3.70(3H), |
| | 1600 | 1495 | 4.70(1H), | 6.33(1H), |
| | 1460 | | 6.56(1H), | 7.03(1H), |
| | | | 7.20(2H), | 7.50(3H), |
| | | | 7.70(1H), | 7.90(1H), |
| | | | 8.73(1H) | |

Table 1B (continued)

| Compounds No. | IR Spectra ($cm^{-1}$) | NMR spectra (in $CDCl_3$) δ (ppm) |
|---|---|---|
| (98B) | 1750 1620 1600 1500 1460 | 3.78(3H), 4.63(2H), 6.45(1H), 6.57(1H), 7.10(1H), 7.23(2H), 7.50(1H), 7.75(1H), 7.88(1H), 8.13(2H), 8.76(1H) |
| (99B) | 1760 1610 1600 1520 1500 | 1.13(3H), 2.05(2H), 3.70(3H), 4.55(1H), 6.35(1H), 6.56(1H), 7.10(1H), 7.23(2H), 7.50(1H), 7.73(1H), 7.87(1H), 8.12(2H), 8.76(1H) |
| (100B) | 1740 1600 1520 1495 1460 | 0.7-1.2(3H), 1.2-1.7(6H), 1.7-2.3(2H), 3.70(3H), 4.57(1H), 6.33(1H), 7.07(1H), 7.25(1H), 7.50(1H), 7.73(1H), 7.90(1H), 8.12(2H), 8.76(1H) |
| (101B) | 3200 1760 1600 1540 1495 1350 1320 1255 | 1.70(3H), 3.73(3H), 4.86(1H), 6.50(1H), 6.57(1H), 7.13(1H), 7.53(1H), 7.75(1H), 8.03(1H), 8.30(4H), 8.97(1H) |

Table 1B (continued)

| Compounds No. | IR Spectra ($cm^{-1}$) | | NMR spectra (in $CDCl_3$) δ (ppm) | |
|---|---|---|---|---|
| (102B) | 3200 | 1765 | 1.70(3H), | 3.73(3H), |
| | 1600 | 1540 | 4.80(1H), | 6.47(1H), |
| | 1490 | 1350 | 6.57(1H), | 7.13(1H), |
| | 1320 | 1255 | 7.53(1H), | 7.75(1H), |
| | | | 8.02(1H) | |
| (103B) | 3200 | 1760 | 1.65(3H), | 3.70(3H), |
| | 1600 | 1540 | 4.75(1H), | 6.46(1H), |
| | 1490 | 1350 | 6.53(1H), | 7.10(1H), |
| | 1320 | 1260 | 7.4-7.8(3H), | 7.95(1H), |
| | | | 8.53(1H), | 8.80(1H), |
| | | | 8.85(1H) | |
| (104B) | 3200 | 1750 | 1.63(3H), | 3.70(3H), |
| | 1600 | 1495 | 4.80(1H), | 6.48(1H), |
| | 1320 | 1250 | 6.58(1H), | 7.05(1H), |
| | | | 7.10(2H), | 7.53(1H), |
| | | | 7.73(1H), | 8.10(2H), |
| | | | 8.20(1H), | 8.95(1H) |
| (105B) | 3200 | 1755 | 1.67(3H), | 3.75(3H), |
| | 1600 | 1495 | 4.00(3H), | 4.80(1H), |
| | 1325 | 1260 | 6.45(1H), | 6.53(1H), |
| | | | 7.10(1H), | 7.50(1H), |
| | | | 7.75(1H), | 7.95(1H), |
| | | | 8.85(1H) | |

Table 1B (continued)

| Compounds No. | IR Spectra ($cm^{-1}$) | | NMR spectra (in $CDCl_3$) δ (ppm) | |
|---|---|---|---|---|
| (106B) | 3200 | 1760 | 1.63(3H), | 3.73(3H), |
| | 1600 | 1590 | 4.75(1H), | 6.40(1H), |
| | 1320 | 1260 | 6.53(1H), | 7.0-8.0(8H), |
| | | | 7.95(1H), | 8.10(1H), |
| | | | 8.80(1H) | |
| (107B) | 3050 | 1755 | 1.67(3H), | 3.75(3H), |
| | 1600 | 1590 | 4.80(1H), | 6.45(1H), |
| | 1430 | 1410 | 6.55(1H), | 7.10(1H), |
| | 1320 | 1260 | 7.57(1H), | 7.77(1H), |
| | | | 7.95(2H), | 8.05(1H), |
| | | | 8.85(2H), | 8.97(1H) |
| (108B) | 3000 | 1750 | 1.63(3H), | 2.60(3H), |
| | 1600 | 1525 | 3.65(3H), | 4.80(1H), |
| | 1495 | 1320 | 6.46(1H), | 6.53(1H), |
| | 1260 | | 7.00(1H), | 7.45(2H), |
| | | | 7.70(1H), | 8.25(4H) |
| (109B) | 3000 | 1755 | 1.75(3H), | 3.05(6H), |
| | 1600 | 1530 | 3.70(3H), | 4.77(1H), |
| | 1490 | 1320 | 6.43(1H), | 6.50(1H), |
| | 1260 | | 6.60(1H), | 7.07(1H), |
| | | | 7.50(1H), | 7.73(1H), |
| | | | 7.95(1H), | 8.10(2H), |
| | | | 8.90(1H) | |

Table 1B (continued)

| Compounds No. | IR Spectra ($cm^{-1}$) | | NMR spectra (in $CDCl_3$) (ppm) | |
|---|---|---|---|---|
| (110B) | 3000 | 1755 | 1.75(3H), | 3.1-3.4(9H), |
| | 1600 | 1495 | 3.70(3H), | 5.20(1H), |
| | 1410 | 1325 | 6.6-6.9(4H), | 7.30(1H), |
| | 1260 | | 7.7-8.1(5H), | 8.90(1H) |
| (111B) | 3020 | 1750 | 1.67(3H), | 3.70(3H), |
| | 1600 | 1490 | 4.80(1H), | 6.50(1H), |
| | 1430 | 1405 | 6.60(1H), | 7.10(1H), |
| | 1320 | 1260 | 7.55(1H), | 7.70(2H), |
| | | | 7.75(1H), | 8.05(1H), |
| | | | 8.23(2H), | 8.95(1H) |
| (112B) | 3020 | 2220 | 1.70(3H), | 3.73(3H), |
| | 1750 | 1600 | 4.80(1H), | 6.50(1H), |
| | 1490 | 1430 | 6.57(1H), | 7.10(1H), |
| | 1405 | 1320 | 7.50(1H), | 7.70(1H), |
| | 1260 | | 7.73(2H), | 8.03(1H), |
| | | | 8.20(2H), | 8.95(1H) |
| (113B) | 3050 | 1750 | 1.70(3H), | 3.77(3H), |
| | 1600 | 1520 | 4.88(1H), | 6.77(1H), |
| | 1495 | 1320 | 6.86(1H), | 8.00(1H), |
| | 1250 | | 8.05(1H), | 8.25(1H), |
| | | | 8.30(4H), | 9.00(1H) |

Table 1B (continued)

| Compounds No. | IR Spectra ($cm^{-1}$) | | NMR spectra (in $CDCl_3$) δ (ppm) | |
|---|---|---|---|---|
| (114B) | 3000 | 1755 | 0.7-1.9(9H), | 3.73(3H), |
| | 1600 | 1530 | 1.9-2.3(2H), | 4.73(1H), |
| | 1495 | 1430 | 6.50(1H), | 6.60(1H), |
| | 1410 | 1320 | 7.20(1H), | 7.55(1H), |
| | 1260 | | 7.76(1H), | 8.05(1H), |
| | | | 8.30(4H), | 9.00(1H) |
| (115B) | 3000 | 1755 | 1.13(3H), | 2.10(2H), |
| | 1600 | 1530 | 3.73(3H), | 4.70(1H), |
| | 1495 | 1430 | 6.50(1H), | 6.60(1H), |
| | 1410 | 1320 | 7.15(1H), | 7.50(1H), |
| | 1260 | | 7.73(1H), | 8.00(1H), |
| | | | 8.30(4H), | 8.95(1H) |
| (116B) | 2900 | 1765 | 3.80(3H), | 4.70(2H), |
| | 1600 | 1530 | 6.53(1H), | 6.60(1H), |
| | 1495 | 1460 | 7.15(1H), | 7.55(1H), |
| | 1330 | 1260 | 7.75(1H), | 8.05(1H), |
| | | | 8.30(4H), | 9.00(1H) |
| (117B) | 3050 | 1755 | 1.63(3H), | 3.70(3H), |
| | 1600 | 1590 | 4.73(1H), | 6.45(1H), |
| | 1430 | 1410 | 6.50(1H), | 6.7-7.6(7H), |
| | 1320 | 1260 | 7.70(2H), | 8.55(1H) |

Table 1B (continued)

| Compounds No. | IR Spectra ($cm^{-1}$) | NMR spectra (in $CDCl_3$) δ (ppm) |
|---|---|---|
| (118B) | 3300 1740<br>1600 1510<br>1490 1320 | 1.63(3H), 3.73(3H),<br>4.80(1H), 6.43-6.67(2H),<br>7.03-7.90(7H), 8.20(1H),<br>8.73(1H) |
| (119B) | 3350 1750<br>1600 1510<br>1490 1320 | 1.63(3H), 3.70(3H),<br>4.77(1H), 6.43-6.67(2H),<br>7.03-7.90(8H), 8.17(1H),<br>8.77(1H) |
| (120B) | 3350 1750<br>1600 1510<br>1490 1320 | 1.65(3H), 3.70(3H),<br>4.77(1H), 6.43-6.67(2H),<br>7.00-7.90(8H), 8.22(1H),<br>8.77(1H) |
| (121B) | 3400 1740<br>1600 1510<br>1490 1320 | 1.65(3H), 3.72(3H),<br>4.77(1H), 6.42-6.65(2H),<br>6.83-7.92(8H), 8.18(1H),<br>8.77(1H) |
| (122B) | 3350 1740<br>1600 1510<br>1490 1320 | 1.67(3H), 3.73(3H),<br>4.82(1H), 6.47-6.67(2H),<br>7.05-8.27(8H), 8.63(1H),<br>8.77(1H) |
| (123B) | 3350 1750<br>1600 1510<br>1490 1320 | 1.67(3H), 3.70(3H),<br>4.77(1H), 6.40-6.67(2H),<br>7.03-7.90(8H), 8.13(1H),<br>8.77(1H) |

Table 1B (continued)

| Compounds No. | IR Spectra ($cm^{-1}$) | | NMR spectra (in $CDCl_3$) δ (ppm) | |
|---|---|---|---|---|
| (124B) | 3100 | 1760 | 1.63(3H), | 3.70(3H), |
| | 1680 | 1600 | 4.70(1H), | 6.50(1H), |
| | 1320 | 1490 | 6.55(1H), | 7.10(1H), |
| | 1260 | | 7.2-7.5(3H), | 7.53(1H), |
| | | | 7.75(1H), | 7.95(1H), |
| | | | 8.80(1H) | |
| (125B) | 3100 | 1750 | 1.63(3H), | 3.70(3H), |
| | 1600 | 1500 | 4.73(1H), | 4.80(2H), |
| | 1460 | 1400 | 6.47(1H), | 6.55(1H), |
| | 1310 | 1260 | 6.90(1H), | 7.0-7.5(3H), |
| | | | 7.53(1H), | 7.77(1H), |
| | | | 7.95(1H), | 8.80(1H) |
| (126B) | 3100 | 1760 | 1.65(3H), | 1.77(3H), |
| | 1600 | 1500 | 3.73(3H), | 4.78(1H), |
| | 1460 | 1400 | 4.93(1H), | 6.45(1H), |
| | 1320 | 1260 | 6.53(1H), | 6.93(1H), |
| | | | 7.10(4H), | 7.56(1H), |
| | | | 7.86(1H), | 7.95(1H), |
| | | | 8.00(1H), | 8.25(1H), |
| | | | 8.83(1H) | |
| (127B) | 3000 | 1760 | 1.63(3H), | 2.20(3H), |
| | 1600 | 1490 | 3.70(3H), | 4.76(1H), |
| | 1430 | 1320 | 6.46(1H), | 6.53(1H), |
| | 1260 | | 7.10(1H), | 7.50(1H), |
| | | | 7.73(1H), | 7.95(1H), |
| | | | 8.73(1H) | |

Table 1B (continued)

| Compounds No. | IR Spectra ($cm^{-1}$) | | NMR spectra (in $CDCl_3$) δ (ppm) | |
|---|---|---|---|---|
| (128B) | 3000 | 1760 | 1.63(3H), | 1.75(3H), |
| | 1600 | 1505 | 3.70(3H), | 4.70(1H), |
| | 1485 | 1325 | 4.85(1H), | 6.45(1H), |
| | 1260 | | 6.53(1H), | 7.00(4H), |
| | | | 7.10(1H), | 6.9-7.5(2H), |
| | | | 7.50(1H), | 7.80(1H), |
| | | | 7.97(1H), | 8.40(1H), |
| | | | 8.80(1H) | |
| (129B) | 3000 | 1780 | 1.37(3H), | 1.63(3H), |
| | 1760 | 1600 | 3.70(3H), | 4.30(2H), |
| | 1495 | 1430 | 4.75(1H), | 6.45(1H), |
| | 1320 | 1260 | 6.53(1H), | 7,10(1H), |
| | | | 7.50(1H), | 7.75(1H), |
| | | | 7.95(1H), | 8.75(1H), |
| (130B) | 2900 | 1760 | 0.6-1.8(17H), | 1.63(3H), |
| | 1600 | 1490 | 2.45(2H), | 3.67(3H), |
| | 1430 | 1320 | 4.75(1H), | 6.45(1H), |
| | 1260 | | 6.53(1H), | 7.10(1H), |
| | | | 7.50(1H), | 7.73(1H), |
| | | | 7.95(1H), | 8.73(1H) |
| (131B) | 3000 | 1760 | 1.63(3H), | 2.40(3H), |
| | 1600 | 1500 | 3.70(3H), | 4.75(1H), |
| | 1440 | 1320 | 6.30(1H), | 6.53(1H), |
| | 1260 | | 7.10(1H), | 7.53(1H), |
| | | | 7.75(1H), | 7.98(1H), |
| | | | 8.90(1H) | |

Table 1B (continued)

| Compounds No. | IR Spectra ($cm^{-1}$) | | NMR spectra (in $CDCl_3$) δ (ppm) | |
|---|---|---|---|---|
| (132B) | 3100 | 2955 | 1.67(3H), | 2.20(3H), |
| | 1760 | 1755 | 3.73(3H), | 4.80(1H), |
| | 1600 | 1500 | 6.73(1H), | 6.80(1H), |
| | 1460 | 1320 | 7.97(1H), | 8.03(1H), |
| | 1270 | | 8.25(1H), | 8.80(1H) |
| (133B) | 3100 | 1760 | 1.63(3H), | 3.70(3H), |
| | 1600 | 1500 | 4.10(2H), | 4.70(1H), |
| | 1420 | 1320 | 6.45(1H), | 6.53(1H), |
| | 1260 | | 7.10(1H), | 7.50(1H), |
| | | | 7.73(2H), | 8.50(1H) |
| (134B) | 3350 | 1740 | 1.03(3H), | 1.23-1.70(4H), |
| | 1600 | 1510 | 1.65(3H), | 3.36(2H), |
| | 1490 | 1320 | 3.73(3H), | 4.75(1H), |
| | | | 6.07-6.40(1H), | 7.10(1H), |
| | | | 6.43-6.68(2H), | 7.57(1H), |
| | | | 7.76-7.90(2H), | 8.75(1H) |
| (135B) | 3400 | 1740 | 1.67(3H), | 3.70(3H), |
| | 1600 | 1490 | 3.17-4.43(2H), | 4.73(1H), |
| | 1320 | | 6.37-6.63(2H), | 8.03(1H), |
| | | | 6.77-7.70(7H), | 8.85(1H) |

Table 1B (continued)

| Compounds No. | IR Spectra ($cm^{-1}$) | NMR spectra (in $CDCl_3$) δ (ppm) |
|---|---|---|
| (136B) | 3350 1740 1660 1590 1490 1320 | 1.67(3H), 3.03(6H), 3.72(3H), 4.77(1H), 6.40-6.65(2H), 6.93(1H), 7.17-8.12(8H), 8.92(1H) |
| (137B) | 3350 1750 1600 1490 1320 | 1.28(3H), 1.68(3H), 3.70(3H), 4.20(2H), 4.73(1H), 6.40-6.62(2H), 6.97-7.70(6H), 7.95-8.10(3H), 8.87(1H) |
| (138B) | 1755 1600 1490 1320 | 1.65(3H), 3.70(3H), 4.75(1H), 6.32(1H), 6.63(1H), 7.10(1H), 7.3-7.7(4H), 7.75(1H), 8.07(1H), 8.10(2H), 8.85(1H) |
| (139B) | 1760 1590 1490 1460 | 1.60(3H), 2.30(6H), 2.53(3H), 3.70(3H), 4.72(1H), 6.40(1H), 6.50(1H), 6.95(1H), 7.02(2H), 7.55(1H), 7.70(1H), 7.80(1H), 8.73(1H) |

Table 1B (continued)

| Compounds No. | IR Spectra ($cm^{-1}$) | | NMR spectra (in $CDCl_3$) δ (ppm) | |
|---|---|---|---|---|
| (140B) | 1750 | 1730 | 1.33(9H), | 1.65(3H), |
| | 1610 | 1490 | 3.70(3H), | 4.75(1H), |
| | | | 6.40-6.67(2H), | 7.07(1H), |
| | | | 7.38-7.57(3H), | 7.71(1H), |
| | | | 7.95-8.13(3H), | 8.90(1H) |
| (141B) | 3300 | 1760 | 1.68(3H), | 3.71(3H), |
| | 1730 | 1675 | 4.18(2H), | 4.76(1H), |
| | 1600 | | 6.40-6.63(2H), | 7.08(1H), |
| | | | 7.25-8.17(7H), | 8.60(1H), |
| | | | 8.90(1H) | |
| (142B) | 3350 | 1750 | 1.33(9H), | 1.67(3H), |
| | 1740 | 1690 | 3.71(3H), | 4.76(1H), |
| | 1595 | | 6.40-6.63(2H), | 7.08(1H), |
| | | | 7.25-7.73(5H), | |
| | | | 8.95-8.13(3H), | 8.93(1H) |
| (143B) | 1760 | 1600 | 1.65(3H), | 3.70(3H), |
| | 1490 | | 4.75(1H), | 6.45(1H), |
| | | | 6.55(1H), | 7.10(1H), |
| | | | 7.4-7.8(4H), | 7.95(1H), |
| | | | 8.00(1H), | 8.90(1H), |
| | | | 8.95(1H) | |

Table 1B (continued)

| Compounds No. | IR Spectra ($cm^{-1}$) | NMR spectra (in $CDCl_3$) δ (ppm) |
|---|---|---|
| (145B) | 3300 1740 1600 1580 1165 1120 | 1.60(3H), 2.30(3H), 3.70(3H), 4.70(1H), 6.45(1H), 6.50(1H), 7.00(1H), 7.40(1H), 7.45(1H), 7.70(1H), 9.80(1H) |
| (146B) | 3300 1600 1580 1510 1480 | 6.5-6.7(2H), 7.1-7.4(4H), 7.50(1H), 7.75(1H), 8.22(2H), 8.53(1H), 9.40(1H) |
| (147B) | 3350 2250 1750 1690 | 1.23(9H), 1.62(3H), 3.70(3H), 4.75(1H), 6.40-6.71(3H), 7.33-7.63(2H), 7.08(1H), 7.70-7.90(2H), 8.55(1H) |
| (148B) | 2220 1750 1600 1520 1490 | 1.65(3H), 3.70(3H), 5.50(1H), 4.80(1H), 6.45(1H), 6.50(1H), 7.10(1H), 7.3-7.7(4H), 8.00(1H), 8.16(2H) 8.93(1H) |
| (149B) | 3350 1750 1680 1600 | 1.60(3H), 3.67(3H), 4.68(1H), 6.37-6.55(2H), 6.68-8.02(10H), 8.45(1H) |

Table 1B (continued)

| Compounds No. | IR Spectra ($cm^{-1}$) | NMR spectra (in $CDCl_3$) δ (ppm) |
|---|---|---|
| (150B) | 3350 2200<br>1755 1600<br>1490 | 1.60(3H), 3.70(3H),<br>4.6-5.0(3H), 5.3-5.6(1H),<br>6.3-6.7(3H), 7.00(1H),<br>7.50(1H), 7.70(1H),<br>7.75(1H), 8.80(1H) |
| (151B) | 3350 1750<br>1735 1600<br>1490 1320 | 1.27(3H), 1.63(3H),<br>3.73(3H), 4.22(2H),<br>4.75(1H), 6.27-6.77(4H),<br>7.07(1H), 7.53(1H),<br>7.73-7.91(2H), 8.57(1H) |
| (152B) | 3350 2250<br>1750 1730<br>1600 1490<br>1320 | 1.63(3H), 3.73(3H),<br>4.76(1H), 6.40-6.63(3H),<br>7.43(1H), 7.57(1H),<br>7.73-8.33(3H), 8.63(1H) |
| (153B) | 3350 1750<br>1690 1600 | 1.60(3H), 3.70(3H),<br>4.73(1H), 6.40-6.60(2H),<br>6.83(1H), 7.00-7.86(8H),<br>8.37(1H), 8.53(1H) |
| (154B) | 3350 1740<br>1670 1590<br>1320 | 1.73(3H), 3.77(3H),<br>5.00(1H), 6.50-6.70(2H),<br>7.03-7.33(4H), 7.60(1H),<br>7.76(1H), 8.03-8.23(3H),<br>11.53(1H) |

Table 1B (continued)

| Compounds No. | IR Spectra ($cm^{-1}$) | | NMR spectra (in $CDCl_3$) δ (ppm) | |
|---|---|---|---|---|
| (155B) | 3350 | 1750 | 1.30(3H), | 1.77(3H), |
| | 1670 | 1600 | 3.80(3H), | 4.25(2H), |
| | 1320 | | 4.60(2H), | 4.95(1H), |
| | | | 6.53(1H), | 6.60(1H), |
| | | | 7.10(1H), | 7.57(1H), |
| | | | 7.75(1H), | 8.13(1H), |
| | | | 11.03(1H) | |
| (156B) | 3350 | 1750 | 1.75(3H), | 3.80(3H), |
| | 1670 | 1600 | 4.60(2H), | 4.70(2H), |
| | 1490 | 1320 | 4.93(1H), | 5.10-5.40(2H), |
| | | | 5.67-6.27(1H), | 6.50(1H), |
| | | | 6.55(1H), | 7.08(1H), |
| | | | 7.53(1H), | 7.72(1H), |
| | | | 8.10(1H), | 11.00(1H) |
| (26B/R-enantiomer) | 1750 | 1590 | 1.65(3H), | 3.68(3H), |
| | 1510 | 1320 | 4.73(1H), | 6.37-6.63(2H), |
| | 1125 | 1080 | 6.97-7.70(5H), | |
| | | | 7.78-8.18(3H), | 8.78(1H) |
| (2B/R-enantiomer) | 3300 | 1755 | 1.62(3H), | 3.70(3H), |
| | 1595 | 1320 | 4.72(1H), | 6.40-6.63(2H), |
| | 1120 | 1080 | 7.00(1H), | 7.47(1H), |
| | | | 7.63-7.80(2H), | 8.50(1H), |
| | | | 8.93(1H) | |

FORMULATION EXAMPLE 1C

One part of the active compound of this invention was added to 5000 parts of a mixture of acetone and water (1 : 1 by volume), and 2.6 parts of a nonionic surfactant (Sorpol 2680, tradename) was added to form

a solution.

## FORMULATION EXAMPLE 2C

One part of the active compound of this invention, 8.7 parts of a mixture of equal amounts of talc and bentonite, and 0.3 part of a mixture of equal amounts of Sorpol 5060 (tradename) and Sorpol 800A (tradename) were well pulverized and mixed to form a wettable powder.

## TEST EXAMPLE 1C

A solution of the active compound of the invention was prepared in accordance with the above Formulation Example 1C.

Seeds of plants were sown in the soil, and after germination, cultivated for 2 to 3 weeks.

The prepared solution was applied to these plants at the rate of application indicated in Table 1C. Thereafter, the plants were continued to be cultivated for 3 weeks without applying the above solution. The results are given in Table 1C.

## TEST EXAMPLE 2C

Seeds of plants to be tested were sown in the soil, and on the second day after sowing, were treated as follows and the growth of the plants was observed for 3 weeks.

A wettable powder containing each of the active compounds of this invention which was prepared in accordance with Formulation Example 2 was suspended in 150 $cc/m^2$ of water so that the total amount of the active compounds became 0.1 $g/m^2$. The suspension was uniformly applied to the surface of the soil after the sowing. Thereafter, without further applying the test chemical, the plants were grown. The results are shown in Table 2C.

The alphabet characters in the plant column of Tables 1C and 2C symbolize the following plants:

a: Digitaria adscendens
b: Setaria viridis
c: Sorghum halepense
d: Chenopodium album var. centrorubrum
e: Amaranthus mangostanus
f: Astragalus sinicus
g: Abutilon theophrosti
h: Solanum nigrum
i: Xanthium strumarium
j: Soybean; Glycine max
k: Corn; Zea mays

Table 1C

| Compound No. | Rate of Application (kg/ha) | Plants | | | | | | | | | | |
|---|---|---|---|---|---|---|---|---|---|---|---|---|
| | | a | b | c | d | e | f | g | h | i | j | k |
| (1B) | 0.5 | 2 | 1 | 1 | 2 | 1 | 1 | 5 | 4 | 1 | 1 | 0 |
| (2B) | 0.5 | 4 | 2 | 2 | 5 | 5 | 5 | 5 | 5 | 5 | 3 | 0 |
| | 0.125 | 3 | 1 | 1 | 2 | 4 | 3 | 5 | 4 | 3 | 1 | 0 |
| (3B) | 0.5 | 2 | 2 | 2 | 5 | 5 | 3 | 5 | 5 | 5 | 3 | 1 |
| | 0.125 | 2 | 2 | 2 | 5 | 2 | 3 | 5 | 5 | 5 | 1 | 0 |
| (5B) | 0.5 | 3 | 2 | 3 | 5 | 5 | 4 | 5 | 5 | 2 | 3 | 0 |
| (6B) | 0.5 | 2 | 1 | 1 | 4 | 5 | 4 | 5 | 3 | 5 | 3 | 1 |
| (7B) | 0.5 | 2 | 2 | 1 | 2 | 5 | 3 | 5 | 3 | 5 | 3 | 0 |
| (8B) | 0.5 | 2 | 1 | 1 | 3 | 5 | 3 | 5 | 4 | 5 | 2 | 1 |
| (11B) | 0.5 | 5 | 3 | 3 | 5 | 5 | 4 | 5 | 5 | 5 | 5 | 1 |
| | 0.125 | 2 | 2 | 2 | 4 | 5 | 3 | 5 | 5 | 5 | 3 | 0 |
| (12B) | 0.5 | 4 | 3 | 3 | 5 | 5 | 4 | 5 | 5 | 5 | 4 | 0 |
| | 0.125 | 1 | 1 | 1 | 5 | 3 | 2 | 5 | 3 | 4 | 3 | 0 |
| (13B) | 0.5 | 2 | 1 | 1 | 4 | 5 | 2 | 5 | 5 | 2 | 3 | 0 |
| | 0.125 | 1 | 1 | 1 | 2 | 2 | 1 | 5 | 3 | 1 | 3 | 0 |
| (14B) | 0.5 | 4- | 3 | 4 | 5 | 5 | 4 | 5 | 5 | 5 | 4 | 1 |
| | 0.125 | 3 | 2 | 3 | 5 | 5 | 4 | 5 | 5 | 5 | 3 | 1 |
| (15B) | 0.5 | 3 | 1 | 2 | 5 | 5 | 4 | 5 | 4 | 3 | 2 | 0 |
| | 0.125 | 1 | 1 | 1 | 5 | 4 | 3 | 4 | 3 | 2 | 1 | 0 |
| (16B) | 0.5 | 1 | 1 | 1 | 4 | 3 | 3 | 5 | 5 | 2 | 4 | 1 |
| | 0.125 | 1 | 1 | 1 | 2 | 2 | 3 | 5 | 2 | 2 | 1 | 0 |
| (17B) | 0.5 | 2 | 1 | 1 | 4 | 3 | 3 | 5 | 5 | 3 | 2 | 0 |
| (18B) | 0.5 | 1 | 1 | 1 | 2 | 1 | 2 | 4 | 3 | 3 | 2 | 0 |
| (19B) | 0.5 | 4 | 4 | 4 | 5 | 5 | 4 | 5 | 5 | 3 | 3 | 2 |
| | 0.125 | 3 | 2 | 3 | 4 | 3 | 2 | 5 | 5 | 3 | 3 | 1 |
| (20B) | 0.5 | 5 | 5 | 5 | 5 | 5 | 5 | 5 | 5 | 5 | 5 | 5 |
| | 0.125 | 5 | 4 | 5 | 5 | 5 | 5 | 5 | 5 | 5 | 4 | 3 |
| | 0.031 | 4 | 1 | 3 | 5 | 5 | 4 | 5 | 5 | 5 | 4 | 2 |
| (21B) | 0.5 | 5 | 5 | 5 | 5 | 5 | 5 | 5 | 5 | 5 | 5 | 5 |
| | 0.125 | 5 | 5 | 5 | 5 | 5 | 4 | 5 | 5 | 5 | 4 | 2 |
| | 0.031 | 3 | 2 | 3 | 5 | 5 | 3 | 5 | 5 | 5 | 4 | 1 |

Table 1C (continued)

| Compound No. | Rate of Application (kg/ha) | Plants | | | | | | | | | | |
|---|---|---|---|---|---|---|---|---|---|---|---|---|
| | | a | b | c | d | e | f | g | h | i | j | k |
| (22B) | 0.5 | 5 | 4 | 5 | 5 | 5 | 5 | 5 | 5 | 5 | 5 | 5 |
| | 0.125 | 5 | 3 | 5 | 5 | 5 | 4 | 5 | 5 | 5 | 5 | 4 |
| | 0.031 | 3 | 1 | 1 | 5 | 5 | 4 | 5 | 5 | 5 | 3 | 2 |
| (23B) | 0.5 | 5 | 4 | 5 | 5 | 5 | 5 | 5 | 5 | 5 | 5 | 5 |
| | 0.125 | 4 | 2 | 4 | 5 | 5 | 5 | 5 | 5 | 5 | 4 | 3 |
| | 0.031 | 2 | 1 | 1 | 5 | 5 | 4 | 5 | 5 | 4 | 2 | 1 |
| (24B) | 0.5 | 5 | 5 | 5 | 5 | 5 | 5 | 5 | 5 | 5 | 5 | 5 |
| | 0.125 | 5 | 4 | 5 | 5 | 5 | 4 | 5 | 5 | 5 | 5 | 3 |
| | 0.031 | 2 | 1 | 2 | 5 | 5 | 5 | 5 | 5 | 5 | 4 | 2 |
| (25B) | 0.5 | 5 | 5 | 5 | 5 | 5 | 5 | 5 | 5 | 5 | 5 | 5 |
| | 0.125 | 5 | 3 | 5 | 5 | 5 | 5 | 5 | 5 | 5 | 4 | 5 |
| | 0.031 | 2 | 1 | 1 | 3 | 3 | 4 | 5 | 5 | 5 | 3 | 2 |
| (26B) | 0.5 | 5 | 5 | 5 | 5 | 5 | 5 | 5 | 5 | 5 | 5 | 5 |
| | 0.125 | 5 | 5 | 5 | 5 | 5 | 5 | 5 | 5 | 5 | 5 | 5 |
| | 0.031 | -3 | 2 | 4 | 5 | 5 | 4 | 5 | 5 | 5 | 5 | 4 |
| (26B/R-enantiomer) | 0.125 | 5 | 5 | 5 | 5 | 5 | 5 | 5 | 5 | 5 | 5 | 5 |
| | 0.04 | 5 | 4 | 5 | 5 | 5 | 5 | 5 | 5 | 5 | 5 | 5 |
| (27B) | 0.5 | 5 | 5 | 5 | 5 | 5 | 5 | 5 | 5 | 5 | 5 | 5 |
| | 0.125 | 5 | 2 | 5 | 5 | 5 | 4 | 5 | 5 | 5 | 5 | 5 |
| | 0.031 | 3 | 1 | 3 | 4 | 5 | 3 | 5 | 5 | 5 | 4 | 3 |
| (28B) | 0.5 | 5 | 5 | 5 | 5 | 5 | 5 | 5 | 5 | 5 | 5 | 5 |
| | 0.125 | 5 | 2 | 3 | 5 | 5 | 4 | 5 | 5 | 5 | 4 | 3 |
| | 0.031 | 2 | 2 | 2 | 1 | 3 | 3 | 5 | 5 | 5 | 3 | 1 |
| (29B) | 0.5 | 5 | 5 | 5 | 5 | 5 | 5 | 5 | 5 | 5 | 5 | 5 |
| | 0.125 | 5 | 2 | 5 | 5 | 5 | 5 | 5 | 5 | 5 | 4 | 2 |
| | 0.031 | 2 | 1 | 1 | 3 | 5 | 3 | 5 | 5 | 2 | 4 | 1 |
| (30B) | 0.5 | 5 | 4 | 5 | 5 | 5 | 5 | 5 | 5 | 5 | 5 | 5 |
| | 0.125 | 4 | 2 | 3 | 5 | 5 | 5 | 5 | 5 | 5 | 4 | 3 |
| | 0.031 | 3 | 1 | 2 | 5 | 5 | 4 | 5 | 5 | 5 | 4 | 1 |
| (31B) | 0.5 | 5 | 5 | 5 | 5 | 5 | 5 | 5 | 5 | 5 | 5 | 5 |
| | 0.125 | 4 | 2 | 5 | 5 | 5 | 5 | 5 | 5 | 5 | 4 | 4 |

Table 1C (continued)

| Compound No. | Rate of Application (kg/ha) | Plants a | b | c | d | e | f | g | h | i | j | k |
|---|---|---|---|---|---|---|---|---|---|---|---|---|
| (32B) | 0.5 | 5 | 5 | 5 | 5 | 5 | 5 | 5 | 5 | 5 | 5 | 3 |
| | 0.125 | 4 | 3 | 5 | 5 | 5 | 4 | 5 | 5 | 5 | 5 | 2 |
| | 0.031 | 3 | 1 | 3 | 2 | 4 | 3 | 5 | 5 | 5 | 4 | 1 |
| (33B) | 0.5 | 5 | 4 | 5 | 5 | 5 | 5 | 5 | 5 | 5 | 5 | 3 |
| | 0.125 | 4 | 2 | 3 | 5 | 5 | 4 | 5 | 5 | 5 | 4 | 1 |
| | 0.031 | 2 | 1 | 1 | 5 | 5 | 4 | 5 | 5 | 4 | 2 | 0 |
| (34B) | 0.5 | 5 | 4 | 5 | 5 | 5 | 5 | 5 | 5 | 5 | 5 | 4 |
| | 0.125 | 4 | 2 | 5 | 5 | 5 | 4 | 5 | 5 | 5 | 4 | 3 |
| | 0.031 | 3 | 1 | 2 | 5 | 5 | 4 | 5 | 5 | 3 | 4 | 0 |
| (35B) | 0.5 | 5 | 4 | 5 | 5 | 5 | 5 | 5 | 5 | 5 | 5 | 2 |
| | 0.125 | 3 | 1 | 4 | 5 | 5 | 4 | 5 | 5 | 5 | 4 | 0 |
| | 0.031 | 1 | 1 | 1 | 2 | 5 | 3 | 5 | 4 | 5 | 3 | 0 |
| (36B) | 0.5 | 5 | 5 | 5 | 5 | 5 | 5 | 5 | 5 | 5 | 5 | 5 |
| | 0.125 | 4 | 3 | 5 | 5 | 5 | 4 | 5 | 5 | 5 | 4 | 2 |
| | 0.031 | 1 | 1 | 3 | 5 | 2 | 4 | 5 | 5 | 5 | 4 | 2 |
| (37B) | 0.5 | 5- | 5 | 5 | 5 | 5 | 5 | 5 | 5 | 5 | 5 | 5 |
| | 0.125 | 5 | 2 | 5 | 5 | 5 | 5 | 5 | 5 | 5 | 5 | 5 |
| | 0.031 | 3 | 1 | 5 | 5 | 3 | 5 | 5 | 5 | 5 | 5 | 1 |
| (38B) | 0.5 | 0 | 0 | 0 | 1 | 1 | 1 | 5 | 3 | 2 | 1 | 0 |
| (39B) | 0.5 | 0 | 0 | 0 | 1 | 1 | 1 | 5 | 3 | 1 | 1 | 0 |
| (40B) | 0.5 | 0 | 0 | 0 | 1 | 1 | 1 | 5 | 3 | 5 | 1 | 0 |
| (41B) | 0.5 | 0 | 0 | 0 | 1 | 1 | 1 | 5 | 5 | 1 | 1 | 0 |
| (43B) | 0.5 | 1 | 0 | 0 | 1 | 1 | 1 | 5 | 3 | 1 | 1 | 0 |
| (46B) | 0.5 | 5 | 2 | 3 | 3 | 5 | 4 | 5 | 5 | 2 | 3 | 3 |
| | 0.125 | 1 | 1 | 2 | 1 | 1 | 1 | 5 | 1 | 2 | 3 | 0 |
| (47B) | 0.5 | 3 | 1 | 2 | 3 | 5 | 2 | 5 | 3 | 5 | 3 | 3 |
| | 0.125 | 1 | 1 | 1 | 1 | 2 | 2 | 5 | 1 | 1 | 2 | 0 |
| (48B) | 0.5 | 2 | 1 | 1 | 2 | 5 | 3 | 5 | 5 | 5 | 3 | 2 |
| | 0.125 | 1 | 1 | 1 | 1 | 1 | 1 | 4 | 3 | 1 | 1 | 0 |
| (49B) | 0.5 | 2 | 2 | 2 | 2 | 2 | 2 | 5 | 5 | 5 | 3 | 3 |
| | 0.125 | 1 | 1 | 1 | 1 | 1 | 1 | 4 | 3 | 1 | 1 | 0 |

Table 1C (continued)

| Compound No. | Rate of Application (kg/ha) | Plants a | b | c | d | e | f | g | h | i | j | k |
|---|---|---|---|---|---|---|---|---|---|---|---|---|
| (50B) | 0.5 | 5 | 5 | 5 | 5 | 5 | 5 | 5 | 5 | 5 | 5 | 5 |
| | 0.125 | 5 | 4 | 4 | 5 | 5 | 5 | 5 | 5 | 5 | 5 | 3 |
| | 0.031 | 3 | 1 | 3 | 5 | 5 | 4 | 5 | 5 | 5 | 4 | 3 |
| (51B) | 0.5 | 5 | 3 | 3 | 5 | 5 | 5 | 5 | 5 | 5 | 4 | 3 |
| | 0.125 | 5 | 2 | 3 | 5 | 5 | 5 | 5 | 5 | 5 | 4 | 1 |
| | 0.031 | 2 | 1 | 1 | 4 | 3 | 3 | 5 | 5 | 1 | 1 | 0 |
| (52B) | 0.5 | 5 | 5 | 5 | 5 | 5 | 5 | 5 | 5 | 5 | 5 | 5 |
| | 0.125 | 5 | 4 | 5 | 4 | 5 | 5 | 5 | 5 | 5 | 5 | 3 |
| | 0.031 | 3 | 2 | 4 | 3 | 5 | 4 | 5 | 5 | 5 | 3 | 2 |
| (53B) | 0.5 | 5 | 4 | 5 | 5 | 5 | 5 | 5 | 5 | 5 | 5 | 5 |
| | 0.125 | 4 | 4 | 4 | 4 | 5 | 4 | 5 | 5 | 5 | 5 | 2 |
| | 0.031 | 3 | 1 | 3 | 3 | 5 | 4 | 5 | 5 | 5 | 3 | 0 |
| (54B) | 0.5 | 5 | 4 | 5 | 5 | 5 | 5 | 5 | 5 | 5 | 5 | 5 |
| | 0.125 | 5 | 1 | 3 | 5 | 5 | 4 | 5 | 5 | 5 | 4 | 2 |
| | 0.031 | 3 | 1 | 3 | 5 | 5 | 4 | 5 | 5 | 5 | 4 | 1 |
| (55B) | 0.5 | 5 | 4 | 5 | 5 | 5 | 5 | 5 | 5 | 5 | 5 | 4 |
| | 0.125 | 5 | 3 | 5 | 5 | 5 | 5 | 5 | 5 | 5 | 4 | 2 |
| | 0.031 | 3 | 1 | 2 | 5 | 3 | 4 | 5 | 5 | 4 | 4 | 0 |
| (56B) | 0.5 | 5 | 4 | 5 | 5 | 5 | 5 | 5 | 5 | 5 | 4 | 2 |
| | 0.125 | 4 | 2 | 3 | 5 | 5 | 5 | 5 | 5 | 5 | 4 | 2 |
| | 0.031 | 3 | 1 | 1 | 4 | 5 | 4 | 5 | 5 | 5 | 3 | 0 |
| (59B) | 0.5 | 2 | 1 | 1 | 3 | 1 | 3 | 5 | 5 | 4 | 3 | 0 |
| | 0.125 | 1 | 1 | 1 | 2 | 1 | 1 | 5 | 1 | 1 | 2 | 0 |
| (60B) | 0.5 | 2 | 1 | 1 | 1 | 3 | 3 | 5 | 4 | 1 | 3 | 1 |
| | 0.125 | 1 | 1 | 1 | 1 | 1 | 1 | 5 | 1 | 1 | 1 | 0 |
| (61B) | 0.5 | 0 | 0 | 0 | 1 | 1 | 1 | 5 | 4 | 1 | 1 | 0 |
| (63B) | 0.5 | 5 | 3 | 4 | 5 | 5 | 5 | 5 | 5 | 5 | 5 | 3 |
| | 0.125 | 2 | 1 | 2 | 5 | 5 | 5 | 5 | 5 | 5 | 5 | 1 |
| | 0.031 | 1 | 1 | 1 | 4 | 5 | 4 | 5 | 4 | 5 | 4 | 0 |
| (65B) | 0.5 | 5 | 3 | 4 | 5 | 5 | 3 | 5 | 5 | 5 | 4 | 0 |
| | 0.125 | 2 | 2 | 1 | 1 | 5 | 1 | 5 | 2 | 5 | 3 | 0 |

Table 1C (continued)

| Compound No. | Rate of Application (kg/ha) | Plants | | | | | | | | | | |
|---|---|---|---|---|---|---|---|---|---|---|---|---|
| | | a | b | c | d | e | f | g | h | i | j | k |
| (66B) | 0.5 | 3 | 1 | 3 | 5 | 5 | 1 | 5 | 5 | 5 | 4 | 0 |
| | 0.125 | 2 | 1 | 1 | 2 | 5 | 1 | 5 | 2 | 5 | 3 | 0 |
| | 0.031 | 1 | 1 | 1 | 5 | 1 | 1 | 4 | 1 | 3 | 1 | 0 |
| (67B) | 0.5 | 3 | 1 | 1 | 5 | 5 | 1 | 5 | 5 | 3 | 1 | 0 |
| | 0.125 | 1 | 1 | 1 | 3 | 2 | 1 | 5 | 2 | 5 | 1 | 0 |
| (68B) | 0.5 | 4 | 4 | 5 | 5 | 5 | 3 | 5 | 5 | 5 | 4 | 1 |
| | 0.125 | 3 | 1 | 1 | 5 | 4 | 2 | 5 | 5 | 5 | 3 | 1 |
| | 0.031 | 2 | 1 | 1 | 5 | 4 | 1 | 5 | 2 | 5 | 2 | 0 |
| (69B) | 0.5 | 3 | 3 | 4 | 5 | 5 | 4 | 5 | 5 | 5 | 4 | 0 |
| | 0.125 | 3 | 1 | 3 | 5 | 2 | 2 | 5 | 5 | 5 | 4 | 0 |
| | 0.031 | 1 | 1 | 1 | 5 | 2 | 2 | 5 | 1 | 5 | 3 | 0 |
| (70B) | 0.5 | 4 | 2 | 4 | 5 | 5 | 4 | 5 | 5 | 5 | 5 | 0 |
| | 0.125 | 2 | 1 | 1 | 5 | 5 | 2 | 5 | 5 | 5 | 1 | 0 |
| (74B) | 0.5 | 4 | 3 | 4 | 5 | 5 | 5 | 5 | 5 | 5 | 5 | 1 |
| | 0.125 | 3 | 1 | 2 | 5 | 5 | 5 | 5 | 5 | 5 | 5 | 1 |
| | 0.031 | 1- | 1 | 1 | 2 | 1 | 1 | 5 | 5 | 1 | 2 | 1 |
| (79B) | 0.5 | 0 | 0 | 0 | 1 | 1 | 1 | 5 | 4 | 1 | 1 | 0 |
| (80B) | 0.5 | 3 | 3 | 3 | 5 | 3 | 5 | 5 | 5 | 5 | 5 | 1 |
| | 0.125 | 2 | 0 | 0 | 5 | 1 | 5 | 5 | 5 | 5 | 4 | 0 |
| | 0.031 | 0 | 0 | 0 | 1 | 1 | 3 | 5 | 5 | 1 | 2 | 0 |
| (81B) | 0.5 | 1 | 1 | 1 | 1 | 1 | 3 | 5 | 5 | 5 | 3 | 0 |
| | 0.125 | 0 | 0 | 0 | 1 | 4 | 0 | 5 | 2 | 5 | 3 | 0 |
| (82B) | 0.125 | 5 | 5 | 5 | 5 | 5 | 5 | 5 | 5 | 5 | 5 | 5 |
| (83B) | 0.125 | 5 | 4 | 5 | 5 | 5 | 5 | 5 | 5 | 5 | 5 | 5 |
| (84B) | 0.5 | 5 | 3 | 5 | 5 | 5 | 5 | 5 | 5 | 5 | 4 | 1 |
| (85B) | 0.5 | 2 | 0 | 2 | 2 | 5 | 3 | 4 | 5 | 2 | 1 | 0 |
| (86B) | 0.5 | 5 | 5 | 5 | 5 | 5 | 4 | 5 | 5 | 5 | 5 | 1 |
| | 0.125 | 5 | 4 | 5 | 5 | 4 | 4 | 5 | 5 | 5 | 4 | 1 |

Table 1C (continued)

| Compound No. | Rate of Application (kg/ha) | Plants | | | | | | | | | | |
|---|---|---|---|---|---|---|---|---|---|---|---|---|
| | | a | b | c | d | e | f | g | h | i | j | k |
| (87B) | 0.5 | 5 | 5 | 5 | 5 | 5 | 5 | 5 | 5 | 5 | 4 | 2 |
| | 0.125 | 4 | 4 | 5 | 5 | 5 | 5 | 5 | 5 | 5 | 4 | 0 |
| (88B) | 0.125 | 1 | 0 | 0 | 0 | 0 | 0 | 5 | 1 | 1 | 2 | 1 |
| (89B) | 0.125 | 4 | 3 | 4 | 5 | 5 | 5 | 5 | 5 | 5 | 5 | 4 |
| (90B) | 0.125 | 3 | 3 | 5 | 5 | 2 | 5 | 5 | 5 | 5 | 5 | 2 |
| (91B) | 0.125 | 5 | 3 | 3 | 5 | 1 | 5 | 5 | 5 | 5 | 5 | 5 |
| | 0.031 | 4 | 3 | 3 | 5 | 1 | 4 | 5 | 5 | 5 | 4 | 2 |
| (92B) | 1.0 | 0 | 0 | 0 | 1 | 0 | 1 | 3 | 2 | 0 | 1 | 0 |
| (93B) | 1.0 | 0 | 0 | 0 | 2 | 5 | 2 | 5 | 5 | 2 | 3 | 0 |
| (94B) | 1.0 | 0 | 0 | 0 | 0 | 0 | 1 | 3 | 1 | 1 | 1 | 0 |
| (95B) | 0.5 | 5 | 5 | 5 | 5 | 5 | 5 | 5 | 5 | 5 | 5 | 3 |
| | 0.125 | 4 | 3 | 5 | 5 | 5 | 5 | 5 | 5 | 5 | 5 | 0 |
| | 0.031 | 2 | 1 | 1 | 3 | 5 | 3 | 5 | 5 | 5 | 4 | 0 |
| (96B) | 0.062 | 5 | 3 | 5 | 4 | 5 | 5 | 5 | 5 | 5 | 4 | 1 |
| | 0.016 | 3 | 3 | 4 | 1 | 3 | 4 | 5 | 5 | 5 | 3 | 1 |
| (97B) | 0.062 | 5 | 3 | 2 | 2 | 5 | 5 | 5 | 5 | 5 | 4 | 1 |
| | 0.016 | 3 | 2 | 1 | 1 | 5 | 3 | 5 | 5 | 3 | 3 | 1 |
| (98B) | 0.5 | 5 | 4 | 5 | 5 | 5 | 5 | 5 | 5 | 5 | 4 | 4 |
| (99B) | 0.5 | 5 | 5 | 5 | 5 | 5 | 5 | 5 | 5 | 5 | 5 | 5 |
| (100B) | 0.5 | 3 | 1 | 1 | 5 | 5 | 5 | 5 | 5 | 5 | 3 | 1 |
| (101B) | 0.125 | 4 | 2 | 4 | 5 | 5 | 4 | 5 | 5 | 5 | 5 | 2 |
| | 0.031 | 2 | 2 | 2 | 5 | 5 | 5 | 5 | 5 | 1 | 4 | 1 |
| (102B) | 0.5 | 5 | 4 | 5 | 5 | 5 | 5 | 5 | 5 | 5 | 5 | 5 |
| | 0.125 | 4 | 3 | 3 | 5 | 4 | 5 | 5 | 5 | 5 | 5 | 0 |
| (103B) | 0.5 | 5 | 4 | 5 | 5 | 5 | 5 | 5 | 5 | 5 | 5 | 5 |
| | 0.125 | 5 | 3 | 3 | 5 | 3 | 4 | 5 | 5 | 5 | 4 | 1 |
| | 0.031 | 1 | 0 | 1 | 4 | 0 | 5 | 5 | 5 | 3 | 4 | 0 |
| (104B) | 0.5 | 5 | 5 | 5 | 5 | 5 | 5 | 5 | 5 | 5 | 5 | 5 |
| | 0.125 | 5 | 3 | 5 | 5 | 5 | 5 | 5 | 5 | 5 | 5 | 3 |
| | 0.031 | 4 | 1 | 1 | 5 | 1 | 5 | 5 | 5 | 3 | 4 | 1 |

Table 1C (continued)

| Compound No. | Rate of Application (kg/ha) | Plants a | b | c | d | e | f | g | h | i | j | k |
|---|---|---|---|---|---|---|---|---|---|---|---|---|
| (105B) | 0.5 | 5 | 4 | 5 | 5 | 5 | 5 | 5 | 5 | 5 | 5 | 5 |
| | 0.125 | 5 | 4 | 4 | 5 | 5 | 5 | 5 | 5 | 5 | 5 | 3 |
| | 0.031 | 1 | 0 | 2 | 5 | 3 | 4 | 5 | 5 | 3 | 4 | 1 |
| (106B) | 0.125 | 3 | 2 | 1 | 5 | 1 | 5 | 5 | 5 | 5 | 5 | 1 |
| | 0.031 | 2 | 1 | 1 | 5 | 1 | 4 | 5 | 5 | 5 | 3 | 1 |
| (107B) | 0.125 | 4 | 2 | 3 | 5 | 5 | 5 | 5 | 5 | 5 | 4 | 1 |
| | 0.031 | 2 | 1 | 2 | 3 | 0 | 4 | 5 | 5 | 3 | 3 | 1 |
| (108B) | 0.125 | 2 | 1 | 1 | 2 | 0 | 1 | 5 | 2 | 0 | 1 | 0 |
| (109B) | 0.125 | 5 | 3 | 5 | 5 | 5 | 5 | 5 | 5 | 5 | 5 | 1 |
| (110B) | 0.125 | 5 | 4 | 5 | 5 | 5 | 5 | 5 | 5 | 5 | 4 | 3 |
| | 0.031 | 4 | 3 | 3 | 4 | 5 | 5 | 5 | 5 | 5 | 4 | 3 |
| (111B) | 0.125 | 2 | 2 | 2 | 5 | 5 | 4 | 5 | 5 | 5 | 5 | 1 |
| | 0.031 | 1 | 0 | 1 | 5 | 1 | 5 | 5 | 5 | 1 | 1 | 0 |
| (112B) | 0.125 | 5 | 2 | 5 | 5 | 5 | 5 | 5 | 5 | 5 | 5 | 5 |
| | 0.031 | 2 | 2 | 1 | 5 | 2 | 4 | 5 | 5 | 2 | 3 | 2 |
| (113B) | 0.125 | 4 | 4 | 5 | 2 | 1 | 3 | 5 | 4 | 5 | 4 | 1 |
| | 0.031 | 3 | 3 | 1 | 3 | 1 | 2 | 5 | 3 | 2 | 3 | 1 |
| (114B) | 0.125 | 1 | 0 | 0 | 3 | 1 | 3 | 5 | 5 | 3 | 3 | 1 |
| (115B) | 0.125 | 3 | 4 | 5 | 5 | 5 | 5 | 5 | 5 | 5 | 4 | 5 |
| | 0.031 | 2 | 0 | 2 | 5 | 3 | 5 | 5 | 5 | 5 | 2 | 1 |
| (116B) | 0.125 | 2 | 2 | 5 | 5 | 3 | 3 | 5 | 5 | 5 | 4 | 5 |
| (117B) | 0.5 | 1 | 0 | 0 | 5 | 1 | 5 | 5 | 5 | 5 | 4 | 1 |
| | 0.031 | 0 | 0 | 0 | 2 | 0 | 2 | 3 | 2 | 0 | 0 | 0 |
| (118B) | 0.5 | 5 | 3 | 5 | 5 | 5 | 5 | 5 | 5 | 5 | 5 | 1 |
| | 0.125 | 3 | 1 | 4 | 5 | 5 | 5 | 5 | 5 | 5 | 4 | 0 |
| | 0.062 | 1 | 1 | 4 | 2 | 5 | 4 | 5 | 5 | 4 | 2 | 1 |
| (119B) | 0.5 | 5 | 3 | 5 | 5 | 5 | 5 | 5 | 5 | 5 | 5 | 1 |
| | 0.125 | 5 | 3 | 5 | 5 | 5 | 5 | 5 | 5 | 5 | 4 | 0 |
| (120B) | 0.5 | 5 | 3 | 3 | 5 | 5 | 5 | 5 | 5 | 5 | 4 | 2 |
| | 0.125 | 4 | 1 | 2 | 5 | 5 | 5 | 5 | 5 | 5 | 5 | 1 |

Table 1C (continued)

| Compound No. | Rate of Application (kg/ha) | Plants | | | | | | | | | | |
|---|---|---|---|---|---|---|---|---|---|---|---|---|
| | | a | b | c | d | e | f | g | h | i | j | k |
| (121B) | 0.5 | 5 | 3 | 5 | 5 | 5 | 5 | 5 | 5 | 5 | 5 | 0 |
| | 0.125 | 5 | 2 | 5 | 5 | 5 | 5 | 5 | 5 | 5 | 5 | 0 |
| (122B) | 0.5 | 5 | 4 | 5 | 5 | 5 | 5 | 5 | 5 | 5 | 5 | 3 |
| | 0.125 | 5 | 3 | 5 | 5 | 5 | 5 | 5 | 5 | 5 | 5 | 2 |
| (123B) | 0.5 | 5 | 4 | 5 | 5 | 5 | 5 | 5 | 5 | 5 | 5 | 2 |
| | 0.125 | 5 | 2 | 2 | 5 | 5 | 3 | 5 | 5 | 5 | 5 | 1 |
| (124B) | 0.062 | 4 | 5 | 4 | 5 | 5 | 4 | 5 | 5 | 5 | 3 | 1 |
| (125B) | 0.5 | 5 | 3 | 5 | 5 | 5 | 5 | 5 | 5 | 5 | 5 | 3 |
| | 0.125 | 4 | 1 | 5 | 5 | 5 | 5 | 5 | 5 | 5 | 4 | 0 |
| (126B) | 0.5 | 5 | 5 | 5 | 5 | 5 | 5 | 5 | 5 | 5 | 5 | 4 |
| | 0.125 | 4 | 3 | 3 | 4 | 3 | 5 | 5 | 5 | 5 | 5 | 3 |
| | 0.031 | 3 | 1 | 2 | 5 | 2 | 5 | 5 | 5 | 5 | 3 | 1 |
| (127B) | 0.5 | 5 | 4 | 5 | 5 | 5 | 5 | 5 | 5 | 5 | 5 | 3 |
| | 0.125 | 5 | 4 | 4 | 5 | 5 | 5 | 5 | 5 | 5 | 5 | 2 |
| | 0.031 | 1 | 0 | 1 | 5 | 1 | 4 | 5 | 5 | 2 | 4 | 1 |
| (128B) | 0.125 | 5- | 1 | 2 | 5 | 1 | 4 | 5 | 5 | 4 | 5 | 1 |
| | 0.031 | 3 | 1 | 1 | 3 | 0 | 3 | 5 | 5 | 1 | 4 | 1 |
| (129B) | 0.125 | 5 | 2 | 3 | 5 | 5 | 5 | 5 | 5 | 5 | 4 | 1 |
| | 0.031 | 3 | 0 | 1 | 5 | 0 | 4 | 5 | 5 | 5 | 4 | 1 |
| (130B) | 0.125 | 4 | 2 | 4 | 5 | 5 | 5 | 5 | 5 | 5 | 5 | 1 |
| (131B) | 0.125 | 3 | 2 | 5 | 5 | 5 | 5 | 5 | 5 | 5 | 5 | 5 |
| | 0.031 | 3 | 0 | 1 | 3 | 1 | 3 | 5 | 5 | 5 | 3 | 0 |
| (132B) | 0.125 | 3 | 4 | 3 | 3 | 0 | 1 | 5 | 3 | 3 | 3 | 2 |
| (133B) | 0.5 | 0 | 0 | 2 | 5 | 2 | 5 | 5 | 5 | 5 | 1 | 0 |
| | 0.031 | 0 | 0 | 0 | 0 | 0 | 1 | 2 | 3 | 0 | 0 | 0 |
| (134B) | 0.5 | 5 | 4 | 5 | 5 | 5 | 5 | 5 | 5 | 5 | 5 | 4 |
| | 0.125 | 5 | 2 | 2 | 5 | 5 | 5 | 5 | 5 | 5 | 5 | 2 |
| (135B) | 0.5 | 5 | 5 | 5 | 5 | 5 | 5 | 5 | 5 | 5 | 5 | 5 |
| | 0.125 | 4 | 3 | 3 | 5 | 5 | 5 | 5 | 5 | 5 | 5 | 5 |
| | 0.031 | 2 | 2 | 2 | 5 | 5 | 5 | 5 | 5 | 5 | 4 | 0 |

Table 1C (continued)

| Compound No. | Rate of Application (kg/ha) | Plants | | | | | | | | | | |
|---|---|---|---|---|---|---|---|---|---|---|---|---|
| | | a | b | c | d | e | f | g | h | i | j | k |
| (136B) | 0.125 | 5 | 3 | 3 | 5 | 5 | 5 | 5 | 5 | 5 | 5 | 2 |
| | 0.031 | 1 | 2 | 3 | 5 | 1 | 5 | 5 | 5 | 5 | 4 | 2 |
| (137B) | 0.125 | 5 | 3 | 4 | 5 | 5 | 5 | 5 | 5 | 5 | 5 | 5 |
| | 0.031 | 3 | 2 | 3 | 5 | 1 | 4 | 5 | 5 | 5 | 4 | 2 |
| (138B) | 0.125 | 4 | 2 | 4 | 5 | 5 | 5 | 5 | 5 | 5 | 5 | 2 |
| (139B) | 0.5 | 5 | 4 | 5 | 5 | 5 | 5 | 5 | 5 | 5 | 5 | 5 |
| | 0.125 | 5 | 3 | 3 | 5 | 5 | 5 | 5 | 5 | 5 | 5 | 1 |
| (140B) | 0.125 | 5 | 4 | 5 | 5 | 5 | 5 | 5 | 5 | 5 | 5 | 4 |
| | 0.031 | 4 | 3 | 4 | 5 | 5 | 5 | 5 | 5 | 5 | 4 | 3 |
| (141B) | 0.125 | 5 | 4 | 4 | 5 | 5 | 5 | 5 | 5 | 5 | 4 | 3 |
| | 0.031 | 3 | 2 | 3 | 4 | 3 | 5 | 5 | 5 | 5 | 4 | 3 |
| (142B) | 0.125 | 5 | 4 | 5 | 5 | 5 | 5 | 5 | 5 | 5 | 5 | 1 |
| | 0.031 | 4 | 3 | 3 | 5 | 2 | 4 | 5 | 5 | 5 | 3 | 0 |
| (143B) | 0.5 | 5 | 5 | 5 | 5 | 5 | 5 | 5 | 5 | 5 | 5 | 5 |
| | 0.125 | 5 | 2 | 3 | 5 | 5 | 5 | 5 | 5 | 5 | 5 | 4 |
| (147B) | 0.125 | 2- | 1 | 2 | 3 | 1 | 4 | 5 | 5 | 5 | 4 | 1 |
| | 0.031 | 1 | 1 | 1 | 2 | 1 | 3 | 5 | 5 | 5 | 2 | 0 |
| (148B) | 0.125 | 3 | 2 | 3 | 5 | 5 | 4 | 5 | 5 | 5 | 4 | 4 |
| | 0.031 | 1 | 0 | 0 | 5 | 1 | 4 | 5 | 5 | 5 | 4 | 0 |
| (149B) | 0.5 | 2 | 2 | 2 | 5 | 5 | 5 | 5 | 5 | 5 | 4 | 0 |
| | 0.125 | 1 | 1 | 1 | 5 | 1 | 3 | 5 | 5 | 1 | 3 | 0 |
| | 0.031 | 1 | 0 | 0 | 5 | 1 | 2 | 4 | 4 | 0 | 0 | 0 |
| (150B) | 0.125 | 1 | 0 | 1 | 5 | 3 | 3 | 4 | 5 | 5 | 2 | 1 |
| (151B) | 0.125 | 2 | 1 | 1 | 4 | 1 | 4 | 5 | 5 | 1 | 3 | 0 |
| | 0.031 | 1 | 1 | 1 | 2 | 1 | 3 | 5 | 5 | 1 | 0 | 0 |
| (152B) | 0.125 | 1 | 1 | 1 | 3 | 1 | 3 | 5 | 5 | 1 | 2 | 0 |
| | 0.031 | 1 | 0 | 1 | 3 | 1 | 3 | 5 | 3 | 1 | 1 | 0 |
| (153B) | 0.125 | 2 | 1 | 2 | 2 | 1 | 4 | 5 | 5 | 5 | 2 | 0 |
| | 0.031 | 1 | 0 | 0 | 3 | 1 | 2 | 5 | 4 | 1 | 1 | 0 |

Table 2C

| Compound No. | Rate of application (kg/ha) | Plants | | | | | | | | | |
|---|---|---|---|---|---|---|---|---|---|---|---|
| | | a | b | c | e | f | g | h | i | j | k |
| (2B) | 2.0 | 4 | 3 | 2 | 5 | 5 | 4 | 5 | 4 | 0 | 0 |
| | 0.5 | 3 | 1 | 2 | 5 | 4 | 2 | 5 | 1 | 0 | 0 |
| (11B) | 2.0 | 5 | 5 | 5 | 5 | 5 | 5 | 5 | 5 | 0 | 0 |
| | 0.5 | 4 | 3 | 3 | 5 | 5 | 2 | 5 | 1 | 0 | 0 |
| (20B) | 2.0 | 5 | 5 | 5 | 4 | 5 | 5 | 5 | 5 | 0 | 0 |
| | 0.5 | 4 | 3 | 3 | 5 | 5 | 4 | 5 | 2 | 0 | 0 |
| (21B) | 2.0 | 5 | 5 | 5 | 5 | 5 | 5 | 5 | 5 | 0 | 0 |
| | 0.5 | 5 | 5 | 5 | 5 | 5 | 5 | 5 | 4 | 0 | 0 |
| (26B) | 2.0 | 5 | 5 | 5 | 5 | 5 | 5 | 5 | 5 | 0 | 0 |
| | 0.5 | 5 | 5 | 5 | 5 | 5 | 5 | 5 | 4 | 0 | 0 |

TEST EXAMPLES 3C - 6C AND TEST COMPARATIVE EXAMPLES 1C - 2C

In each run, each of the oxime derivatives shown in Table 3C alone or with each of the N-phosphonomethylglycine derivatives shown in Table 3C (in the indicated mixing ratios) was dissolved in 16 parts by volume of a mixture of water and acetone (1 : 1 by volume; containing 0.05 % of a nonionic surfactant, Sorpol-2680) to prepare a spray solution. The amounts of the herbicidal compounds were such that the rates of application were as shown in Table 3C.

The plants tested were grown in vinyl resin pots (diameter 10 cm) filled with soil in a green house for 2 to 3 weeks after germination from seeds or tubers.

The spray solution was applied to the plants so that the total volume sprayed became 4 cc/100 $cm^2$, and the herbicidal activity was examined.

The results are shown in Table 3C.

The alphabetical symbols in the Table represent the following plants, respectively:

l: Ipomoea hederacea
m: Cossia obtusifolia
n: Sida spinosa
o: Ambrosia artemisiaefolia var. elator
p: Datula stramonium
q: Avena fatua
r: Agropyron repens
s: Amaranthus lividus
t: Panicum texanum

Table 3C

| | Compound No. | Rate of Application (kg/ha) | Days After Treatment | Plants l | m | n | o | p | q | r | s | t |
|---|---|---|---|---|---|---|---|---|---|---|---|---|
| Test comparative example 1C | (117B) | 0.5 | 3 | 0 | 0 | 0 | 0 | 0 | 0 | 0 | 0 | 0 |
| | | | 7 | 0 | 0 | 0 | 0 | 0 | 1 | 2 | 3 | 2 |
| | | | 14 | 1 | 1 | 1 | 0 | 0 | 2 | 3 | 3 | 4 |
| Test comparative example 2C | (117B) | 0.375 | 3 | 0 | 0 | 0 | 0 | 0 | 0 | 0 | 0 | 0 |
| | | | 7 | 0 | 0 | 0 | 0 | 0 | 1 | 1 | 2 | 1 |
| | | | 14 | 1 | 1 | 0 | 0 | 0 | 1 | 2 | 2 | 3 |
| Test example 3C | (117B) | 0.375 | 3 | 4 | 2 | 4 | 4 | 4 | 2 | 1 | 3 | 2 |
| | (11B) | 0.125 | 7 | 4 | 3 | 4 | 5 | 4 | 3 | 3 | 4 | 3 |
| | | | 14 | 4 | 4 | 5 | 5 | 5 | 3 | 4 | 5 | 4 |
| Test example 4C | (11B) | 0.125 | 3 | 3 | 1 | 3 | 3 | 3 | 1 | 1 | 2 | 1 |
| | | | 7 | 3 | 2 | 4 | 4 | 4 | 1 | 1 | 3 | 1 |
| | | | 14 | 4 | 2 | 5 | 5 | 5 | 1 | 1 | 3 | 1 |
| Test example 5C | (117B) | 0.375 | 3 | 5 | 4 | 4 | 5 | 4 | 3 | 3 | 4 | 4 |
| | (20B) | 0.125 | 7 | 5 | 5 | 5 | 5 | 5 | 5 | 4 | 5 | 5 |
| | | | 14 | 5 | 5 | 5 | 5 | 5 | 5 | 5 | 5 | 5 |

Table 3C (continued)

| | Compound No. | Rate of Application (kg/ha) | Days After Treatment | Plants l | m | n | o | p | q | r | s | t |
|---|---|---|---|---|---|---|---|---|---|---|---|---|
| Test example 6C | (20B) | 0.125 | 3 | 4 | 2 | 3 | 4 | 4 | 2 | 3 | 3 | 3 |
| | | | 7 | 5 | 5 | 4 | 5 | 5 | 3 | 3 | 5 | 4 |
| | | | 14 | 5 | 5 | 5 | 5 | 5 | 4 | 3 | 5 | 4 |

TEST EXAMPLES 7C - 14C AND TEST COMPARATIVE EXAMPLES 3C - 4C

In each run, each of the oxime derivative shown in Table 4C alone or with each of the N-phosphonomethylglycine derivatives shown in Table 4C (in the indicated mixing ratios) was dissolved in 16 parts by volume of a mixture of water and acetone (1 : 1 by volume; containing 0.05 % of a nonionic surfactant, Sorpol-2680) to prepare a spray solution. The amounts of the herbicidal compounds were such that the rates of application were as shown in Table 4C.

The plants tested were grown in vinyl resin pots (diameter 10 cm) filled with soil in a green house for 2 to 3 weeks after germination from seeds or tubers.

The spray solution was applied to the plants so that the total volume sprayed became 4 cc/100 $cm^2$, and the herbicidal activity was examined.

The results are shown in Table 4C.

When the results of Test Examples 7C to 14C and Test Comparative Examples 3C to 4C are compared, it is found that the herbicidal compositions of this invention comprising the two types of herbicidal compounds indicated above exhibit herbicidal activity earlier and thus have better quick-acting efficacy than the N-phosphonomethylglycine derivatives alone.

In contrast, the compositions of this invention, for example that used in Test Example 13C, could kill all of the weeds shown in Table 4C within about 1 week. By applying a combination of the oxime derivative and the N-phosphonomethylglycine derivative, the herbicidal composition of this invention surprisingly showed increased quick-acting efficacy and a broadened herbicidal spectrum at low application rate as the synergistic effect of the combined use.

Table 4C

| | Compound No. | Rate of Application (kg/ha) | Days After Treatment | Plants a | b | c | d | e | f | g | h | i |
|---|---|---|---|---|---|---|---|---|---|---|---|---|
| Test comparative example 3C | (117) | 0.5 | 3 | 0 | 0 | 0 | 0 | 0 | 0 | 0 | 0 | 0 |
| | | | 7 | 1 | 0 | 1 | 1 | 0 | 1 | 0 | 0 | 1 |
| | | | 14 | 4 | 4 | 4 | 4 | 2 | 4 | 1 | 1 | 4 |
| Test comparative example 4C | (117) | 0.375 | 3 | 0 | 0 | 0 | 0 | 0 | 0 | 0 | 0 | 0 |
| | | | 7 | 1 | 0 | 1 | 0 | 0 | 1 | 0 | 0 | 1 |
| | | | 14 | 3 | 4 | 4 | 0 | 2 | 4 | 1 | 1 | 4 |
| Test example 7C | (117) | 0.375 | 3 | 4 | 2 | 4 | 4 | 3 | 3 | 4 | 4 | 4 |
| | | | 7 | 5 | 4 | 5 | 5 | 4 | 5 | 5 | 5 | 5 |
| | (101B) | 0.125 | 14 | 5 | 4 | 5 | 5 | 5 | 5 | 5 | 5 | 5 |
| Test example 8C | (101B) | 0.125 | 3 | 3 | 2 | 4 | 4 | 3 | 2 | 4 | 4 | 4 |
| | | | 7 | 4 | 3 | 4 | 5 | 3 | 3 | 5 | 5 | 5 |
| | | | 14 | 4 | 3 | 4 | 5 | 5 | 4 | 5 | 5 | 5 |
| Test example 9C | (117) | 0.375 | 3 | 4 | 2 | 4 | 4 | 2 | 3 | 4 | 4 | 4 |
| | | | 7 | 4 | 3 | 4 | 5 | 2 | 3 | 5 | 5 | 5 |
| | (127B) | 0.125 | 14 | 5 | 4 | 5 | 5 | 5 | 5 | 5 | 5 | 5 |

Table 4C (continued)

| | Compound No. | Rate of Application (kg/ha) | Days After Treatment | Plants a | b | c | d | e | f | g | h | i |
|---|---|---|---|---|---|---|---|---|---|---|---|---|
| Test example 10C | | | 3 | 3 | 2 | 3 | 4 | 1 | 3 | 4 | 4 | 4 |
| | (127B) | 0.125 | 7 | 4 | 3 | 4 | 5 | 1 | 4 | 5 | 5 | 5 |
| | | | 14 | 4 | 3 | 4 | 5 | 4 | 4 | 5 | 5 | 5 |
| Test example 11C | (117) | 0.375 | 3 | 4 | 3 | 4 | 4 | 1 | 2 | 4 | 4 | 4 |
| | | | 7 | 4 | 3 | 5 | 5 | 1 | 2 | 5 | 5 | 5 |
| | (129B) | 0.125 | 14 | 5 | 4 | 5 | 5 | 5 | 4 | 5 | 5 | 5 |
| Test example 12C | | | 3 | 4 | 2 | 3 | 4 | 4 | 2 | 4 | 4 | 4 |
| | (129B) | 0.125 | 7 | 4 | 3 | 4 | 5 | 5 | 2 | 5 | 5 | 5 |
| | | | 14 | 4 | 3 | 4 | 5 | 5 | 4 | 5 | 5 | 5 |
| Test example 13C | (117) | 0.375 | 3 | 4 | 4 | 4 | 4 | 4 | 4 | 4 | 4 | 4 |
| | | | 7 | 4 | 5 | 4 | 5 | 4 | 5 | 5 | 5 | 5 |
| | (26B) | 0.125 | 14 | 5 | 5 | 5 | 5 | 5 | 5 | 5 | 5 | 5 |
| Test example 14C | | | 3 | 4 | 3 | 3 | 4 | 4 | 3 | 3 | 4 | 4 |
| | (26B) | 0.125 | 7 | 4 | 4 | 4 | 5 | 4 | 4 | 5 | 5 | 5 |
| | | | 14 | 4 | 4 | 5 | 5 | 5 | 5 | 5 | 5 | 5 |

TEST EXAMPLES 15C - 16C AND TEST COMPARATIVE EXAMPLES 5C -6C

In each run, each of the oxime derivative shown in Table 5C and atrazine (in the indicated mixing ratios) was dissolved in 16 parts by volume of a mixture of water and acetone (1 : 1 by volume; containing 0.05 % of a nonionic surfactant, Sorpol-2680) to prepare a spray solution. The amounts of the herbicidal compounds were such that the rates of application were as shown in Table 5C.

The plants tested were grown in vinyl resin pots (diameter 10 cm) filled with soil in a green house for 2

to 3 weeks after germination from seeds or tubers.

The spray solution was applied to the plants so that the total volume sprayed became 4 cc/100 $cm^2$, and the herbicidal activity was examined.

The results 3 weeks after treatment are shown in Table 5C.

Table 5C

| | Compound No. | Rate of Application (kg/ha) | Plants | | | | | | | | |
|---|---|---|---|---|---|---|---|---|---|---|---|
| | | | a | b | c | d | e | f | g | h | i |
| Test comparative example 5C | Atrazine | 0.5 | 1 | 0 | 0 | 1 | 0 | 5 | 5 | 5 | 1 |
| Test comparative example 6C | Atrazine | 0.25 | 0 | 0 | 0 | 0 | 0 | 1 | 0 | 5 | 0 |
| Test example 15C | Atrazine (26B) | 0.5 0.125 | 5 | 5 | 5 | 5 | 5 | 5 | 5 | 5 | 5 |
| Test example 16C | Atrazine (26B) | 0.25 0.125 | 5 | 5 | 5 | 5 | 5 | 5 | 5 | 5 | 5 |

TEST EXAMPLES 17C - 18C AND TEST COMPARATIVE EXAMPLES 7C -8C

In each run, the compound (26B) and compound (500) were dissolved in 16 parts by volume of a mixture of water and acetone (1 : 1 by volume; containing 0.05 % of a nonionic surfactant, Sorpol-2680) so that the mixing ratios and the application rates became prescribed values, respectively to prepare a spray solution.

The plants tested were grown in vinyl resin pots (diameter 10 cm) filled with soil in a green house for 2 to 3 weeks after germination from seeds or tubers.

The spray solution was applied to the plants so that the total volume sprayed became 4 cc/100 $cm^2$, and the herbicidal activity was examined.

The results are shown in Table 6C.

Table 6C

| | Compound No. | Rate of Application (kg/ha) | Days After Treatment | Plants a | b | c | d | e | f | g | h | i |
|---|---|---|---|---|---|---|---|---|---|---|---|---|
| Test comparative example 7C | (500) | 0.25 | 3 | 1 | 1 | 1 | 1 | 0 | 1 | 0 | 0 | 0 |
| | | | 7 | 3 | 3 | 2 | 3 | 1 | 4 | 2 | 2 | 2 |
| | | | 14 | 4 | 4 | 2 | 3 | 1 | 5 | 2 | 4 | 4 |
| Test comparative example 8C | (500) | 0.125 | 3 | 1 | 1 | 1 | 1 | 0 | 1 | 0 | 0 | 0 |
| | | | 7 | 3 | 3 | 2 | 1 | 1 | 4 | 2 | 2 | 2 |
| | | | 14 | 4 | 4 | 2 | 1 | 1 | 4 | 2 | 3 | 3 |
| Test example 17C | (500) | 0.25 | 3 | 4 | 2 | 4 | 4 | 3 | 3 | 4 | 4 | 4 |
| | | | 7 | 5 | 4 | 5 | 5 | 4 | 5 | 5 | 5 | 5 |
| | (26B) | 0.125 | 14 | 5 | 5 | 5 | 5 | 5 | 5 | 5 | 5 | 5 |
| Test example 18C | (500) | 0.125 | 3 | 4 | 3 | 4 | 4 | 3 | 3 | 4 | 4 | 4 |
| | | | 7 | 4 | 3 | 5 | 5 | 4 | 5 | 5 | 4 | 5 |
| | (26B) | 0.125 | 14 | 5 | 4 | 5 | 5 | 5 | 5 | 5 | 5 | 5 |

When the results of Test Examples 3C to 14C and Test Comparative Examples 1C to 4C are compared, the herbicidal compositions of this invention exhibited herbicidal activity earlier and thus have better quick-acting efficacy than the N-phosphonomethylglycine derivatives alone.

The N-phosphonomethylglycine derivatives, used alone, particularly in low application rates, have lowered herbicidal activity against broad-leaved weeds including Ipomoea hederacea, Sida spinosa and other weeds and 14 days after the treatment showed hardly any activity or showed only insufficient activity.

In contrast, the compositions of this invention, for example, the composition in Test Example 13C could kill all of the weeds shown in Table 4C within about 1 week. By applying a combination of the oxime

derivative and N-phosphonomethylglycine derivative, the herbicidal composition of this invention surprisingly showed increased quick-acting efficacy and a broadened herbicidal spectrum at low application rate as the synergistic effect of the combined use.

When Compound (500) was used alone, the herbicidal activity of the compound was low against broad-leaved weeds such as Chenopodium album var. centrorubrum, Amaranthus viridis or Abutilon theophrasti in low application rates, as shown in Table 6C. On the contrary, the herbicidal composition of this invention overcomes these defects and has sufficient herbicidal activity.

**Claims**

1. Oxime derivatives of formula (I)

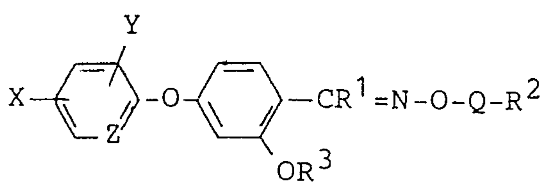

(I)

wherein
X and Y are identical or different and each represents a hydrogen atom, a halogen atom, $-CF_3$, or an alkyl group having 1 to 5 carbon atoms;
Z is =CH- or =N-;
$R^1$ represents a hydrogen atom, a hydroxyl group, an alkyl group having 1 to 5 carbon atoms, or an alkoxy group having 1 to 5 carbon atoms;
$R^2$ represents a group selected from the following groups shown in a) to g):
a) hydrogen atom:
b) a saturated or unsaturated aliphatic hydrocarbon group having 1 to 10 carbon atoms which may be optionally substituted by the following substituents:
substituents:
i) a halogen atom;
ii) a hydroxyl group;
iii) an alkoxy group having 1 to 5 carbon atoms;
iv) $-COR^4$;
wherein $R^4$ is a hydroxyl group, an alkyl group having 1 to 5 carbon atoms which may be substituted by a halogen atom, a phenyl group which may be substituted by a halogen atom, an alkoxy group having 1 to 5 carbon atoms, a alkenyloxy group having 1 to 5 carbon atoms or a group of the formula

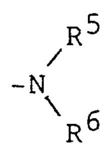

in which $R^5$ and $R^6$ are identical or different and each represents a hydrogen atom or an alkyl group having 1 to 5 carbon atoms;
v) a phenyl group;
wherein the phenyl group may be substituted by a halogen atom, a hydroxyl group, $-CF_3$, $-NO_2$, -CN, an alkyl group having 1 to 5 carbon atoms, an alkoxy group having 1 to 5 carbon atoms, $-COR_4$ or

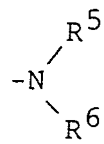

;

vi) a phenoxy group;

wherein the phenoxy group may be substituted by the groups cited in v), in addition to phenyl, phenoxy or pyridyloxy group (wherein the phenyl, phenoxy or pyridyloxy group may be substituted by halogen atom or $-CF_3$) vii)

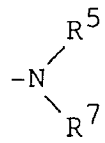

wherein $R^7$ is a hydrogen atom, an alkyl group having 1 to 5 carbon atoms or $-COR^4$;

viii) -CN;

c) an alkoxy group which may be substituted by the following substituents:

substituents:

i) a halogen atom;

ii) a phenyl group (wherein the phenyl group may be substituted by substituents v) in b);

d) a phenoxy group which may be substituted by the following substituents:

substituents: the same substituents as v) in b);

e) an aromatic hydrocarbon group having 6 to 20 carbon atoms which may be substituted by the following substituents:

substituents:

i) a halogen atom;

ii) a hydroxyl group;

iii) $-CF_3$;

iv) $-NO_2$;

v) -CN;

vi) an alkyl group having 1 to 5 carbon atoms;

vii) an alkoxy group having 1 to 5 carbon atoms;

viii)$-COR^4$;

ix)

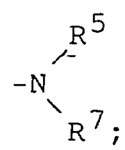

x) $-N^+R^5R^6R^8$

wherein $R^8$ is an alkyl group having 1 to 5 carbon atoms;

xi) a phenyl group

wherein the phenyl group may be substituted by the substituents v) in b);

xii) a phenoxy group

wherein the phenoxy group may be substituted by the substituents vi) in b);

xiii)$-CH_2COR^4$;

f) an aromatic heterocyclic group containing at least one nitrogen atom having 3 to 20 carbon atoms which may be substituted by the following substituents:

substituents: the same substituents as shown in e);

g)

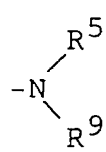

wherein $R^9$ is b), e) or f);

$R^3$ is a hydrogen atom ; or an aliphatic hydrocarbon group having 1 to 10 carbon atoms which may be substituted by a halogen atom, a hydroxyl group, an alkoxy group, $-COR^4$ or

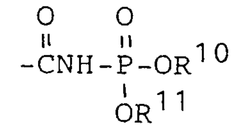

wherein $R^{10}$ and $R^{11}$ are identical or different and represent a hydrogen atom, an alkyl group having 1 to 5 carbon atoms, or a phenyl group;
Q represents a direct bond,

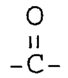, 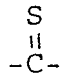 or 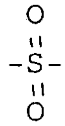;

wherein in case that Q is a direct bond, $R^2$ is not c), d) or g);
and their salts.
2. Benzaldehyde derivatives of formula (II)

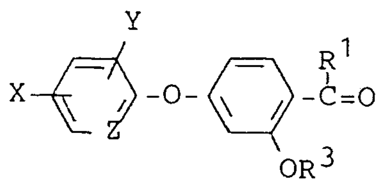

(II)

wherein
X, Y, Z, $R^1$ and $R^3$ have the same meanings as in the formula (I);
herein
when Z is =CH-, $R^1$ is not an alkyl group having 1 to 5 carbon atoms, and $R^3$ is not an aliphatic hydrocarbon group having 1 to 10 carbon atoms which is substistuted by $-COR^4$;
and their salts.
3. A herbicidal composition comprising a herbicidally effective amount of compound of claim 1 and at least one component selected from the group composed of additives, carriers, pesticides and other herbicidal agents.
4. A herbicidal composition comprising a herbicidally effective amount of a compound of claim 1 as an active ingredient, and a carrier and/or a surfactant.
5. A process for producing a herbicidal composition characterized in that a compound of claim 1 is mixed with at least one component selected from the group composed of additives, carriers, pesticides and other herbicidal agents.
6. A herbicidal composition comprising as a herbicidal ingredient a combination of herbicidally effective amounts of a compound of claim 1 and an N-phosphonomethylglycine derivative of formula (III)

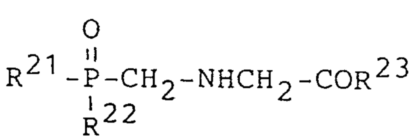

(III)

wherein
$R^{21}$ and $R^{22}$ are identical or different, and each represents a hydroxyl group or $-OR^{24}$
$R^{23}$ is a hydroxyl group, $-OR^{24}$ or

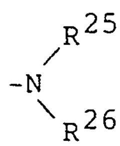;

wherein
$R^{24}$ represents an alkyl group having 1 to 5 carbon atoms, a cyclohexyl group, a haloalkyl group having 1 to 5 carbon atoms, an alkenyl group having 2 to 5 carbon atoms or an alkoxyalkyl, haloalkoxyalkyl or alkoxyalkoxyalkyl group (herein each of the alkoxy, haloalkoxy and alkyl groups has 1 to 5 carbon atoms) and a phenoxy group;
$R^{25}$ and $R^{26}$ are identical or different and each represents a hydrogen atom, an alkyl group having 1 to 5 carbon atoms, a hydroxyalkyl group having 1 to 5 carbon atoms, an alkenyl group having 2 to 5 carbon atoms, or $R^{25}$ and $R^{26}$, together with the nitrogen atom to which they are attached, can form a morpholino group, a piperidino group or a pyrrolidino group,
and/or a glufosinate compound of formula (IV)

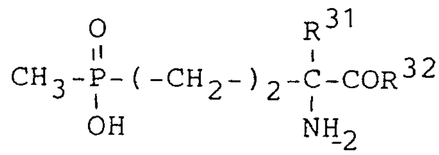
(IV)

wherein
$R^{31}$ represents a hydrogen atom or an alkyl group having 1 to 4 carbon atoms,
$R^{32}$ represents -OH, $-NH_2$, $-NHNH_2$, $-NHC_6H_5$ or an alkoxy group having 1 to 12 carbon atoms which may be substituted by -OH,
or its acid addition salt or a salt with a base, and a carrier and/or a surfactant.
7. Process for preparing oxime derivatives represented by represented by the following formula (I)

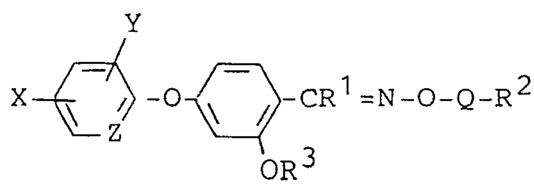
(I)

wherein
X and Y are identical or different and each represents a hydrogen atom, a halogen atom, $-CF_3$, or an alkyl group having 1 to 5 carbon atoms;
Z is =CH- or =N-,
$R^1$ represents a hydrogen atom, a hydroxyl group, an alkyl group having 1 to 5 carbon atoms, or an alkoxy group having 1 to 5 carbon atoms;
$R^2$ represents a group selected from the following groups shown in a) to g):
a) hydrogen atom:
b) a saturated or unsaturated aliphatic hydrocarbon group having 1 to 10 carbon atoms which may be optionally substituted by the following substituents: substituents:
i) a halogen atom;
ii) a hydroxyl group;
iii) an alkoxy group having 1 to 5 carbon atoms;
iv) $-COR^4$;
wherein $R^4$ is a hydroxyl group, an alkyl group having 1 to 5 carbon atoms which may be substituted by a halogen atom, a phenyl group which may be substituted by a halogen atom, an alkoxy group having 1 to 5 carbon atoms, a alkenyloxy group having 1 to 5 carbon atoms or a group of the formula

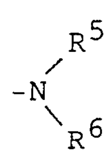

in which $R^5$ and $R^6$ are identical or different and each represents a hydrogen atom or an alkyl group having 1 to 5 carbon atoms;

v) a phenyl group;
wherein the phenyl group may be substituted by a halogen atom, a hydroxyl group, $-CF_3$, $-NO_2$, -CN, an alkyl group having 1 to 5 carbon atoms, an alkoxy group having 1 to 5 carbon atoms, $-COR_4$ or

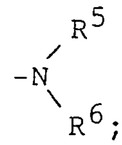

vi) a phenoxy group;
wherein the phenoxy group may be substituted by the groups cited in v), in addition to phenyl, phenoxy or pyridyloxy group (wherein the phenyl, phenoxy or pyridyloxy group may be substituted by halogen atom or $-CF_3$) vii)

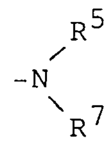

wherein $R^7$ is a hydrogen atom, an alkyl group having 1 to 5 carbon atoms or $-COR^4$;
viii) -CN;
c) an alkoxy group which may be substituted by the following substituents:
substituents:
i) a halogen atom;
ii) a phenyl group (wherein the phenyl group may be substituted by substituents v) in b);
d) a phenoxy group which may be substituted by the following substituents:
substituents: the same substituents as v) in b); e) an aromatic hydrocarbon group having 6 to 20 carbon atoms which may be substituted by the following substituents:
substituents:
i) a halogen atom;
ii) a hydroxyl group;
iii) $-CF_3$;
iv) $-NO_2$;
v) -CN;
vi) an alkyl group having 1 to 5 carbon atoms;
vii) an alkoxy group having 1 to 5 carbon atoms;
viii) $-COR^4$;
ix)

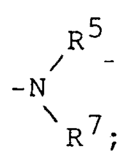

x) $-N^+R^5R^6R^8$
wherein $R^8$ is an alkyl group having 1 to 5 carbon atoms;
xi) a phenyl group
wherein the phenyl group may be substituted by the substituents v) in b);
xii) a phenoxy group
wherein the phenoxy group may be substituted by the substituents vi) in b);
xiii) $-CH_2COR^4$;
f) an aromatic heterocyclic group containing at least one nitrogen atom having 3 to 20 carbon atoms which may be substituted by the following substituents:
substituents: the same substituents as shown in e);

g) 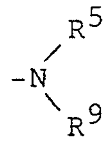

wherein $R^9$ is b), e) or f);

$R^3$ is a hydrogen atom ; or an aliphatic hydrocarbon group having 1 to 10 carbon atoms which may be substituted by a halogen atom, a hydroxyl group, an alkoxy group, $-COR^4$ or

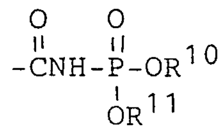

wherein $R^{10}$ and $R^{11}$ are identical or different and represent a hydrogen atom, an alkyl group having 1 to 5 carbon atoms, or a phenyl group;

Q represents a direct bond,

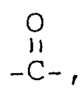, 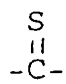 or 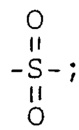;

wherein in case that Q is a direct bond, $R^2$ is not c), d) or g);

or their salts characterized by

(A) converting the compound of formula (V) into the compound of formula (I) according to the following reaction scheme:

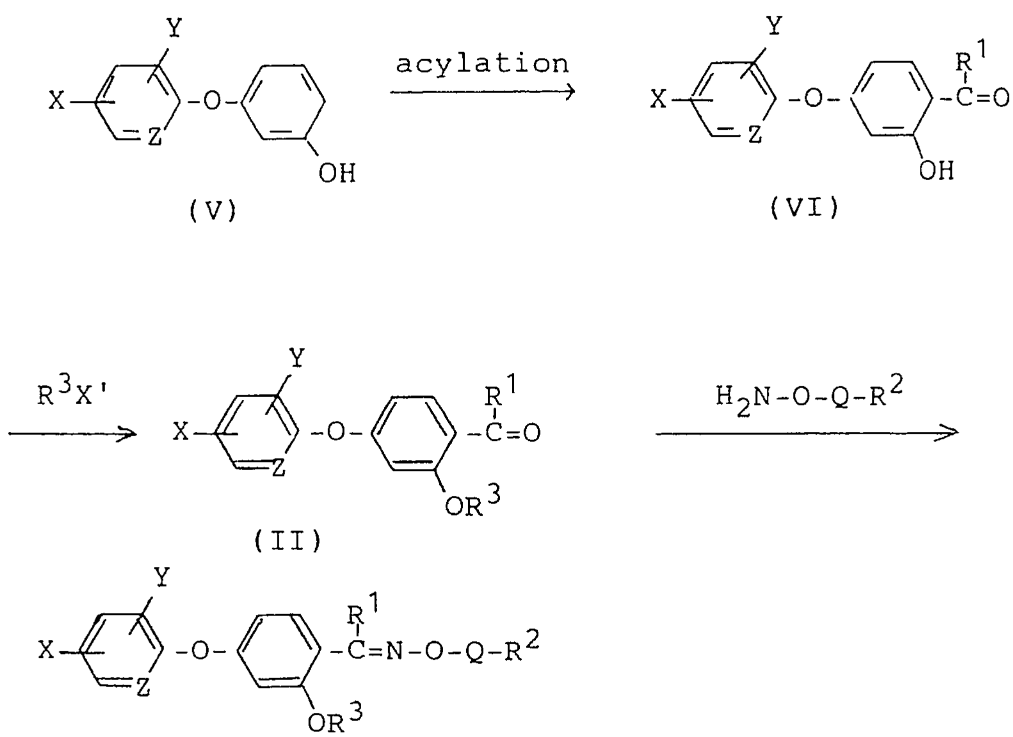


(V) (VI) (II) (I)

or

(B) converning the compound of formula (V) into the compound of formula (I) according to the following reaction scheme:

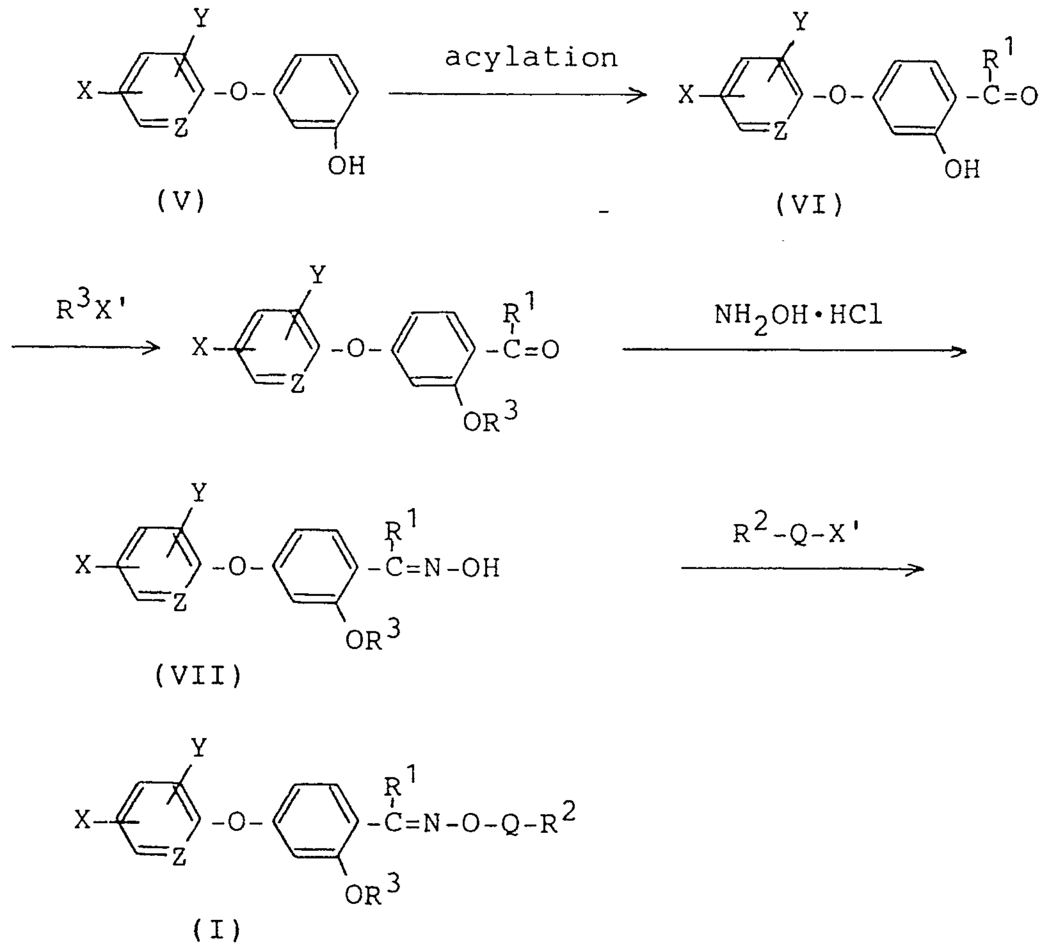


(V) (VI) (VII) (I)

(C) by starting with the reaction between the compounds of formula (VIII) and formula (IX) and converting the product of formula (X) into the compounds of formula (I) according to the following reaction scheme:

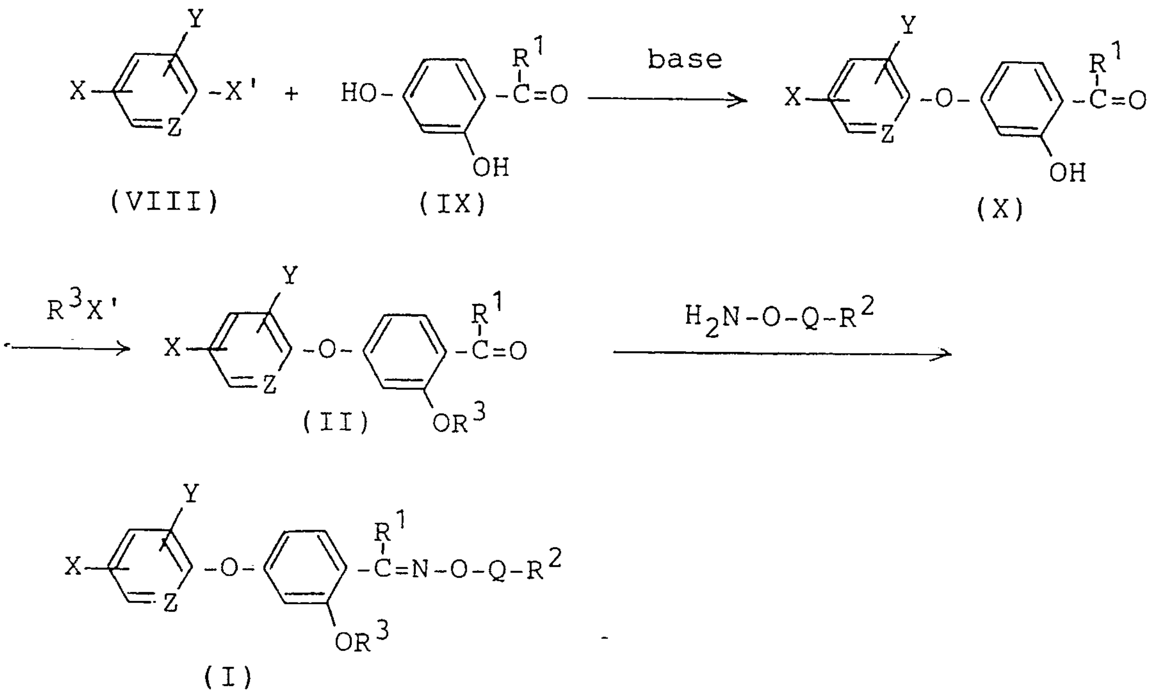

(VIII) (IX) (X)
(II)
(I)

(D) or by starting with the reaction between the compounds of formula (VIII) and formula (IX) and converting the product of formula (X) into the compounds (I) according to the following reaction scheme:

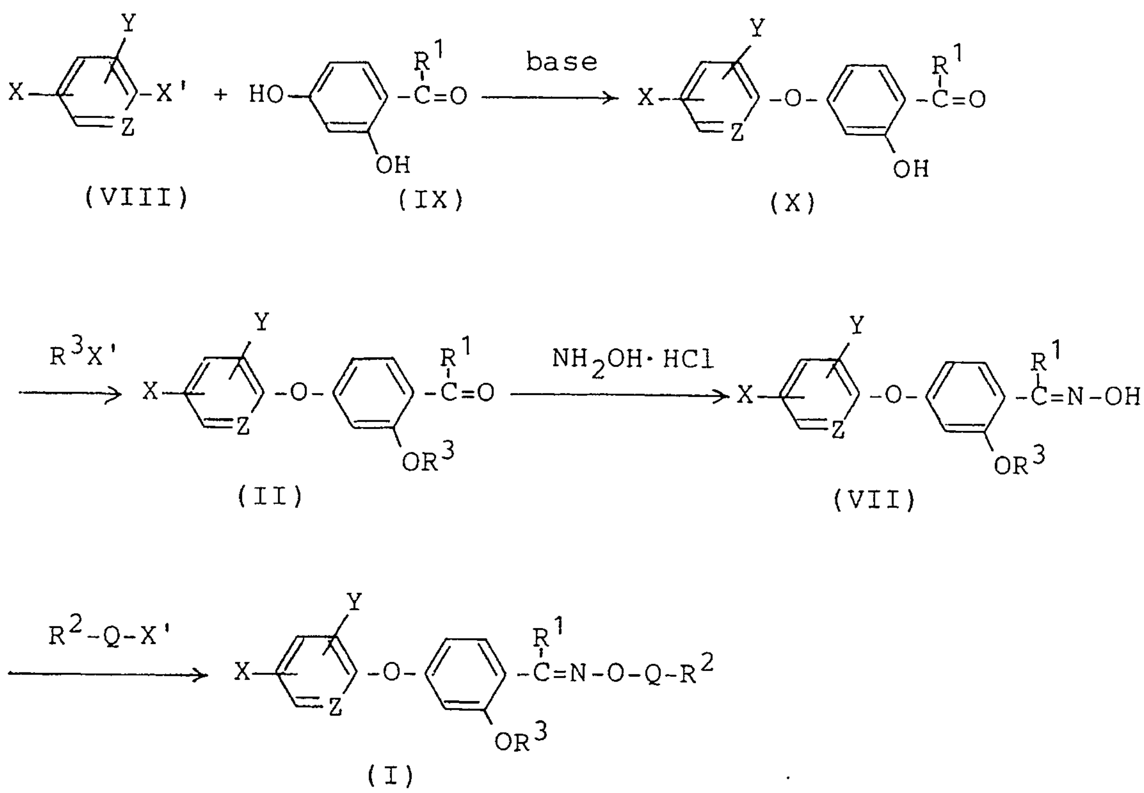

(VIII) (IX) (X)
(II) (VII)
(I)

(E) or converting the compound of formula (X) into the desired compound of formula (I) according to the following scheme:

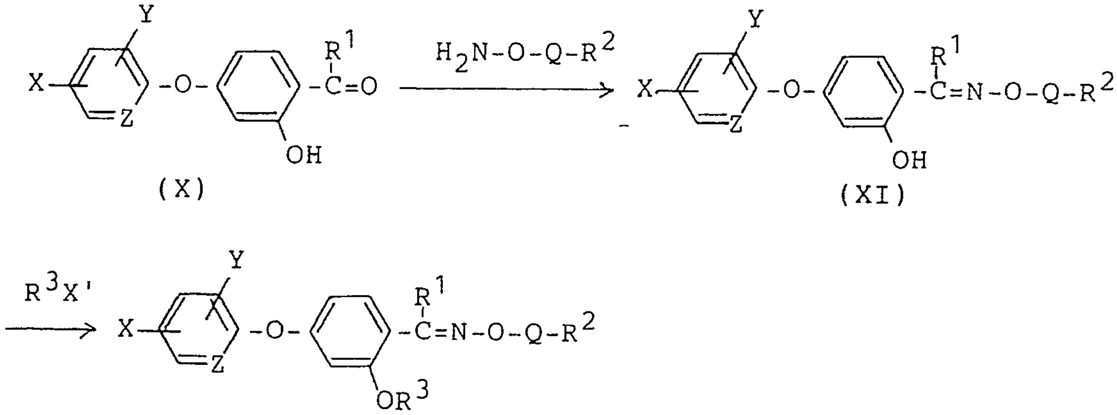


(X) (XI)

(I)

(F) by converting the compound of formula (XII) into the desired compound of formula (I) according to the following reaction scheme:

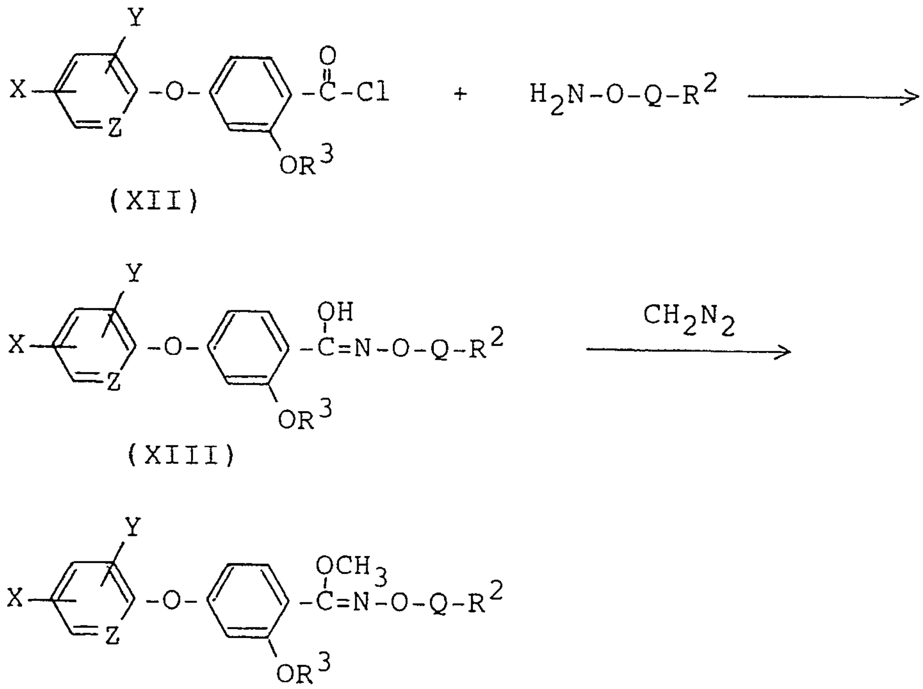


(XII)

(XIII)

(XIV)

(G) or converting the compound of formula (VII) into the compound of formual (XV) according to the following reaction scheme:

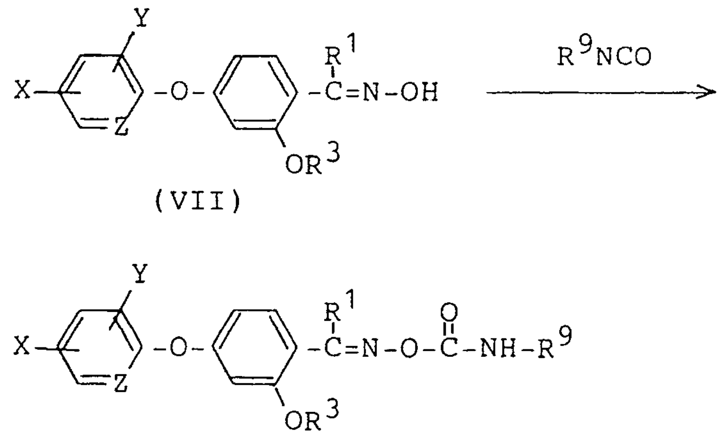


(VII)

(XV)

(H) or converting the compound of formula (VII) into the compound of formula (XVI) according to the following reaction scheme:

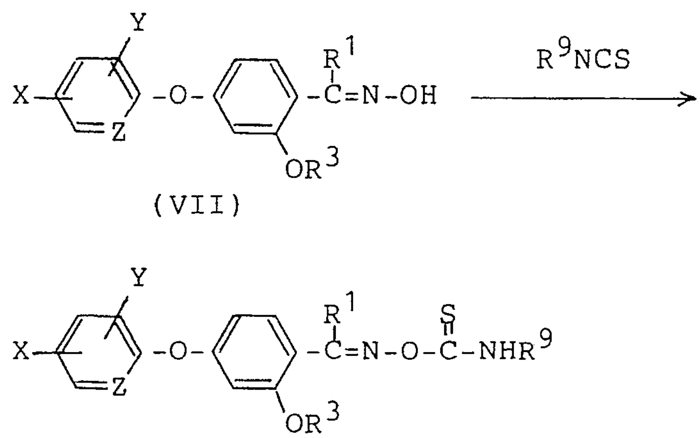


(VII)

(XVI)

wherein X, Y, Z, $R^1$, $R^2$, $R^3$, $R^9$ and Q are the same as in formula (I) and $X'$ means a halogen atom or $-OR^{40}$,
wherein $R^{40}$ is an alkylsulfonyl or arylsulfonyl.

8. A process for producing a herbicidal composition characterized in that a compound of claim 1 is mixed with at least one component selected from the group composed of carriers and surfactants.

9. A process for producing a herbicidal composition characterized in that a compound of claim 1 is mixed with at least one component selected from the group composed of an N-phosphonomethylglycine derivative of formula (III)

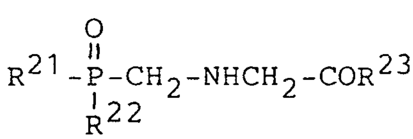
(III)

wherein
$R^{21}$ and $R^{22}$ are identical or different, and each repre sents a hydroxyl group or $-OR^{24}$
$R^{23}$ is a hydroxyl group, $-OR^{24}$ or

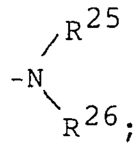

wherein
$R^{24}$ represents an alkyl group having 1 to 5 carbon atoms, a cyclohexyl group, a haloalkyl group having 1 to 5 carbon atoms, an alkenyl group having 2 to 5 carbon atoms or an alkoxyalkyl haloalkoxyalkyl or alkoxyalkoxyalkyl group (herein each of the alkoxy, haloalkoxy and alkyl groups has 1 to 5 carbon atoms) and a phenoxy group;
$R^{25}$ and $R^{26}$ are identioal or different and each represents a hydrogen atom, an alkyl group having 1 to 5 carbon atoms, a hydroxyalkyl group having 1 to 5 carbon atoms, a hydroxyalkyl group having 1 to 5 carbon atoms, an alkenyl group having 2 to 5 carbon atoms, or $R^{25}$ and $R^{26}$, together with the nitrogen atom to which they are attached, can form a morpholino group, a piperidino group or a pyrrolidino group, and/or a glufosinate compound of formula (IV)

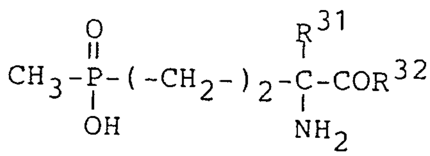
(IV)

wherein
$R^{31}$ represents a hydrogen atom or an alkyl group having 1 to 4 carbon atoms,
$R^{32}$ represents -OH, $-NH_2$, $-NHNH_2$, $-NHC_6H_5$ or an alkoxy group having 1 to 12 carbon atoms which may be substituted by -OH, or its acid addition salt or a salt with a base, and a carrier and/or a surfactant.

10. A method for controlling undesired weeds characterized by applying a compound of formula (I) according to claim 1 as such or in the form of a composition according to claim 3, 4 or 5 to the weeds and/or their seeds directly or to the soil in an amount enough to satisfactorily inhibit their growth or eradicate them in the places where useful plants and/or their seeds coexist or possibly coexist with undesired weeds and/or their seeds.

European Patent Office

# EUROPEAN SEARCH REPORT

Application Number

EP 90 30 1439

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,A | EP-A-0 281 103 (TEIJIN LTD) * Examples 24,2,3,4,5,7-15,25,28,29,31 * | 2,1 | C 07 C 251/48<br>C 07 D 251/66<br>C 07 C 327/00<br>C 07 C 309/00<br>C 07 D 213/643<br>A 01 N 35/10<br>A 01 N 43/00 |
| X | CHEMICAL ABSTRACTS, vol. 84, 1976, page 173, abstract no. 55320u, Columbus, Ohio, US; & JP-A-75 125 026 (ISHIHARA MINING AND CHEMICAL CO., LTD) 01-10-1975 * Abstract, online graphic * & GENERIC DARC, online graphic of CHEMICAL ABSTRACTS, registry no. 57945-85-4 | 2 | |
| X | CHEMICAL ABSTRACTS, vol. 109, 1988, page 792, abstract no. 201599v, Columbus, Ohio, US; & JP-A-63 22 682 (FUJI PHOTO FILM CO., LTD) 30-01-1988 * Abstract, online graphic * & GENERIC DARC, online graphic of CHEMICAL ABSTRACTS, registry no. 116239-20-4 | 2 | **TECHNICAL FIELDS SEARCHED (Int. Cl.5)** |
| X | JOURNAL OF MEDICINAL CHEMISTRY, vol. 28, no. 6, 1985, pages 717-727, American Chemical Society; D.W. ROBERTSON et al.: "Structure-activity relationships of arylimidazopyridine cardiotonics: discovery and inotropic activity of 2-[2-methoxy-4-(methylsulfinyl)phenyl]-1H-imidazo[4,5-c]pyridine" * Starting material compound #55, registry no. 95420-77-2 * | 2 | C 07 C 251/00<br>C 07 C 327/00<br>C 07 D 309/00<br>C 07 D 213/00 |

The present search report has been drawn up for all claims

| Place of search | Date of completion of the search | Examiner |
|---|---|---|
| THE HAGUE | 12-07-1990 | WELLS A.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)